# EUROPEAN PATENT APPLICATION

(11) **EP 4 410 313 A1**
(43) Date of publication of application: **07.08.2024**
(21) Application number: 23154322.4
(22) Date of filing: 31.01.2023
(51) Int. Cl.: A61K 47/68, A61P 35/00

(54) **HOMOGENEOUS ANTIBODY-CONJUGATES WITH HIGH PAYLOAD LOADING**

(71) Applicant: Synaffix B.V., 5349 AB Oss (NL)
(72) Inventor: The designation of the inventor has not yet been filed
(74) Representative: Nederlandsch Octrooibureau

(57) **Abstract**

The invention concerns homogenous antibody-conjugates with high payload loading (high DAR) obtained by site-specific conjugation to a single antibody N-glycan. The conjugates according to the invention are homogeneous, i.e. have a DAR at or close to the theoretical DAR with a narrow distribution, and do not require any genetic modification of the antibody. The invention further concerns a modular, non-genetic preparation method for such conjugates, involving three simple steps and starting from any antibody. These steps are (a) enzymatic remodeling of the glycan to give an antibody functionalized with two or four click probes per antibody, (b) strain-promoted cycloaddition with a multivalent, bifunctional reagent comprising one cyclic alkyne and at least two click probes that are not reactive towards the cyclic alkyne, and (c) inverse electron-demand Diels-Alder cycloaddition of the click probes with branched linker-drug constructs comprising one cyclic alkyne or strained alkene, connected to one or more payloads preferably connected through a cleavable linker. The resulting conjugates, with DAR6 or higher, are rapidly generated with high homogeneity and with surprising stability. In addition, HIC profiles of the resulting ADCs indicate small relative retention time and therefore show high potential in the targeting of tumour cells and/or the treatment of cancer.

## Description

### Field of the invention

The present invention is in the field of medicine. More specifically, the present invention relates to antibody-conjugates with a high payload loading, that are homogeneous and do not require genetic antibody modification. Such antibody-conjugates can be applied for the more effective treatment of disease, in particular cancer.

### Background

Antibody-drug conjugates (ADC), considered as one of the major classes of targeted therapy, are comprised of an antibody to which is attached a pharmaceutical agent. The antibodies (also known as binding agents or ligands) can be small protein formats (scFv's, Fab fragments, DARPins, Affibodies, etc.) but are generally monoclonal antibodies (mAbs) of IgG type which have been selected based on their high selectivity and affinity for a given antigen, their long circulating half-lives, and little to no immunogenicity. Thus, mAbs as ligands for a carefully selected biological receptor provide an ideal targeting platform for selective delivery of pharmaceutical drugs. For example, a monoclonal antibody known to bind selectively with a specific cancer-associated antigen can be used for delivery of a chemically conjugated cytotoxic agent to the tumour, via binding, internalization, intracellular processing and finally release of active catabolite. The cytotoxic agent may be small molecule toxin, a protein toxin or other formats, like oligonucleotides. As a result, the tumour cells can be selectively eradicated, while sparing normal cells which have not been targeted by the antibody. Similarly, chemical conjugation of an antibacterial drug (antibiotic) to an antibody can be applied for treatment of bacterial infections, while conjugates of anti-inflammatory drugs are under investigation for the treatment of autoimmune diseases and for example attachment of an oligonucleotide to an antibody is a potential promising approach for the treatment of neuromuscular diseases. Hence, the concept of targeted delivery of an active pharmaceutical drug to a specific cellular location of choice is a powerful approach for the treatment of a wide range of diseases, with many beneficial aspects versus systemic delivery of the same drug.

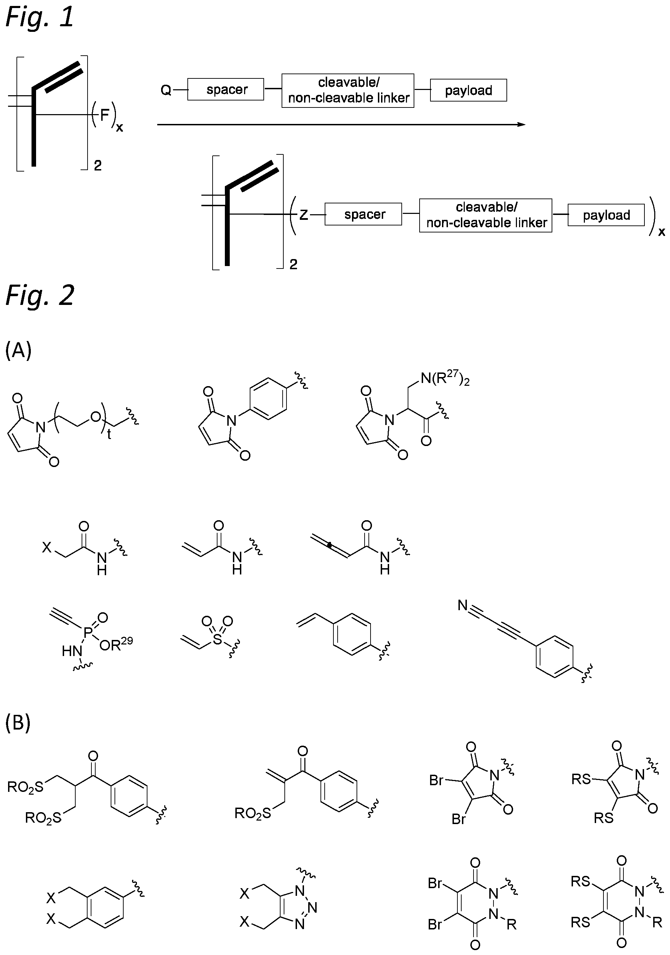

ADCs are prepared by conjugation of a linker-drug to a protein, a process known as bioconjugation. Many technologies are known for bioconjugation, as summarized in G.T. Hermanson, "Bioconjugate Techniques", Elsevier, 3rd Ed. 2013**,** incorporated by reference. Conceptually, the method the preparation of an ADC by bioconjugation entails the reaction of x number of reactive moieties F present on the antibody with a complementary reactive moiety Q present on the pharmaceutical drug (the payload), see Figure 1.

Typically, a chemical linker is present between Q and the payload. This linker needs to possess a number of key attributes, including the requirement to be stable in plasma after drug administration for an extended period of time. A stable linker enables localization of the ADC to the projected site or cells in the body and prevents premature release of the payload in circulation, which would indiscriminately induce undesired biological response of all kinds, thereby lowering the therapeutic index of the ADC. Upon internalization, the ADC should be processed such that the payload is effectively released so it can exert its mode-of-action inside the cell. The linker can also contain a spacer element. There are two families of linkers, non-cleavable and cleavable. Non-cleavable linkers consist of a chain of atoms between the antibody and the payload, which is fully stable under physiological conditions, irrespective of which organ or biological compartment the antibody-drug conjugate resides in. As a consequence, liberation of the payload from an ADC with a non-cleavable linker relies on the complete (lysosomal) degradation of the antibody after internalization of the ADC into a cell. As a result of this degradation, the payload will be released, still carrying the linker, as well as a peptide fragment and/or the amino acid from the antibody the linker was originally attached to. Cleavable linkers utilize an inherent property of a cell or a cellular compartment for selective release of the payload from the ADC, which generally leaves no trace of linker after processing. For cleavable linkers, there are three commonly used mechanisms: (1) susceptibility to specific enzymes, (2) pH-sensitivity, and (3) sensitivity to redox state of a cell (or its microenvironment). The cleavable linker may also contain a self-immolative unit, for example based on a *para*-aminobenzyl alcohol group or *para*-hydroxybenzyl alcohol and derivatives and/or analogues thereof or a cyclization linker based on for example 1,2-diaminoethane carbamate derivatives. A linker may also contain an additional element, often referred to as spacer or stretcher unit, to connect the linker with a reactive group for attachment to the antibody via a reactive moiety F present on the antibody.

The reactive moiety F can be naturally present in the antibody, for example the reactive moiety can be the side chain of lysine or cysteine, which can be employed for acylation (lysine side chain) or alkylation (cysteine side chain).

Acylation of the ε-amino group in a lysine side-chain is typically achieved by subjecting the protein to a reagent based on an activated ester or activated carbonate derivative, for example SMCC is applied for the manufacturing of Kadcyla^{®}. Based on the fact that a given antibody may contain 60-90 occurrences of lysine, of which the vast majority will display reactivity to the acylating agent, careful titration of the acylating agent is required which nevertheless results in a highly heterogeneous mixture of conjugation with only an average drug loading based on stochastic distribution of drugs attached to the antibody. For example, Kadcyla^{®} has an average drug-to-antibody ratio (DAR) of approximately four but in fact consists of a mixture of components with DAR 0-12. Addition of a larger quantity of acylating agents will lead to a higher average DAR, for example DAR 6 or DAR8 can be achieved, based on a stochastic distribution containing even higher DAR species (i.e. >DAR 12).

Various reagents are known for alkylation of the thiol group in cysteine side-chain, see Figure 2. Amongst the cysteine alkylation strategies, the vast majority is based on the use of maleimide reagents, as is for example applied in the manufacturing of Adcetris^{®}, Polivy^{®} and Padcev^{®}. Besides standard maleimide reagents, a range of maleimide variants are also applied for more stable cysteine conjugation, as for example demonstrated by James Christie et al., J. Contr. Rel. 2015, 220, 660-670 and Lyon et al., Nat. Biotechnol. 2014, 32, 1059-1062, both incorporated by reference. Other approaches for cysteine alkylation involve for example nucleophilic substitution of haloacetamides (typically bromoacetamide or iodoacetamide), see for example Alley et al., Bioconj. Chem. 2008, 19, 759-765, incorporated by reference, or various approaches based on nucleophilic addition on unsaturated bonds, such as reaction with acrylate reagents, see for example Bernardim et al., Nat. Commun. 2016, 7, 13128 and Ariyasu et al., Bioconj. Chem. 2017, 28, 897-902, both incorporated by reference, reaction with phosphonamidates, see for example Kasper et al., Angew. Chem. Int. Ed. 2019, 58, 11625-11630, incorporated by reference, reaction with allenamides, see for example Abbas et al., Angew. Chem. Int. Ed. 2014, 53, 7491-7494, incorporated by reference, reaction with cyanoethynyl reagents, see for example Kolodych et al., Bioconj. Chem. 2015, 26, 197-200, incorporated by reference, reaction with vinylsulfones, see for example Gil de Montes et al., Chem. Sci. 2019, 10, 4515-4522, incorporated by reference, or reaction with vinylpyridines, see for example Seki et al, Chem. Sci., 2021, 12, 9060-9068 and https://iksuda.com/science/permalink/ (accessed July 26^{th}, 2020).

In terms of DAR, similar to lysine conjugation this is controlled by titration of alkylating reagent for reaction with free cysteine side-chains (liberated by reduction of interchain disulfides with for example TCEP or DTT). The final DAR is typically an average number comprised of a stochastic mixture of different components, again similar to lysine conjugation. However, a few notable differences can be noted: (a) the different DAR species typically consist of a multitude of 2 (*i.e*. 2, 4, 6, 8) and (b) the maximum DAR that can be achieved is 8 (if all liberated interchain cysteine side-chains have reacted). This also means that by comprehensive alkylation of all interchain cysteine side-chains, a homogeneous DAR8 ADC can be achieved. This is by far the most common method to generate DAR8 ADCs and likely the only method employed for any clinical ADC with DAR8. It must be noted that such approach cannot generate homogeneous DAR6 ADC or ADCs with DAR>8, unless specific cysteines are engineered out or added into the antibody sequence by recombinant DNA technology. It must also be noted that any method involving a reduction step may lead to antibody degradation (reduction of additional disulfide bonds) or fragment scrambling (due to exchange of light chains for example).

An alternative approach to antibody conjugation to interchain disulfide bridges involves the use of a cysteine cross-linking reagent, *i.e*. a reagent that will react with two cysteine side-chains concurrently. Examples of such cross-linking agents are bis-sulfone reagents, see for example Balan et al., Bioconj. Chem. 2007, 18, 61-76 and Bryant et al., Mol. Pharmaceutics 2015, 12, 1872-1879, both incorporated by reference, mono- or bis-bromomaleimides, see for example Smith et al., J. Am. Chem. Soc. 2010, 132, 1960-1965 and Schumacher et al., Org. Biomol. Chem. 2014, 37, 7261-7269, both incorporated by reference, bis-maleimide reagents, see for example WO2014114207, bis(phenylthio)maleimides, see for example Schumacher et al., Org. Biomol. Chem. 2014, 37, 7261-7269 and Aubrey et al., Bioconj. Chem. 2018, 29, 3516-3521, both incorporated by reference, bis-bromopyridazinediones, see for example Robinson et al., RSC Advances 2017, 7, 9073-9077, incorporated by reference, bis(halomethyl)benzenes, see for example Ramos-Tomillero et al., Bioconj. Chem. 2018, 29, 1199-1208, incorporated by reference or other bis(halomethyl)aromatics, see for example WO2013173391. Typically, ADCs prepared by cross-linking of cysteines have a drug-to-antibody loading of four (DAR4), which is achieved by complete alkylation of all cysteine side-chains liberated by reduction.

Another useful technology for conjugation to a cysteine side chain is by means of formation of a novel disulfide bond, by treatment of a liberated cysteine side-chain with thiolating agent (*i.e*. a non-symmetrical disulfide bond of which one thiol is part of a good leaving group), leading to a bioactivatable connection that has been utilized for reversibly connecting protein toxins, chemotherapeutic drugs, and probes to carrier molecules (see for example Pillow et al., Chem. Sci. 2017, 8, 366-370, incorporated by reference). Similar to cysteine alkylation, the average DAR of such ADCs can be tailored to around 2-8 in case native interchain disulfide bonds are reduced.

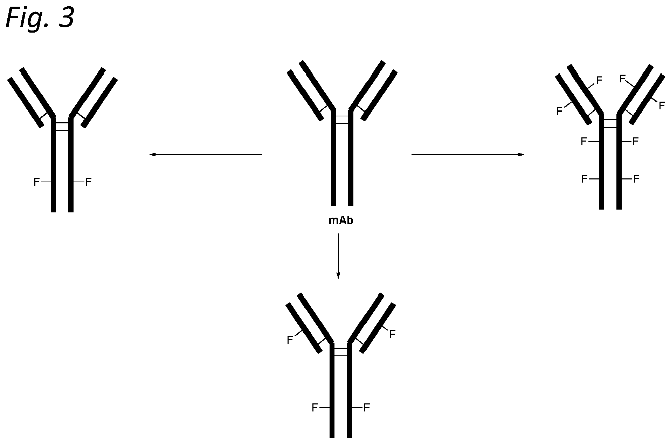

Besides conjugation to the side chains of the naturally present amino acids lysine or cysteine, a range of other conjugation technologies has been explored based on a two-stage strategy involving (a) introduction on a novel reactive group F, followed by (b) reaction with another complementary reactive group Q. For example, a method can be used to introduce a given number of reactive moieties F onto an antibody, which can be two, four or eight, see Figure 3.

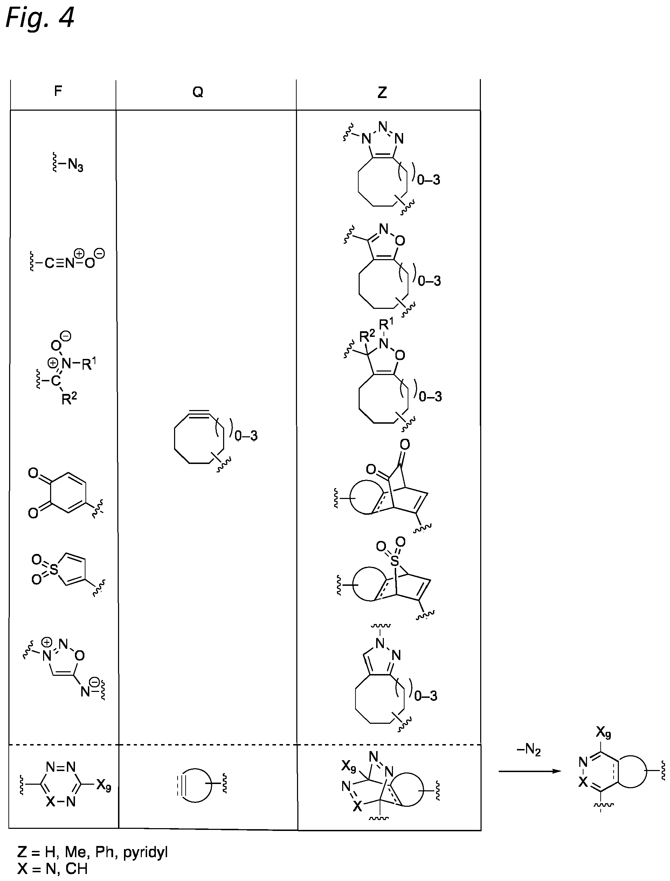

An example of an unnatural reactive functionality F that can be employed for bioconjugation of linker-drugs is the oxime group, suitable for oxime ligation or the azido group, suitable for click chemistry conjugation. The oxime can be installed in the antibody by genetic encoding of a non-natural amino acid, *e.g*. *p*-acetophenylalanine, as for example demonstrated by Axup et al. Proc. Nat. Acad. Sci. 2012, 109, 16101-16106, incorporated by reference, or by enzymatic alkylation of a cysteine present in a CAAX sequence with a prenyl group containing a remote keto group, as for example disclosed in WO2012153193. The azide can be installed in the antibody by genetic encoding of *p*-azidomethylphenylalanine or *p*-azidophenylalanine, as for example demonstrated by Axup et al. Proc. Nat. Acad. Sci. 2012, 109, 16101-16106, incorporated by reference. Similarly, Zimmerman et al., Bioconj. Chem. 2014, 25, 351-361, incorporated by reference have employed a cell-free protein synthesis method to introduce *p*-azidomethylphenylalanine (AzPhe) into monoclonal antibodies for conversion into ADCs by means of metal-free click chemistry. Also, it has also be shown by Nairn et al., Bioconj. Chem. 2012, 23, 2087-2097, incorporated by reference, that a methionine analogue like azidohomoalanine (Aha) can be introduced into protein by means of auxotrophic bacteria and further converted into protein conjugates by means of click chemistry. Finally, genetic encoding of aliphatic azides in recombinant proteins using a pyrrolysyl-tRNA synthetase/tRNA_{CUA} pair was shown by Nguyen et al., J. Am. Chem. Soc. 2009, 131, 8720-8721, incorporated by reference, and labelling was achieved by click chemistry, either by copper-catalyzed alkyne-azide cycloaddition (CuAAC) or strain-promoted alkyne-azide cycloaddition (SPAAC). Besides, CuAAC and SPAAC, bioconjugation of linker-drugs to antibodies (and other biomolecules such as glycans, nucleic acids) can be achieved by a range of other metal-free click chemistries, see e.g. Nguyen and Prescher, Nature Rev. Chem. 2020, 4, 476-489, incorporated by reference. For example, oxidation of a specific tyrosine in a protein can give an *ortho*-quinone, which readily undergoes cycloaddition with strained alkenes (*e.g*. TCO) or strained alkynes, see e.g. Bruins et al., Chem. Eur. J. 2017, 24, 4749-4756, incorporated by reference. Besides cyclooctyne, certain cycloheptynes are also suitable for metal-free click chemistry, as reported by Wetering et al. Chem. Sci. 2020, 11, 9011-9016, incorporated by reference. A tetrazine moiety can also be introduced into a protein or a glycan by various means, for example by genetic encoding or chemical acylation, and may also undergo cycloaddition with cyclic alkenes and alkynes. A list of pairs of functional groups F and Q for metal-free click chemistry is provided in Figure 4.

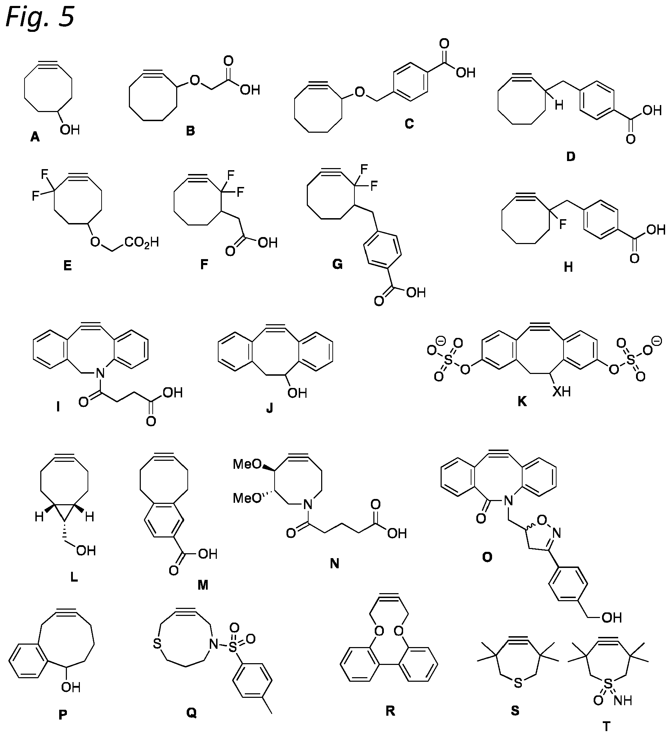

In a SPAAC bioconjugation, the linker-drug is functionalized with a cyclic alkyne and the cycloaddition with azido-modified antibody is driven by relief of ring-strain. Conversely, the linker-drug can be functionalized with azide and the antibody with cyclic alkyne. Various strained alkynes suitable for metal-free click chemistry are indicated in Figure 5.

A method of increasing popularity in the field of ADCs is based on enzymatic installation of a non-natural functionality F. For example, Lhospice etal., Mol. Pharmaceut. 2015, 12, 1863-1871, incorporated by reference, employ the bacterial enzyme transglutaminase (BTG or TGase) for installation of an azide moiety onto an antibody. A genetic method based on C-terminal TGase-mediated azide introduction followed by conversion in ADC with metal-free click chemistry was reported by Cheng et al., Mol. Cancer Therap. 2018, 17, 2665-2675, incorporated by reference.

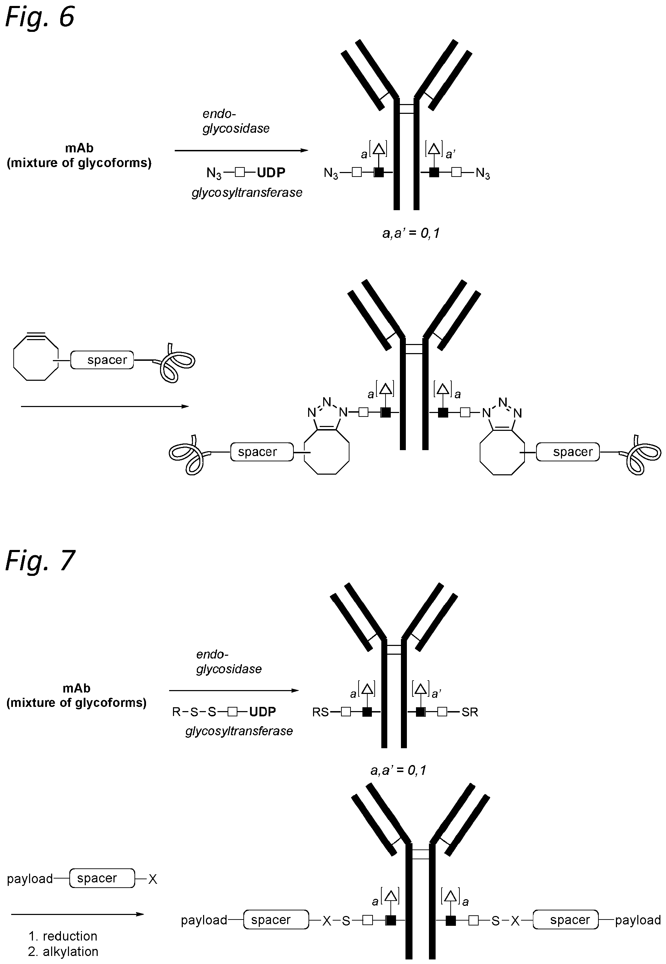

It has been shown in WO2014065661, by van Geel et al., Bioconj. Chem. 2015, 26, 2233-2242, Verkade et al., Antibodies 2018, 7, 12, and Wijdeven at al. MAbs 2022, 14, 2078466, all incorporated by reference, that enzymatic remodelling of the native antibody glycan at N297 enables introduction of an azido-modified sugar, suitable for attachment of cytotoxic payload using metal-free click chemistry, see Figure 6. Similarly, the enzymatic glycan remodelling protocol can also be employed to install a free thiol group on an antibody (see Figure 7) for conjugation based on any of the methods described above for cysteine conjugation.

β-Glucuronidase is an enzyme that plays a role in the breakdown of endogenous glucuronic acid-containing glycosaminoglycans and is normally localized to the lysosome of cells, including in the lysosome of first-pass tissues such as the liver and intestine. β-Glucuronidase concentrations in many solid tumors, including lung, breast, and gastrointestinal cancers, as well as in the tumor microenvironment, are reported to be higher than those in normal tissues, and the enzyme is not found in the general circulation. In addition, these enzyme levels are elevated in necrotic regions of tumors due to release of the lysosomal enzyme into the extracellular region from dying cells. Lysosomal concentrations of b-glucuronidase are also high in inflammatory immune cells, including neutrophils and eosinophils, and these cells can release the enzyme at sites of inflammation, such as the necrotic regions of human tumors. Exploitation of β-glucuronidase overexpression in certain tumors is a well-known approach in oncology research, where β-glucuronidase-cleavable triggers have been applied to anthracyclines, auristatins, duocarmycin and camptothecins, as well as in a multi-compound releasing prodrug approach, PBD prodrugs, and ADCs. Similarly, β-galactosidase is an enzyme that is upregulated in tumor microenvironment and can be used for the selective release of payloads capped with a β-galactoside attached to a phenolic position, for example a *p-*hydroxybenzyloxycarbonyl group.

Other enzymes that are upregulated in the tumor microenvironment have also been considered for release of cytotoxic payloads including cathepsins, matrix metalloproteinases (MMPs), legumain and serine protease elastase, as for example summarized by M. Poreba, FEBS J., 2020, 287, 1936-1969. Investigation into these two TME-related enzymes has led to the development of cleavable linkers based on specific peptide sequences Val-Cit or Val-Ala for cathepsins, Pro-Leu-Gly (PLG) for metalloproteinases (MMPs), Asn-Asn, Ala-Asn or Asn-Ala for legumain and Asn-Pro-Val (NPV) for serine protease elastase, for example.

Currently, cytotoxic payloads include for example microtubule-disrupting agents [e.g. auristatins such as monomethyl auristatin E (MMAE) and monomethyl auristatin F (MMAF), maytansinoids such as DM1 and DM4, tubulysins], DNA-damaging agents [e.g., calicheamicin, pyrrolobenzodiazepine (PBD) dimers, indolinobenzodiapine dimers, duocarmycins, anthracyclines such as PNU-159,682, topoisomerase inhibitors [e.g. DXd, exatecan, SN-38] or RNA polymerase II inhibitors [e.g. amanitin]. ADCs that have reached market approval include for example payloads MMAE, MMAF, DM1, DM4, calicheamicin, SN-38, DXd and a PBD dimer and a pivotal trial is running for an ADC based on duocarmycin. A larger variety of payloads is still under clinical evaluation or has been in clinical trials in the past, *e.g*. eribulin, indolinobenzodiazepine dimer, PNU-159,682, amanitin, hemi-asterlin and others. Finally, various ADCs in early clinical or late-stage preclinical stage are conjugated to novel payloads for example, KSP inhibitors, MMAD, cryptophycins, and others. Various other payloads have been evaluated in the past as well but have been abandoned due to lack of sufficient efficacy, for example doxorubicin, vinca alkaloids, duocarmycin SA. Even less potent payloads have not even been assessed clinically for the same reason (lack of sufficient efficacy).

One way to widen the scope of payloads employable for ADCs, by employing drugs of lesser potency than the current arsenal, is by increasing the DAR of the ADC, thereby effectively delivering more drug to the tumor per internalization event. For example, Mersana Therapeutics have developed the so-called Fleximer^{®} technology that enables ADCs with DAR12-15, which has been employed in their lead program XMT-1536 (UpRi). To achieve such high DAR, a polymeric, highly polar linker technology technology was developed first to be able to accommodate the multitude of hydrophobic payloads and to avoid aggregation/degradation of the high DAR ADC, which is essential to avoid severely compromised pharmacokinetic behavior of a high DAR ADC. In fact, it has been convincingly shown by Hamblett et al., Clin. Cane. Res. 2004, 10, 7063-7070, incorporated by reference, that DAR8 ADCs show better in vitro efficacy due to the higher drug loading, while the in vivo performance was worse than the equivalent DAR4 ADC, due to significantly faster clearance of the DAR8 species. Such poor PK properties become apparent by measuring the profile with hydrophilic interaction chromatography (HIC), in particular large relative retention times versus naked antibody can be taken as an indication of poor PK performance, as disclosed by Burke et al., Mol. Canc. Ther. 2017, 16, 116-123, incorporated by reference. Other solutions to mitigate poor PK properties and enable DAR8 ADCs typically involve the insertion of a branched, hydrophilic spacer to shield the hydrophobic payload, for example based on polyethylene glycol (PEG), as disclosed by Burke et al., Mol. Canc. Ther. 2017, 16, 116-123, incorporated by reference, employed in SGN-CD228A and SGN-CD48A, polysarcosine, as disclosed by Viricel et al., Chem. Sci. 2019, 10, 4048-4053, or polysaccharide such as chitosan, as for example disclosed in WO2022058548 and WO2022048883 and employed in M1231.

To date, all high DAR ADCs (~DAR8 or above) that have reached the clinic are based on a conjugation technology employing cysteine alkylation with a linear linker-drug, which therefore is limited at DAR8 unless additional cysteines are engineered into the antibody by means of recombinant DNA technology. XMT-1536 has a drug loading of 12-15 and is therefore the exception to this rule, however XMT-1536 is also a highly heterogenous ADC, consisting of stochastically conjugated cysteines and a highly heterogeneous polymer attached to each cysteine.

### Summary of the invention

The inventors have for the first time been able to prepare homogenous antibody-conjugates with high payload loading (high DAR) by site-specific conjugation to a single antibody N-glycan. The conjugates according to the invention are homogeneous, i.e. have a DAR at or close to the theoretical DAR with a narrow distribution, and do not require any genetic modification of the antibody. The conjugates according to the invention have a payload-to-antibody ratio (DAR) of 6 or higher, preferably 8 or higher. Conjugates with a DAR up to 64 can be readily prepared.

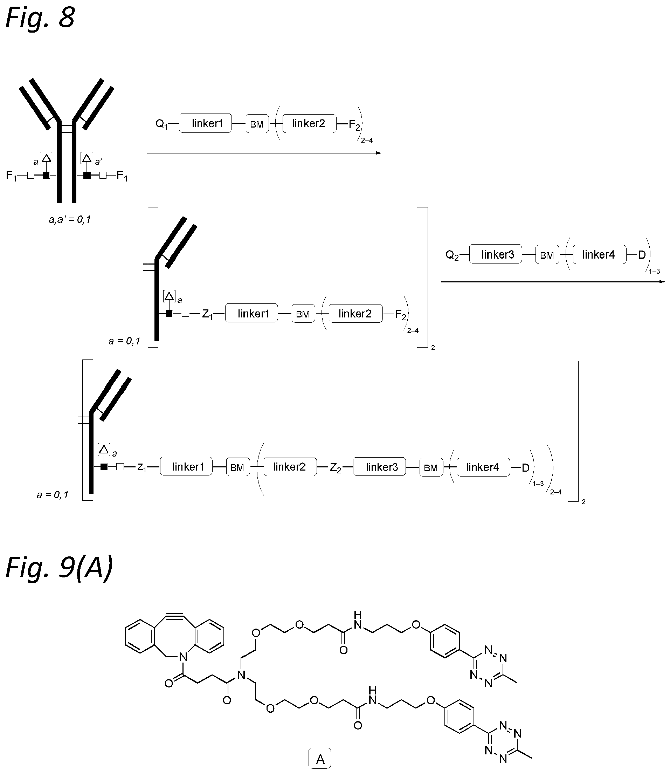

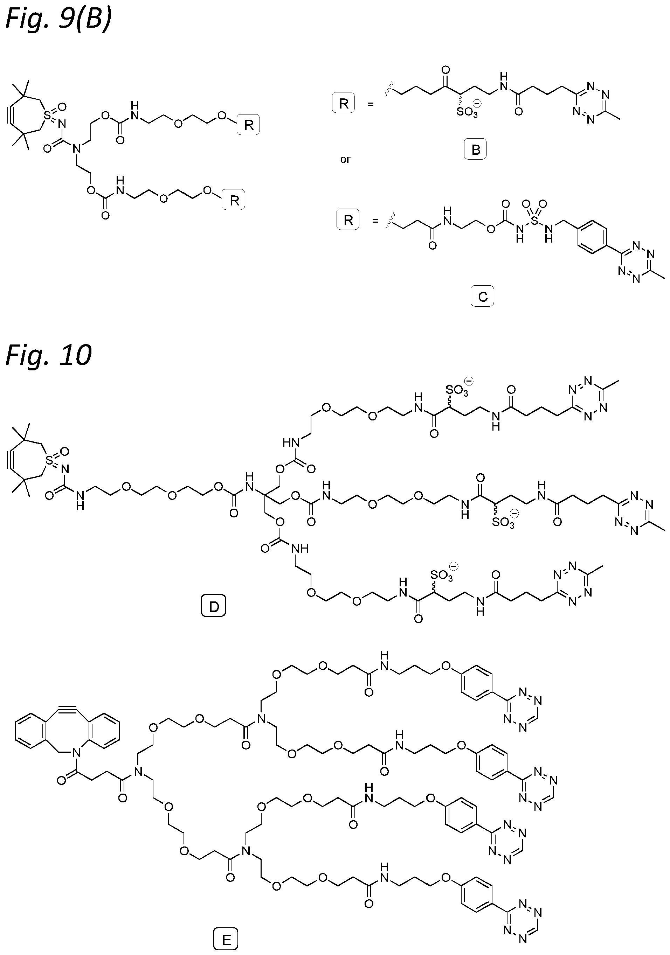

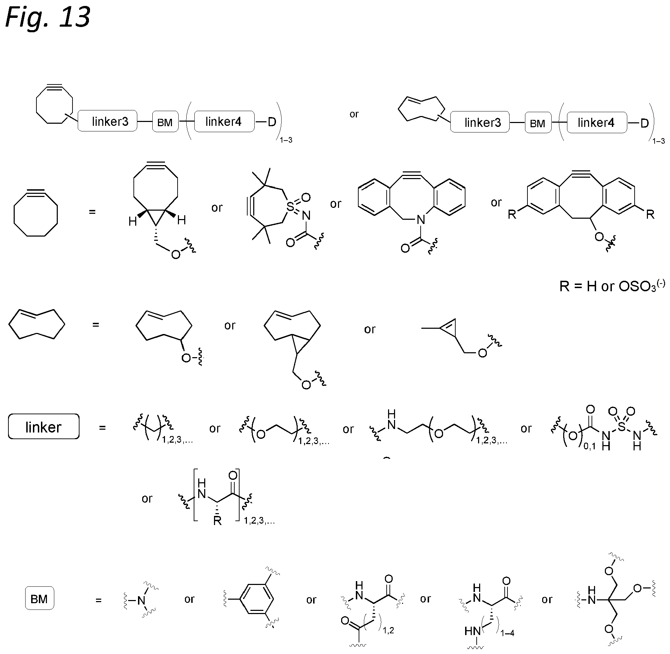

The inventors have developed a modular, non-genetic preparation method for such conjugates, involving three simple steps and starting from any antibody (see Figure 8). These steps are (a) enzymatic remodeling of the glycan to give an antibody functionalized with two or four click probes (e.g. an azide) per antibody, (b) strain-promoted azide-alkyne cycloaddition with a multivalent, bifunctional reagent comprising one cyclic alkyne and at least two click probes that are not reactive towards the cyclic alkyne (e.g. tetrazines) (see Figures 9-12), and (c) inverse electron-demand Diels-Alder cycloaddition of the click probes with branched linker-drug constructs comprising one cyclic alkyne or strained alkene, connected to one or more payloads preferably connected through a cleavable linker (see Figure 13-16). The resulting conjugates, with DAR6 or higher, are rapidly generated with high homogeneity and with surprising stability. In addition, HIC profiles of the resulting ADCs indicate small relative retention time and therefore show high potential in the treatment of cancer.

### Detailed description

### Definitions

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the elements is present, unless the context clearly requires that there is one and only one of the elements. The indefinite article "a" or "an" thus usually means "at least one".

A linker is herein defined as a moiety that connects (covalently links) two or more elements of a compound. A linker may comprise one or more spacer moieties. A spacer-moiety is herein defined as a moiety that spaces (i.e. provides distance between) and covalently links together two (or more) parts of a linker. The linker may be part of e.g. a linker-construct, a linker-conjugate, a linker-payload (e.g. linker-drug) or an antibody-conjugate, as defined below.

A "hydrophilic group" or "polar linker" is herein defined as any molecular structure containing one or more polar functional groups that imparts improved polarity, and therefore improved aqueous solubility, to the molecule it is attached to. Preferred hydrophilic groups are selected from a carboxylic acid group, an alcohol group, an ether group, a polyethylene glycol group, an amino group, an ammonium group, a sulfonate group, a phosphate group, an acyl sulfamide group or a carbamoyl sulfamide group. In addition to higher solubility other effects of the hydrophilic group include improved click conjugation efficiency, and, once incorporated into an antibody-drug conjugate: less aggregation, improved pharmacokinetics resulting in higher efficacy and in vivo tolerability.

The term "salt thereof" means a compound formed when an acidic proton, typically a proton of an acid, is replaced by a cation, such as a metal cation or an organic cation and the like. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt. The term "pharmaceutically accepted" salt means a salt that is acceptable for administration to a patient, such as a mammal (salts with counterions having acceptable mammalian safety for a given dosage regime). Such salts may be derived from pharmaceutically acceptable inorganic or organic bases and from pharmaceutically acceptable inorganic or organic acids. "Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

The term "enediyne" or "enediyne antibiotic" or "enediyne-containing cytotoxin" refers to any cytotoxin characterized by the presence of a 3-ene-1,5-diyne structural feature as part of a cyclic molecule as known in the art and include neocarzinostatin (NCS), C-1027, kedarcidin (KED), maduropeptin (MDP), N1999A2, the sporolides (SPO), the cyanosporasides (CYA and CYN), and the fijiolides, calicheamicins (CAL), the esperamicins (ESP), dynemicin (DYN), namenamicin, shishijimicin, and uncialamycin (UCM).

The term "alkylaminosugar" as used herein means a tetrahydropyranyl moiety connected to an alcohol function via its 2-position, thereby forming an acetal function, and further substituted by (at least) one N-alkylamino group in position 3, 4 or 5. "N-alkylamino group" in this context refers to an amino group having one methyl, ethyl or 2-propyl group.

The term "click probe" refers to a functional moiety that is capable of undergoing a click reaction, i.e. two compatible click probes mutually undergo a click reaction such that they are covalently linked in the product. Compatible probes for click reactions are known in the art, and preferably include (cyclic) alkynes and azides. In the context of the present invention, click probe Q in the compound according to the invention is capable of reacting with click probe F on the (modified) protein, such that upon the occurrence of a click reaction, a conjugate is formed wherein the protein is conjugated to the compound according to the invention. Herein, F and Q are compatible click probes.

The term "(hetero)alkyl" refers to alkyl groups and heteroalkyl groups. Heteroalkyl groups are alkyl groups wherein one or more carbon units in the alkyl chain (e.g. CH₂, CH or C) are replaced by heteroatoms, such as O, S, S(O), S(O)₂ or NR⁴. In other words, the alkyl chain is interrupted with one ore more elements selected from O, S, S(O), S(O)₂ and NR⁴. Such interruptions are distinct from substituents, as they occur within the chain of an alkyl group, whereas substituents are pendant groups, monovalently attached to e.g. a carbon atom of an alkyl chain. In a preferred embodiment, the (hetero)alkyl group is an alkyl group, e.g. ethyl (Et), isopropyl (i-Pr), n-propyl (n-Pr), tert-butyl (t-Bu), isobutyl (i-Bu), n-butyl (n-Bu) or n-pentyl.

Likewise, the term "(hetero)aryl" refers to aryl groups and heteroaryl groups. Heteroaryl groups are aryl groups wherein one or more carbon units in the ring (e.g. CH) are replaced by heteroatoms, such as O, S, N or NR⁴.

An "acylsulfamide moiety" is herein defined as a sulfamide moiety (H₂NSO₂NH₂) that is N-acylated or N-carbamoylated on one end of the molecule and N-alkylated (mono or bis) at the other end of the molecule. In the context of the present invention, especially in the examples, this group is also referred to as "HS".

A "domain" may be any region of a protein, generally defined on the basis of sequence homologies and often related to a specific structural or functional entity. CEACAM family members are known to be composed of Ig-like domains. The term domain is used in this document to designate either individual Ig-like domains, such as "N-domain" or for groups of consecutive domains, such as "A3-B3 domain".

A "coding sequence" or a sequence "encoding" an expression product, such as a RNA, polypeptide, protein, or enzyme, is a nucleotide sequence that, when expressed, results in the production of that RNA, polypeptide, protein, or enzyme, i.e., the nucleotide sequence encodes an amino acid sequence for that polypeptide, protein or enzyme. A coding sequence for a protein may include a start codon (usually ATG) and a stop codon.

The term "gene" means a DNA sequence that codes for, or corresponds to, a particular sequence of amino acids which comprises all or part of one or more proteins or enzymes, and may or may not include regulatory DNA sequences, such as promoter sequences, which determine for example the conditions under which the gene is expressed. Some genes, which are not structural genes, may be transcribed from DNA to RNA, but are not translated into an amino acid sequence. Other genes may function as regulators of structural genes or as regulators of DNA transcription. In particular, the term gene may be intended for the genomic sequence encoding a protein, i.e. a sequence comprising regulator, promoter, intron and exon sequences.

The term "glycoprotein" is herein used in its normal scientific meaning and refers to a protein comprising one or more monosaccharide or oligosaccharide chains ("glycans") covalently bonded to the protein. A glycan may be attached to a hydroxyl group on the protein (O-linked-glycan), *e.g*. to the hydroxyl group of serine, threonine, tyrosine, hydroxylysine or hydroxyproline, or to an amide function on the protein (*N*-glycoprotein), *e.g*. asparagine or arginine, or to a carbon on the protein (*C*-glycoprotein), *e.g*. tryptophan. A glycoprotein may comprise more than one glycan, may comprise a combination of one or more monosaccharide and one or more oligosaccharide glycans, and may comprise a combination of N-linked, O-linked and C-linked glycans. It is estimated that more than 50% of all proteins have some form of glycosylation and therefore qualify as glycoprotein. Examples of glycoproteins include PSMA (prostate-specific membrane antigen), CAL (candida antartica lipase), gp41, gp120, EPO (erythropoietin), antifreeze protein and antibodies.

The term "glycan" is herein used in its normal scientific meaning and refers to a monosaccharide or oligosaccharide chain that is linked to a protein. The term glycan thus refers to the carbohydrate-part of a glycoprotein. The glycan is attached to a protein via the C-1 carbon of one sugar, which may be without further substitution (monosaccharide) or may be further substituted at one or more of its hydroxyl groups (oligosaccharide). A naturally occurring glycan typically comprises 1 to about 10 saccharide moieties. However, when a longer saccharide chain is linked to a protein, said saccharide chain is herein also considered a glycan. A glycan of a glycoprotein may be a monosaccharide. Typically, a monosaccharide glycan of a glycoprotein consists of a single N-acetylglucosamine (GIcNAc), glucose (Glc), mannose (Man) or fucose (Fuc) covalently attached to the protein. A glycan may also be an oligosaccharide. An oligosaccharide chain of a glycoprotein may be linear or branched. In an oligosaccharide, the sugar that is directly attached to the protein is called the core sugar. In an oligosaccharide, a sugar that is not directly attached to the protein and is attached to at least two other sugars is called an internal sugar. In an oligosaccharide, a sugar that is not directly attached to the protein but to a single other sugar, *i.e*. carrying no further sugar substituents at one or more of its other hydroxyl groups, is called the terminal sugar. For the avoidance of doubt, there may exist multiple terminal sugars in an oligosaccharide of a glycoprotein, but only one core sugar. A glycan may be an O-linked glycan, an N-linked glycan or a C-linked glycan. In an O-linked glycan a monosaccharide or oligosaccharide glycan is bonded to an O-atom in an amino acid of the protein, typically via a hydroxyl group of serine (Ser) or threonine (Thr). In an N-linked glycan a monosaccharide or oligosaccharide glycan is bonded to the protein via an N-atom in an amino acid of the protein, typically via an amide nitrogen in the side chain of asparagine (Asn) or arginine (Arg). In a C-linked glycan a monosaccharide or oligosaccharide glycan is bonded to a C-atom in an amino acid of the protein, typically to a C-atom of tryptophan (Trp).

The term "antibody" (AB) is herein used in its normal scientific meaning. An antibody is a protein generated by the immune system that is capable of recognizing and binding to a specific antigen. An antibody is an example of a glycoprotein. The term antibody herein is used in its broadest sense and specifically includes monoclonal antibodies, polyclonal antibodies, dimers, multimers, multispecific antibodies (e.g. bispecific antibodies), antibody fragments, and double and single chain antibodies. The term "antibody" is herein also meant to include human antibodies, humanized antibodies, chimeric antibodies and antibodies specifically binding cancer antigen. The term "antibody" is meant to include whole antibodies, but also fragments of an antibody, for example an antibody Fab fragment, F(ab')₂, Fv fragment or Fc fragment from a cleaved antibody, a scFv-Fc fragment, a minibody, a diabody or a scFv. Furthermore, the term includes genetically engineered antibodies and derivatives of an antibody. Antibodies, fragments of antibodies and genetically engineered antibodies may be obtained by methods that are known in the art.

An antibody may be a natural or conventional antibody in which two heavy chains are linked to each other by disulfide bonds and each heavy chain is linked to a light chain by a disulfide bond. There are two types of light chain, lambda (I) and kappa (k). The light chain includes two domains or regions, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties, such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The immunoglobulin can be of any type (e.g. IgG, IgE, IgM, IgD, and IgA), class (e.g. IgG1 , IgG2, IgG3, IgG4, IgA1 and IgA2) or subclass, or allotype (e.g. human G1m1 , G1 m2, G m3, non-G1m1 [that, is any allotype other than G1m1], G1m17, G2m23, G3m21 , G3m28, G3m1.1, G3m5, G3m13, G3m14, G3m10, G3m15, G3m16, G3m6, G3m24, G3m26, G3m27, A2m1, A2m2, Km1, Km2 and Km3) of immunoglobulin molecule. Preferred allotypes for administration include a non-G1m1 allotype (nG1m1), such as G1m17,1, G1m3, G1m3.1, G1m3.2 or G1m3.1.2. More preferably, the allotype is selected from the group consisting of the G1m17, 1 or G1m3 allotype. The antibody may be engineered in the Fc-domain to enhance or nihilate binding to Fc-gamma receptors, as summarized by Saunders et al. Front. Immunol. 2019, 10, doi: 10.3389/fimmu.2019.01296 and Ward et al., Mol. Immunol. 2015, 67, 131-141. For example, the combination of Leu234Ala and Leu235Ala (commonly called LALA mutations) eliminate FcyRIIa binding. Elimination of binding to Fc-gamma receptors can also be achieved by mutation of the N297 amino acid to any other amino acid except asparagine, by mutation of the T299 amino acid to any other amino acid except threonine or serine, or by enzymatic Deglycosylation or trimming of the fully glycosylated antibody with for example PNGase F or an endoglycosidase. The immunoglobulins can be derived from any species, including human, murine, or rabbit origin. Each chain contains distinct sequence domains.

A percentage of "sequence identity" may be determined by comparing the two sequences, optimally aligned over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. A sequence "at least 85% identical to a reference sequence" is a sequence having, on its entire length, 85%, or more, for instance 90%, 91%, 92%, 93%, 94%, 95%, 96%, 97%, 98% or 99% sequence identity with the entire length of the reference sequence.

The term "CDR" refers to complementarity-determining region: the specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs therefore refer to amino acid sequences which together define the binding affinity and specificity of the natural Fv region of a native immunoglobulin binding site. The light and heavy chains of an immunoglobulin each have three CDRs, designated CDR1-L, CDR2-L, CDR3-L and CDR1-H, CDR2-H, CDR3-H, respectively. A conventional antibody antigen-binding site, therefore, includes six CDRs, comprising the CDR set from each of a heavy and a light chain V region. "CDR"

The term "monoclonal antibody" or "mAb" as used herein refers to an antibody molecule of a single amino acid sequence, which is directed against a specific antigen, and is not to be construed as requiring production of the antibody by any particular method. A monoclonal antibody may be produced by a single clone of B cells or hybridoma, but may also be recombinant, i.e. produced by protein engineering.

The term "chimeric antibody" refers to an engineered antibody which, in its broadest sense, contains one or more regions from one antibody and one or more regions from one or more other antibodies. In an embodiment, a chimeric antibody comprises a VH domain and a VL domain of an antibody derived from a non-human animal, in association with a CH domain and a CL domain of another antibody, in an embodiment, a human antibody. As the non-human animal, any animal such as mouse, rat, hamster, rabbit or the like can be used. A chimeric antibody may also denote a multispecific antibody having specificity for at least two different antigens.

The term "humanised antibody" refers to an antibody which is wholly or partially of non-human origin and which has been modified to replace certain amino acids, for instance in the framework regions of the VH and VL domains, in order to avoid or minimize an immune response in humans. The constant domains of a humanized antibody are most of the time human CH and CL domains. "Fragments" of (conventional) antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "multivalent" refers to a linker molecule or linker part of a bigger molecule, with multiple connecting groups. For a linker molecule, these connecting groups are formed by reactive groups capable of covalently attaching to other molecules (e.g. payload or antibody). For a linker part of a bigger molecule, such as a conjugate, the connecting groups are covalent attachment to other parts of the molecule (e.g. payload or antibody). A bivalent linker has two connecting groups, a trivalent linker has three connecting groups, etc. In the context of the current invention, bivalent is also referred to as 2-valent, trivalent is also referred to a 3-valent, etc.

The term "heterofunctional" refers to a linker molecule or linker part of a bigger molecule with connecting groups that are not identical and have different reactivity or are obtained by different reactivity. Typically, such connecting groups in a heterofunctional molecule are mutually nonreactive.

### The invention

The inventors have for the first time been able to prepare homogenous antibody-conjugates with high payload loading (high DAR) by site-specific conjugation to a single antibody N-glycan. The conjugates according to the invention are homogeneous, i.e. have a DAR at or close to the theoretical DAR with a narrow distribution, and do not require any genetic modification of the antibody. The conjugates according to the invention have a payload-to-antibody ratio (DAR) of 6 or higher, preferably 8 or higher. Conjugates with a DAR up to 64 can be readily prepared.

The inventors have developed a modular, non-genetic preparation method for such conjugates, involving three simple steps and starting from any antibody. These steps are (a) enzymatic remodeling of the glycan to give an antibody functionalized with two or four click probes (e.g. an azide) per antibody, (b) strain-promoted azide-alkyne cycloaddition with a multivalent, bifunctional reagent comprising one cyclic alkyne and at least two click probes that are not reactive towards the cyclic alkyne (e.g. tetrazines), and (c) inverse electron-demand Diels-Alder cycloaddition of the click probes with branched linker-drug constructs comprising one cyclic alkyne or strained alkene, connected to one or more payloads preferably connected through a cleavable linker. The resulting conjugates with DAR6 or higher are rapidly generated with high homogeneity and with surprising stability. In addition, HIC profiles of the resulting ADCs indicate small relative retention time and therefore show high potential in the treatment of cancer.

In a first aspect, the invention concerns a process for preparing an antibody-payload-conjugate having a payload-to-antibody ratio (DAR) of 6 or higher, comprising:
(a) providing a modified antibody having the structure Ab(F¹)_{z}, wherein Ab is an antibody, z is 2 or 4 and F¹ is a click probe;
(b) reacting the modified antibody with z equivalents of Q¹L^{A}(F²)_{y}, wherein Q¹ is a click probe that is reactive towards F¹, L^{A} is a heterobifunctional (y + 1)-valent linker, y is 2, 3 or 4, and F¹ is a click probe that is not reactive towards Q¹, to obtain an antibody-linker construct of structure Ab(Z¹L^{A}(F²)_{y})_{z}, wherein Z¹ is a connecting group obtained by reaction of F¹ and Q¹;
(c) reacting the antibody-linker construct with _{z} × y equivalents of Q²(L^{B})Dₓ, wherein Q² is a click probe that is reactive towards F², L^{B} is a (x + 1)-valent linker, x is 1, 2, 3 or 4, provided that x + y is at least 4, and D is a payload molecule, to obtain the conjugate of structure Ab(Z¹L^{A}(Z²(L^{B})Dₓ)_{y})_{z}, wherein Z² is a connecting group obtained by reaction of F² and Q².

In a second aspect, the invention concerns conjugates obtainable by the process according to the first aspect of the invention. Alternatively, the conjugates according to the second aspect can also be defined as having structure Ab(Z¹L^{A}(Z²(L^{B})Dₓ)_{y})_{z}, wherein:
- Ab is an antibody;
- Z¹ and Z² are connecting groups obtainable by reaction between two click probes;
- x is 1, 2, 3 or 4;
- y is 2, 3 or 4, wherein x + y is at least 4;
- z is 2 or 4;
- L^{A} is an heterobifunctional (y + 1)-valent linker; and
- L^{B} is an heterobifunctional (x + 1)-valent linker; and
- D is a payload.

The conjugates according to the second aspect of the invention are ideally suited for the targeting of tumour cells, in particular for the treatment of cancer. Therefore, the invention also concerns a method for targeting a tumour cell expressing a specific extracellular receptor, comprising contacting the conjugate according to the second aspect of the invention with cells that may possibly express the extracellular receptor, wherein the antibody specifically targets the extracellular receptor. Likewise, the invention also concerns a method for the treatment of cancer, comprising administering to a subject in need thereof the conjugate according to the second aspect of the invention, wherein the cancer cells specifically express an extracellular receptor.

The invention according to the present aspect further concerns a pharmaceutical composition comprising the conjugate according to the second aspect of the invention and a pharmaceutically acceptable carrier.

In a third aspect, the invention concerns heterobifunctional linkers having structure Q¹L^{A}(F²)_{y}, wherein:
- Q¹ is a click probe;
- L^{A} is a heterobifunctional (y + 1)-valent linker;
- y is 2, 3 or 4; and
- F¹ is a click probe that is not reactive towards Q¹.

The heterobifunctional linkers according to the third aspect of the invention are ideally suited as intermediates in the synthesis of the conjugates according to the second aspect.

Also contemplated within the present invention are salts, preferably pharmaceutically acceptable salts, of the conjugates and heterobifunctional linkers according to the invention.

Here below, the conjugate according to the second aspect is first defined. The structural features of the conjugate according to the invention also apply to the heterobifunctional linkers according to the invention and the process for preparing an antibody-payload-conjugate according to the invention. As the skilled person will appreciate, the structural features of the conjugates according to the invention also apply to the heterobifunctional linkers according to the invention. The skilled person understands that any structure feature that is unchanged in the conjugation reaction is defined equally for each of the molecules according to the invention. In the conjugation reactions, only reactive moieties F and Q are transformed into connecting groups Z. As will be understood by the skilled person, the definition of the chemical moieties, as well as their preferred embodiments, apply to all aspects of the invention.

### Conjugate of general structure (2)

The invention concerns conjugates of general structure (**2**):

Ab[Z¹L^{A}(Z²(L^{B})Dₓ)_{y}]_{z} (**2**)

wherein:
- Ab is an antibody;
- Z¹ and Z² are connecting groups obtainable by reaction between two click probes;
- x is 1, 2, 3 or 4;
- y is 2, 3 or 4, wherein x + y is at least 4;
- z is 2 or 4;
- L^{A} is an heterobifunctional (y + 1)-valent linker; and
- L^{B} is an heterobifunctional (x + 1)-valent linker; and
- D is a payload.

### The antibody Ab

Ab is an antibody. Antibodies are known in the art and include IgA, IgD, IgE, IgG, IgM, Fab, VHH, scFv, diabody, minibody, affibody, affylin, affimers, atrimers, fynomer, Cys-knot, DARPin, adnectin/centryin, knottin, anticalin, FN3, Kunitz domain, OBody, bicyclic peptides and tricyclic peptides. Preferably, the antibody is a monoclonal antibody, more preferably selected from the group consisting of IgA, IgD, IgE, IgG and IgM antibodies. Even more preferably Ab is an IgG antibody. The IgG antibody may be of any IgG isotype. The antibody may be any IgG isotype, e.g. IgG1, IgG2, Igl3 or IgG4. Preferably Ab is a full-length antibody, but Ab may also be a Fc fragment.

The antibody Ab is typically specific for an extracellular receptor on a tumour cell, preferably wherein the extracellular receptor on the tumour cell is selected from the group consisting of 5T4, ADAM-9, AMHRII, ASCT2, ASLG659, ASPHD1, av-integrin, Axl, B7-H3, B7-H4, BAFF-R, BCMA, BMPR1B, Brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha, Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSCA hlg, PSMA, PTK7, RET, RNF43, RON, ROR1, ROR2, Sema 5b, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin.

The antibody may also be specific to an extracellular protein resulting from a viral infection, *e.g*. human polio virus (HPV), human cytomegalovirus (HCMV) or human papillomavirus (HPV). The antibody may also be specific for a tumour-associated carbohydrate antigen (TACA) that is selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le^{a}), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis" (Le^{x}), Sialyl-Lewis^{x} (SLe^{x}), 6-Sialyl-Lewis^{x} (6SLe^{x}), Lewis^{y} (Le^{y}), Sialyl-Lewis^{y} (SLe^{y}), 6-Sialyl-Lewis^{y} (6SLe^{y}) and or combinations thereof. The antibody may also be specific to both an extracellular protein and a TACA at the same time.

The number of payloads D attached to a single antibody is known in the art as the DAR (drug-to-antibody ratio). Even though DAR values up to 64 are possible in the context of the present invention, it is preferred that DAR is an even integer in the range 6 - 32, more preferably 6, 8, 10 or 12, most preferably DAR = 8. It will be appreciated that these are theoretical DAR values, and in practice the DAR may slightly deviate from this value, by virtue of incomplete conjugation. Thus, the conjugates may be obtained as mixture of conjugates with varying DAR. In such cases, DAR often refers to the average DAR of the mixture. This is well-known in the art of bioconjugation. The conjugation technique used in the present invention, conjugating site-specifically via the glycan of the antibody and using click reactions, provides conjugates with a narrow distribution with DAR values close to the theoretical value and with narrow standard deviation. For example, when the theoretical DAR is 8, DAR values above 7.4 or even above 7.6 are readily obtained, indicating that most antibodies in the reaction mixture have reacted completely and have a DAR of 8. No antibody conjugates with a DAR above 8 will be present.

In one embodiment of the conjugates according to the invention, only the conserved glycosylation site is used for conjugation as described below, i.e. z = 2, and the antibody is functionalized with 2 × x × y occurrences of payload D. Herein, x is 1, 2, 3 or 4 and y is 2, 3 or 4, wherein x + y is at least 4. Thus x + y is an integer in the range 4-8. The exact number of x and y determines the DAR. In one preferred embodiment, y = 2 and x = 1, 2, 3 or 4, preferably x = 2, 3 or 4, more preferably x = 2. In another preferred embodiment, x = 2 and y = 2, 3 or 4, preferably y = 2 or 4, more preferably y = 2. In another preferred embodiment, x = y, preferably x = y = 2 or 4, more preferably x = y = 2.

In an alternative embodiment of the conjugates according to the invention, the conserved glycosylation site and a second glycosylation site are both used for conjugation as described below, i.e. z = 4, and the antibody is functionalized with 4 × x × y occurrences of payload D. Herein, x is 1, 2, 3 or 4 and y is 2, 3 or 4, wherein x + y is at least 4. Thus x + y is an integer in the range 4-8. The exact number of x and y determines the DAR. In one preferred embodiment, y = 2 and x = 1, 2, 3 or 4, preferably x = 1, 2 or 3, more preferably x = 2. In another preferred embodiment, x = 1 and y = 2, 3 or 4, preferably y = 2 or 4, more preferably y = 2. In another preferred embodiment, x = y, preferably x = y = 2 or 4, more preferably x = y = 2.

With these values of z, x and y, conjugates with a DAR in the range of DAR 6 - 64 are readily formed. Conjugates according to the invention have an even DAR. In a preferred embodiment, the DAR = 6, with z = 2, x = 1 and y = 3. In another preferred embodiment, the DAR = 8, with z = 2, x = 2 and y = 2. In another preferred embodiment, the DAR = 8, with z = 2, x = 1 and y = 4. In another preferred embodiment, the DAR = 12, with z = 2, x = 2 and y = 3. In another preferred embodiment, the DAR = 16, with z = 2, x = 2 and y = 4. In another preferred embodiment, the DAR = 32, with z = 2, x = 4 and y = 4. In another preferred embodiment, the DAR = 24, with z = 2, x = 3 and y = 4 or with z = 2, x = 4 and y = 3. In another preferred embodiment, the DAR = 12, with z = 4, x = 1 and y = 3. In another preferred embodiment, the DAR = 16, with z = 4, x = 2 and y = 2. In another preferred embodiment, the DAR = 16, with z = 4, x = 1 and y = 4. Preferred conjugates have a DAR in the range of 6 - 32, more preferably in the range of 6 - 16, even more preferably in the range of 6 -12. In a further preferred embodiment, the DAR is at least 8, preferably the DAR is in the range of 8 - 32, more preferably in the range of 8 - 16, even more preferably in the range of 8 - 12. Most preferably, the DAR = 8.

The conjugates according to the invention contain two connecting groups Z¹, which is formed during a click reaction wherein the antibody of structure Ab(F¹)_{z} and a heterobifunctional (y + 1)-valent linker construct of structure of Q¹L^{A}(F²)_{y}. In a first click reaction, F¹ and Q¹ react to form a covalent connection between the antibody and z × y moieties F² by forming connecting groups Z¹.

Herein, "(y + 1)-valent" refers to the number of connecting points, being either a reactive group F or Q (before reaction) or a connecting group Z (after reaction). Thus, in case y = 2, the linker construct contains one click probe Q¹ and two click probes F² and the linker has a valency of 2 + 1 = 3. Thus, when y = 2, the L^{A} is trivalent. Thus, in case y = 3, the linker construct contains one click probe Q¹ and three click probes F² and the linker has a valency of 3 + 1 = 4. Thus, when y = 3, the L^{A} is tetravalent. Thus, in case y = 4, the linker construct contains one click probe Q¹ and four click probes F² and the linker has a valency of 4 + 1 = 5. Thus, when y = 4, the L^{A} is pentavalent.

Part of the antibody may be a linker L⁶ that connects the click probe F¹ or connecting group Z¹ to the peptide part of the cell-binding agent. Preferably, the connecting group Z¹ is connected to the cell-binding agent CB via a glycan of Ab. Thus, the conjugate according to the invention is preferably represented by:

Ab[(L⁶)Z¹L^{A}(Z²(L^{B})Dₓ)_{y})]_{z}, (3)

wherein:
- L⁶ is -GlcNAc(Fuc)_{w}-(G)ⱼ-S-(L⁷)_{w'}-, wherein G is a monosaccharide, j is an integer in the range of 0 - 10, S is a sugar or a sugar derivative, GlcNAc is N-acetylglucosamine and Fuc is fucose, w is 0 or 1, w' is 0 or 1 and L⁷ is -N(H)C(O)CH₂-, -N(H)C(O)CF₂- or -CH₂-.

### Linker L⁶

Linker L⁶ is preferably present, wherein reactive group F¹ may be introduced at a specific position of the antibody. This is for example the case for conjugation via an artificially introduced reactive group F¹, such as for example using transglutaminase, using sortase or by enzymatic glycan modification (e.g. glycosyltransferase or α-1,3-mannosyl-glycoprotein-2-β-N-acetylglucosaminyl-transferase). For example, a modified sugar residue S(F¹)₂ may be introduced at the glycan, extending the glycan with one monosaccharide residue S, which introduces two reactive groups F¹ on the glycan of an antibody. In a most preferred embodiment, conjugation occurs via the glycan of the antibody. The site of conjugation is preferably at the heavy chain of the antibody.

All recombinant antibodies, generated in mammalian host systems, contain the conserved N-glycosylation site at the asparagine residue at or close to position 297 of the heavy chain (Kabat numbering), which is modified by a glycan of the complex type. This naturally occurring glycosylation site of antibodies is preferably used, but other glycosylation sites, including artificially introduced ones, may also be used for the connection of linker L⁶. Thus, in a preferred embodiment, L⁶ is connected to an amino acid of the antibody which is located at a position in the range of 250 - 350 of the heavy chain, preferably in the range of 280 - 310 of the heavy chain, more preferably in the range of 295 - 300 of the heavy chain, most preferably at position 297 of the heavy chain. Using this conserved glycosylation position of the antibody, the obtained conjugates are formed as symmetrical dimers, wherein each half antibody contains one F², and both click probes F² will form an arm with 3 or more payloads. Some antibodies may have a second glycosylation site per half antibody, which is not used as conjugation site in the embodiment where z = 2. The skilled person is able to perform the enzymatic conversions in such a way that only the main glycosylation site is utilized for conjugation. Alternatively, the skilled person is able to perform the enzymatic conversion in such a way that also the second glycosylation site is utilized for conjugation, thereby doubling the DAR of the antibody-drug conjugate. In this embodiment, z = 4 and each half antibody contains two occurrences of F², and all four click probes F² will form an arm with 3 or more payloads.

L⁶ is a linker that connects Ab to F¹ or Z¹, and is represented by -GlcNAc(Fuc)_{w}-(G)ⱼ-S-(L ⁷)_{w'}-, wherein G is a monosaccharide, j is an integer in the range of 0 - 10, S is a sugar or a sugar derivative, GlcNAc is N-acetylglucosamine and Fuc is fucose, w is 0 or 1, w' is 0 or 1 and L⁷ is - N(H)C(O)CH₂-, -N(H)C(O)CF₂- or-CHz-. Typically, L⁶ is at least partly formed by the glycan of an antibody.

The -GlcNAc(Fuc)_{w}-(G)ⱼ- of L⁶ is the glycan, or part thereof. The -GlcNAc(Fuc)_{w}-(G)ⱼ- of the glycan thus typically originates from the original antibody, wherein GlcNAc is an *N-*acetylglucosamine moiety and Fuc is a fucose moiety. Fuc is typically bound to GlcNAc via an α-1,6-glycosidic bond. Normally, antibodies may (w = 1) or may not be fucosylated (w = 0). In the context of the present invention, the presence of a fucosyl moiety is irrelevant, and similar effects are obtained with fucosylated (w = 1) and non-fucosylated (w = 0) antibody conjugates. The GIcNAc residue may also be referred to as the core-GIcNAc residue and is the monosaccharide that is directly attached to the peptide part of the antibody.

S may be directly connected to the core-GlcNAc(Fuc)_{w} moiety, i.e. j = 0, meaning that the remainder of the glycan is removed from the core-GlcNAc(Fuc)_{w} moiety before S is attached. Such trimming of glycans is well-known in the art and can be achieved by the action of an endoglycosidase. Alternatively, there are one or more monosaccharide residues present in between the core-GlcNAc(Fuc)_{w} moiety and S, i.e. j is an integer in the range of 1 - 10, preferably j = 1-5. In one preferred embodiment, (G)ⱼ is an oligosaccharide fraction comprising j monosaccharide residues G, wherein j is an integer in the range of 2 - 5. In another preferred embodiment, (G); is a monosaccharide fraction comprising j monosaccharide residues G, wherein j is 1. (G)ⱼ is connected to the GIcNAc moiety of GlcNAc(Fuc)_{w}, typically via a β-1,4 bond. In a preferred embodiment, j is 0, 1, 3, 4 or 5, more preferably, j is 0 or 1, most preferably j is 0.

Although any monosaccharide that may be present in a glycan may be employed as G, each G is preferably individually selected from the group consisting of galactose, glucose, *N-*acetylgalactosamine, *N*-acetylglucosamine, mannose and *N*-acetylneuraminic acid. More preferred options for G are galactose, *N*-acetylglucosamine, mannose. In case j = 1, it is preferred that G = galactose and S = *N*-acetylneuraminic acid.

When j is 3 - 10, (G)ⱼ may be linear or branched. Preferred examples of branched oligosaccharides (G)ⱼ are (**a**), (**b**), (**c**), (**d**), (**e**), (**f**), (**h**) and (**h**) as shown below.

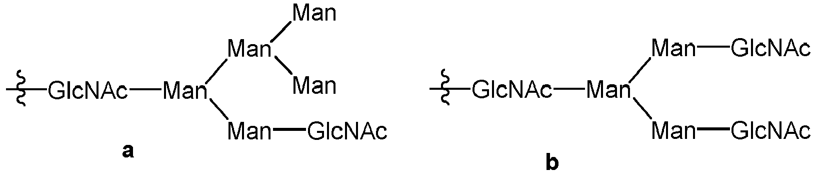

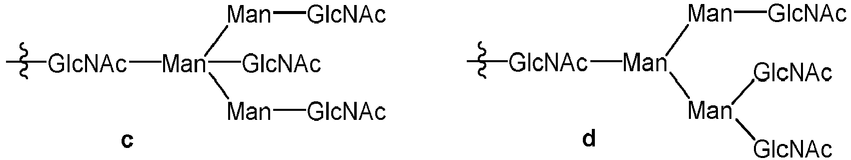

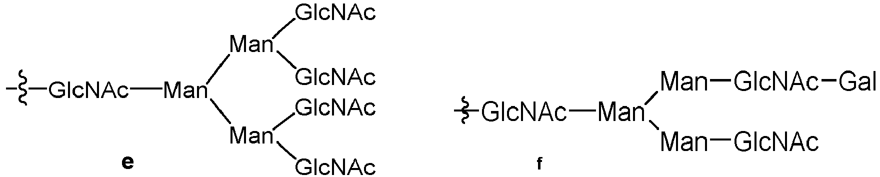

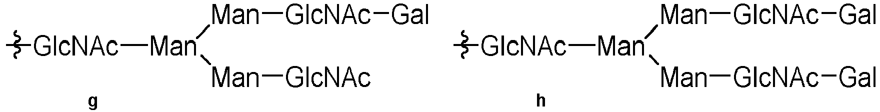

In case (G)ⱼ is present with j > 2, it is preferred that it ends in GIcNAc or Gal, preferably GlcNAc. In other words, the monosaccharide residue directly connected to S is preferably GIcNAc or Gal. The presence of a GIcNAc moiety facilitates the synthesis of the functionalized antibody, as monosaccharide derivative S can readily be introduced by glycosyltransfer onto a terminal GIcNAc residue. The presence of a Gal moiety facilitates the synthesis of the functionalized antibody, as monosaccharide derivative S sialic acid can readily be introduced by sialyltransferase onto a terminal Gal residue. In the above preferred embodiments for (G)ⱼ, having structure (**a**) - (**h**), moiety S may be connected to any of the terminal GlcNAc residues, i.e. not the one with the wavy bond, which is connected to the core GlcNAc residue on the antibody.

Antibodies and antibody conjugates having j = 0 or 1 show no or significantly reduced binding to Fc-gamma receptors, while antibodies and antibody conjugates having j in the range of 4 - 10 do bind to Fc-gamma receptors. Thus, by selecting a certain value for j, the desired extent of binding to Fc-gamma receptors can be obtained. It is thus preferred that j = 0, 1, 4, 5, 6, 7, 8, 9 or 10, more preferably j = 0, 1, 4 or 5, most preferably the antibody is trimmed and j = 0.

S is a sugar or sugar derivative. The term "sugar derivative" is herein used to indicate a derivative of a monosaccharide sugar, i.e. a monosaccharide sugar comprising substituents and/or functional groups. Suitable examples for S include glucose (Glc), galactose (Gal), mannose (Man), fucose (Fuc), amino sugars and sugar acids, e.g. glucosamine (GlcNH₂), galactosamine (GalNH₂) N-acetylglucosamine (GIcNAc), N-acetylgalactosamine (GaINAc), sialic acid (Sia) which is also referred to as N-acetylneuraminic acid (NeuNAc), and N-acetylmuramic acid (MurNAc), glucuronic acid (GlcA) and iduronic acid (IdoA). Preferably, S is selected from Gal, GalNAc and NeuNAc. In an especially preferred embodiment, S is GaINAc.

Connecting group Z¹ or reactive group F¹ may be attached directly to S, or there may be a linker L⁷ present in between S and Z¹ or F¹. Thus, L⁷ may be present (w' = 1 or 2) or absent (w' = 0). Typically, each moiety Z may be connected to S via a linker L⁷, thus in one embodiment w' = 0 of x. Preferably, L⁷ is absent and each connecting moiety Z is directly attached to S. If present, L⁷ may be selected from -N(H)C(O)CH₂-, -N(H)C(O)CF₂- or -CH₂-. In a preferred embodiment, x = 1 and w' = 0 or 1, most preferably x = 1 and w' = 0.

### Connecting group Z¹ and Z²

Z¹ and Z² are connecting groups, which covalently connect the antibody with the payloads of the conjugate according to the invention. The term "connecting group" herein refers to the structural element, resulting from a reaction, here between Q and F, connecting one part of the conjugate with another part of the same conjugate. Z¹ is formed by a click reaction between Q¹ and F¹. Likewise, Z² is formed by a click reaction between Q² and F². Herein, Z refers to Z¹ and Z², Q refers to Q¹ and Q², and F refers to . F¹ and F²

As will be understood by the person skilled in the art, the exact nature of the connecting group depends on the exact structure of the click probes Q and F. The skilled person is aware of complementary click probes which are reactive towards each other and form suitable reaction partners Q¹/F¹ and Q²/F². For example, when F comprises or is an alkynyl group, complementary groups Q include azido groups. For example, when F comprises or is an azido group, complementary groups Q include alkynyl groups. For example, when F comprises or is a cyclopropenyl group, a trans-cyclooctene group, a cycloheptyne or a cyclooctyne group, complementary groups Q include tetrazinyl groups. In these particular cases, Z is only an intermediate structure and will expel N₂, thereby generating a dihydropyridazine (from the reaction with alkene) or pyridazine (from the reaction with alkyne) as shown in Figure 4.

Connecting groups Z are obtained by a cycloaddition reaction, preferably wherein the cycloaddition is a [4+2] cycloaddition or a 1,3-dipolar cycloaddition. Conjugation reactions via cycloadditions are known to the skilled person, and the skilled person is capable of selecting appropriate reaction partners F and Q, and will understand the nature of the resulting connecting group Z. Preferred cycloadditions are a [4+2]-cycloaddition (e.g. a Diels-Alder reaction) or a [3+2]-cycloaddition (e.g. a 1,3-dipolar cycloaddition). Preferably, the cycloaddition is the Diels-Alder reaction or the 1 ,3-dipolar cycloaddition. The preferred Diels-Alder reaction is the inverse electron-demand Diels-Alder cycloaddition. In another preferred embodiment, the 1,3-dipolar cycloaddition is used, more preferably the alkyne-azide cycloaddition. Cycloadditions, such as Diels-Alder reactions and 1,3-dipolar cycloadditions are known in the art, and the skilled person knows how to perform them.

Preferably, Z contains a moiety selected from the group consisting of a triazole, a cyclohexene, a cyclohexadiene, a [2.2.2]-bicyclooctadiene, a [2.2.2]-bicyclooctene, an isoxazoline, an isoxazolidine, a pyrazoline, a piperazine, a thioether, an amide or an imide group. Triazole moieties are especially preferred to be present in Z. In one embodiment, Z comprises a (hetero)cycloalkene moiety, i.e. formed from Q comprising a (hetero)cycloalkyne moiety. In an alternative embodiment, Z comprises a (hetero)cycloalkane moiety, i.e. formed from Q comprising a (hetero)cycloalkene moiety. Herein, aromatic rings such as a triazole ring are considered a heterocycloalkane ring, since it is formed by reaction of an alkyne moiety and an azide moiety.

In a preferred embodiment, Z has the structure (Z1): 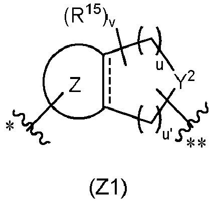 Herein, the bond depicted as --- is a single bond or a double bond. Furthermore:
- ring Z is obtained by a cycloaddition, preferably ring Z is selected from (Za) - (Zm) defined below, wherein the carbon atoms labelled with ** correspond to the two carbon atoms of the bond depicted as --- of (Z1) to which ring Z is fused;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;

- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
- v = an integer in the range 8-16;
- Ring Z is formed by the cycloaddition, and is preferably selected from (Za) - (Zm).

In a preferred embodiment, u + u' = 0, 4, 5, 6, 7 or 8, more preferably 0, 4 or 5. In case the bond depicted as --- is a double bond, it is preferred that u + u' = 4, 5, 6, 7 or 8, more preferably u + u' = 4 or 5. In case the bond depicted as --- is a single bond, it is preferred that u + u' = 0 or 5. Preferably, the wavy bond labelled with * is connected to CB, optionally via L⁶, and the wavy bond labelled with ** is connected to L.

It is especially preferred that Z comprises a (hetero)cycloalkene moiety, i.e. the bond depicted as - - - is a double bond. In a preferred embodiment, Z is selected from the structures (Z2) - (Z20), depicted here below:

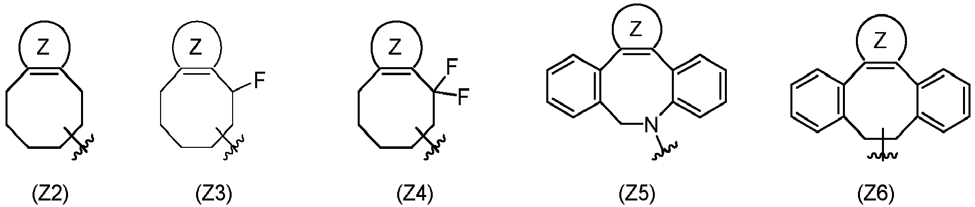

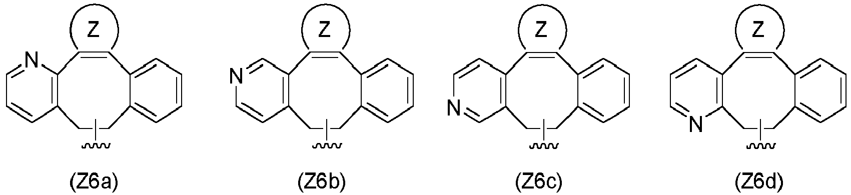

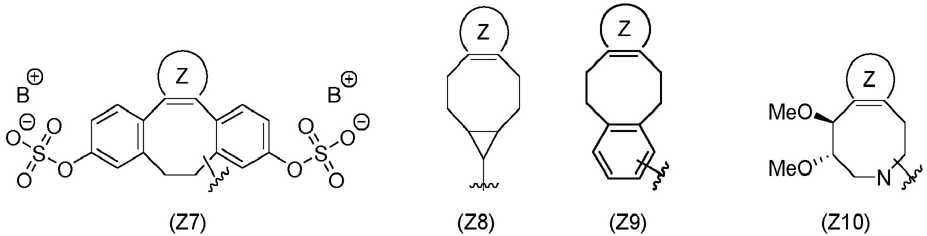

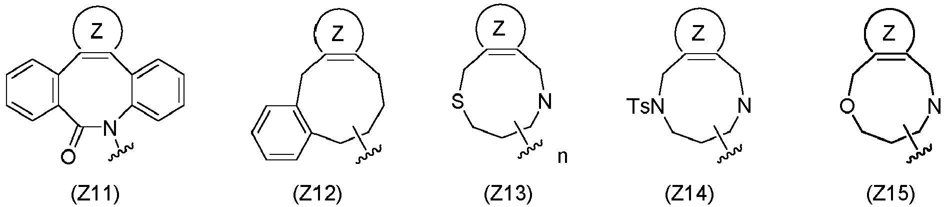

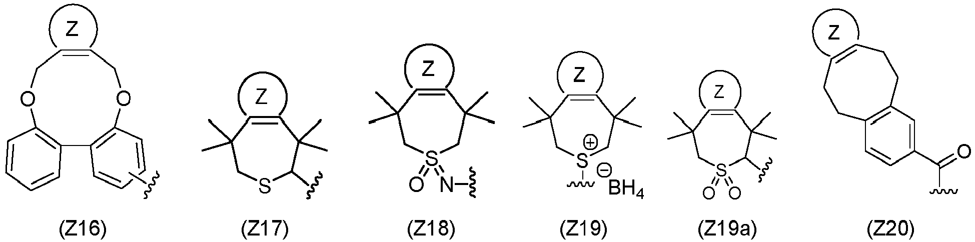

Herein, the connection to L is depicted with the wavy bond. B⁽⁻⁾ is an anion, preferably a pharmaceutically acceptable anion. B⁽⁺⁾ is a cation, preferably a pharmaceutically acceptable cation. Ring Z is formed by the cycloaddition reaction, and preferably is a triazole, a cyclohexene, a cyclohexadiene, a [2.2.2]-bicyclooctadiene, a [2.2.2]-bicyclooctene, an isoxazoline, an isoxazolidine, a pyrazoline or a piperazine. Most preferably, ring Z is a triazole ring. Ring Z may have the structure selected from (Za) - (Zm) depicted below, wherein the carbon atoms labelled with ** correspond to the two carbon atoms of the (hetero)cycloalkane ring of (Z2) - (Z20), to which ring Z is fused. Since the connecting group Z is formed by reaction with a (hetero)cycloalkyne in the context of the present embodiment, the bond depicted above as --- is a double bond.

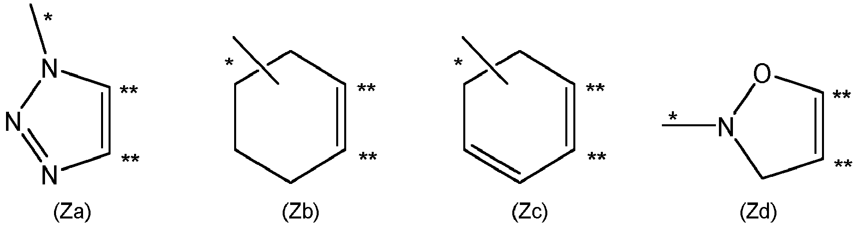

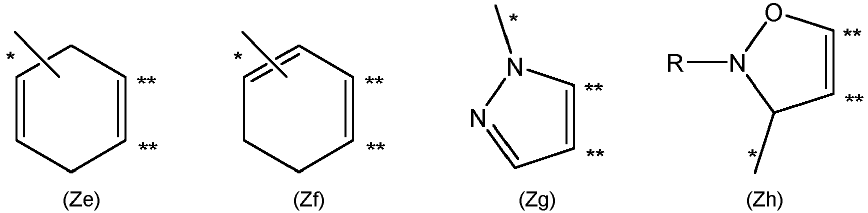

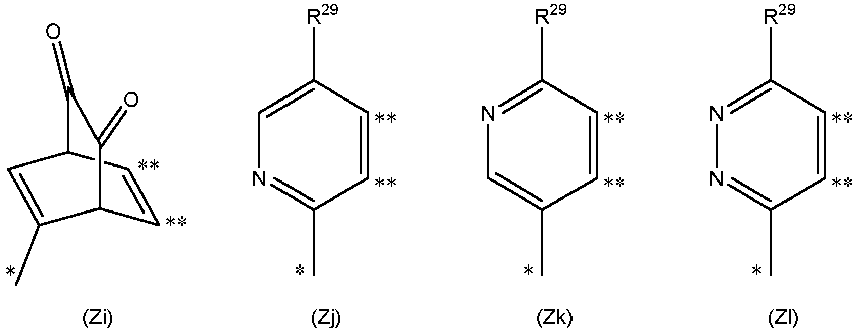

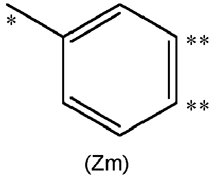

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction, especially in case ring (Zl) is formed. Thus, in a preferred embodiment, ring Z is (Zl) wherein R²⁹ is selected from hydrogen, methyl, phenyl, pyridyl, pyridinyl and pyrimidinyl, more preferably R²⁹ is hydrogen.

In case Z comprises a (hetero)cycloalkene moiety, it is preferred that ring Z is selected from (Za), (Zj), (Zk) or (Zl), more preferably ring Z is according to structure (Za) or (Zl).

In a further preferred embodiment, Z is selected from the structures (Z21) - (Z38a), depicted here below:

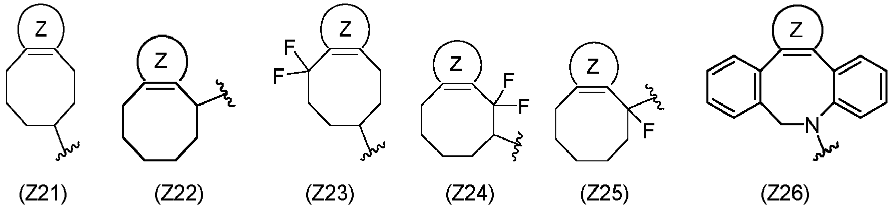

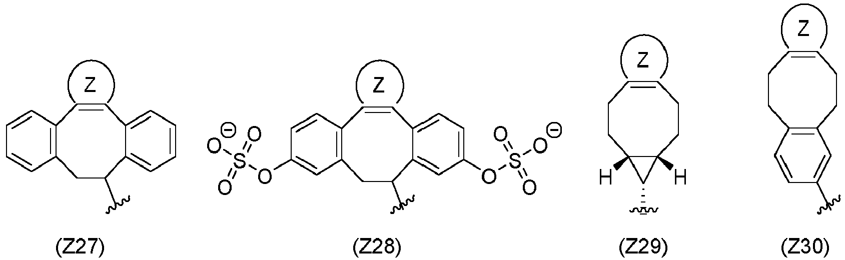

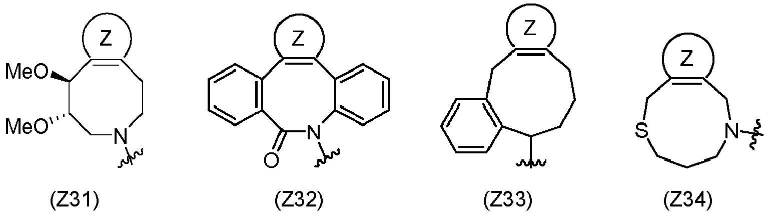

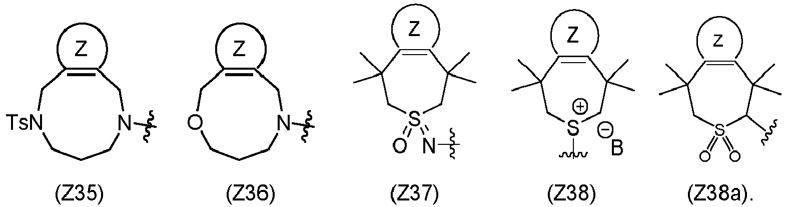

Herein, the connection to L is depicted with the wavy bond. Structure (Z29) can be in endo or exo configuration, preferably it is in endo configuration. In structure (Z38), B⁽⁻⁾ is an anion, preferably a pharmaceutically acceptable anion. Ring Z is selected from structures (Za) - (Zm), as defined above.

In a preferred embodiment, Z comprises a (hetero)cyclooctene moiety or a (hetero)cycloheptene moiety, preferably according to structure (Z8), (Z26), (Z27), (Z28), (Z37) or (Z38a), which are optionally substituted. Each of these preferred options for Z are further defined here below.

Thus, in a preferred embodiment, Z comprises a heterocycloheptene moiety according to structure (Z37), which is optionally substituted. Preferably, the heterocycloheptene moiety according to structure (Z37) is not substituted.

In a preferred embodiment, Z comprises a (hetero)cyclooctene moiety according to structure (Z8), more preferably according to (Z29), which is optionally substituted. Preferably, the cyclooctene moiety according to structure (Z8) or (Z29) is not substituted. In the context of the present embodiment, Z preferably comprises a (hetero)cyclooctene moiety according to structure (Z39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Z39), I is most preferably 1. Most preferably, Z is according to structure (Z42), defined further below.

In an alternative preferred embodiment, Z comprises a (hetero)cyclooctene moiety according to structure (Z26), (Z27) or (Z28), which is optionally substituted. In the context of the present embodiment, Z preferably comprises a (hetero)cyclooctene moiety according to structure (Z40) or (Z41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Z40) are optionally O-sulfated at one or more positions, whereas the rings of (Z41) may be halogenated at one or more positions. Preferably, the (hetero)cyclooctene moiety according to structure (Z40) or (Z41) is not further substituted. Most preferably, Z is according to structure (Z43), defined further below.

In an alternative preferred embodiment, Z comprises a heterocycloheptenyl group and is according to structure (Z37).

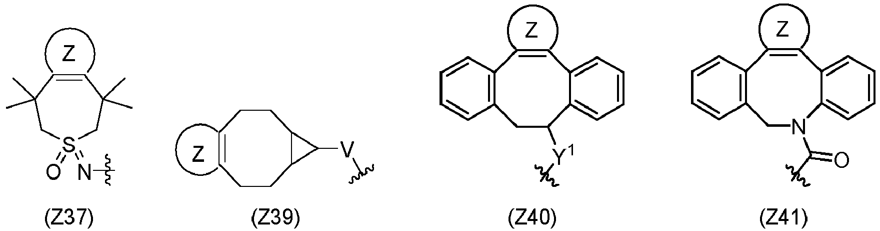

In an especially preferred embodiment, Z comprises a cyclooctenyl group and is according to structure (Z42): 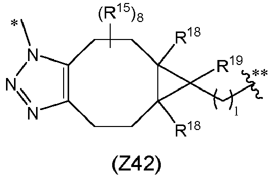 Herein:
- the bond labelled with * is connected to CB and the wavy bond labelled with ** is connected to L;
- R¹⁵ is independently selected from the group consisting of hydrogen, halogen, -OR¹⁶, -NO₂, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Z (or Q) or D connected via a spacer moiety; and
- I is an integer in the range 0 to 10.

In a preferred embodiment of the group according to structure (Z42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the group according to structure (Z42), R¹⁸ is independently selected from the group consisting of hydrogen, C₁ - C₆ alkyl groups, most preferably both R¹⁸ are H. In a preferred embodiment of the group according to structure (Z42), R¹⁹ is H. In a preferred embodiment of the group according to structure (Z42), I is 0 or 1, more preferably I is 1.

In an especially preferred embodiment, Z comprises a (hetero)cyclooctenyl group and is according to structure (Z43): 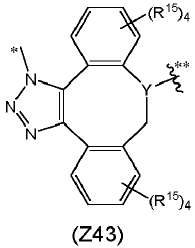 Herein:
- the bond labelled with * is connected to CB and the wavy bond labelled with ** is connected to L;
- R¹⁵ is independently selected from the group consisting of hydrogen, halogen, -OR¹⁶, -NO₂, -CN, -S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵;
- a carbon atom in the fused aromatic rings may be replaced by a nitrogen atom, as in (Z6a) - (Z6d), preferably wherein Y is CR¹⁵.

In a preferred embodiment of the group according to structure (Z43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the group according to structure (Z43), Y is N or CH, more preferably Y = N.

In an especially preferred embodiment, Z comprises a heterocycloheptenyl group and is according to structure (Z37) or (Z38a), wherein ring Z is a triazole.

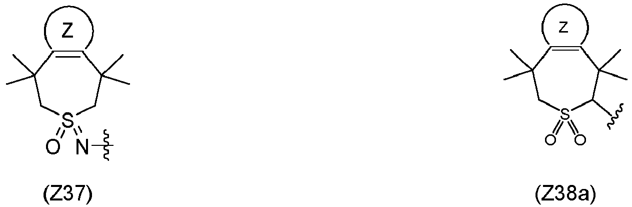

In an alternative preferred embodiment, connecting group Z comprises a (hetero)cycloalkane moiety, i.e. the bond depicted as - - - is a single bond. The (hetero)cycloalkane group may also be referred to as a heterocycloalkyl group or a cycloalkyl group, preferably a cycloalkyl group, wherein the (hetero)cycloalkyl group is optionally substituted. Preferably, the (hetero)cycloalkyl group is a (hetero)cyclopropyl group, a (hetero)cyclobutyl group, a norbornyl group, a norbornenyl group, a (hetero)cycloheptyl group, a (hetero)cyclooctyl group, which may all optionally be substituted. Especially preferred are (hetero)cyclopropyl groups, (hetero)cycloheptyl group or (hetero)cyclooctyl groups, wherein the (hetero)cyclopropyl group, the (hetero)cycloheptyl group or the (hetero)cyclooctyl group is optionally substituted. Preferably, Z comprises a cyclopropyl moiety according to structure (Z44), a hetereocyclobutane moiety according to structure (Z45), a norbornane or norbornene group according to structure (Z46), a (hetero)cycloheptyl moiety according to structure (Z47) or a (hetero)cyclooctyl moiety according to structure (Z48). Herein, Y³ is selected from C(R²³)₂, NR²³ or O, wherein each R²³ is individually hydrogen, C₁ - C₆ alkyl or is connected to L, optionally via a spacer, and the bond labelled --- is a single or double bond. In a further preferred embodiment, the cyclopropyl group is according to structure (Z49). In another preferred embodiment, the (hetero)cycloheptane group is according to structure (Z50) or (Z51). In another preferred embodiment, the (hetero)cyclooctane group is according to structure (Z52), (Z53), (Z54), (Z55) or (Z56).

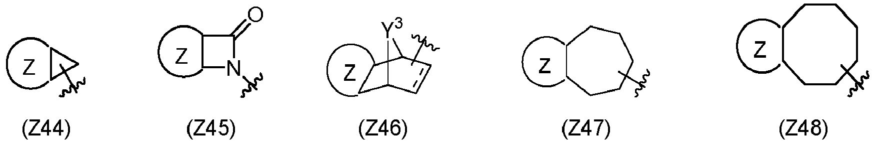

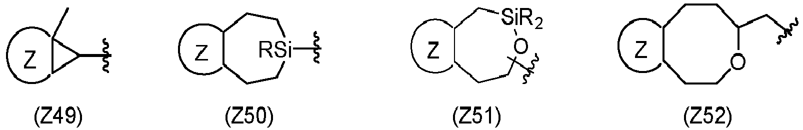

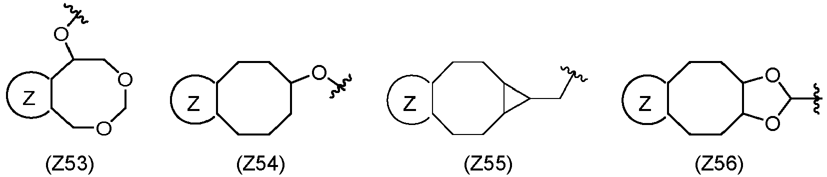

Herein, the R group(s) on Si in (Z50) and (Z51) are typically alkyl or aryl, preferably C₁-C₆ alkyl. Ring Z is formed during the cycloaddition reaction and is typically selected from structures (Zn) - (Zu), wherein the carbon atoms labelled with ** correspond to the two carbon atoms of the (hetero)cycloalkane ring of (Z44) - (Z56) to which ring Z is fused, and the carbon a carbon labelled with * is connected to CB. Since the connecting group Z is formed by reaction with a (hetero)cycloalkene in the context of the present embodiment, the bound depicted above asis a single bond.

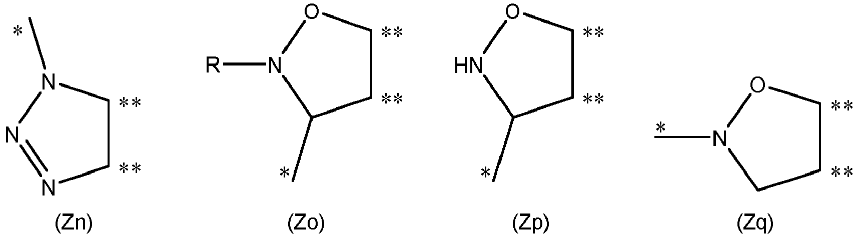

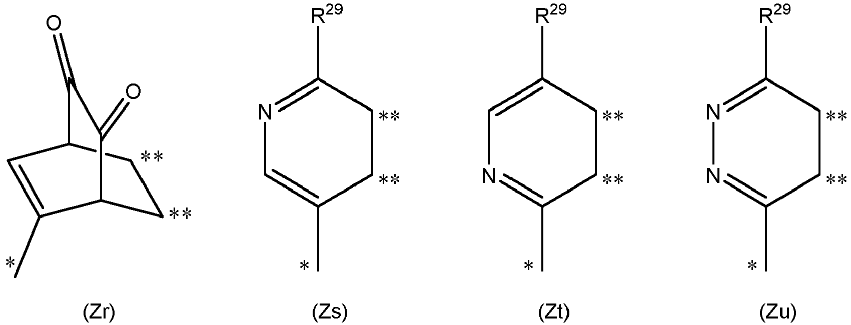

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction, especially in case ring (Zu) is formed. Thus, in a preferred embodiment, ring Z is (Zu) wherein R²⁹ is selected from hydrogen, methyl, phenyl, pyridyl, pyridinyl and pyrimidinyl, more preferably R²⁹ is hydrogen.

In case Z comprises a (hetero)cycloalkane moiety, it is preferred that ring Z is selected from (Zn), (Zs), (Zt) or (Zu), most preferably ring Z is according to structure (Zu).

In a preferred embodiment, connection group Z comprise a moiety selected from (Z1) - (Z56), wherein ring Z is selected from (Za) - (Zu).

In the present invention, the exact structure of Z¹ and Z² typically differ, as Z¹ is formed by reaction of Q¹ and F¹, whereas Z² is formed by reaction of Q² and F². Herein, F² is reactive towards Q² but not towards Q¹, such that Q¹ and Q² should differ.

In a preferred embodiment, F¹ is azide and Q¹ is a benzoannulated or tetramethylated (hetero)cycloalkyne, while F² is tetrazine and Q² is bicyclononyne. Herein, the reaction of F¹ and Q¹ will preferably form a connecting group Z¹ according to structure (Z5), (Z6), (Z7), (Z11), (Z17), (Z18), (Z19) or (Z19a), wherein ring Z is according to structure (Za), preferably according to structure (Z26), (Z27), (Z28), (Z32), (Z37), (Z38) or (Z38a), more preferably according to structure (Z40), (Z41) or (Z43). Herein, the reaction of F² and Q² will preferably form a connecting group Z² according to structure (Z8), wherein ring Z is according to structure (Zl), preferably according to structure (Z29), more preferably according to structure (Z42).

### Linker L^{A}

Linker L^{A} connects payloads D, via linker L^{B} and connecting groups Z², with Ab via connecting group Z¹ (in the conjugates according to the invention) or connects reactive group F² with reactive group Q¹ (in the linker according to the invention). Linkers are known in the art and may be cleavable or non-cleavable. Linker L^{A} preferably is not cleavable linker, while linker L^{B} preferably is cleavable.

Linker L^{A} is connected to one occurrence of Z¹ (or Q¹) and y occurrences of Z² (or F²). Thus, the valency of linker L^{A} is y + 1 or "(y + 1)-valent". Herein, "(y + 1)-valent" refers to the number of connecting points, being either a reactive group F or Q (before reaction) or a connecting group Z (after reaction). Thus, in case y = 2, the linker is connected to one Z¹/Q¹ and two Z²/F² and the linker has a valency of 2 + 1 = 3. Thus, when y = 2, the L^{A} is trivalent. Thus, in case y = 3, the linker is connected to one Z¹/Q¹ and three Z²/F² and the linker has a valency of 3 + 1 = 4. Thus, when y = 3, the L^{A} is tetravalent. Thus, in case y = 4, the linker is connected to one Z¹/Q¹ and four Z²/F² and the linker has a valency of 4 + 1 = 5. Thus, when y = 4, the L^{A} is pentavalent.

Linker L^{A} may be referred to as "heterobifunctional", which means that is contains two different functionalities, referring to the chemically different connectivities to Z¹/Q¹ and Z²/F².

In a preferred embodiment, L^{A} is a heterobifunctional linker of structure

-(L¹¹)-BM(L¹²-)_{y}

wherein:
- L¹¹ is connected to Q¹ or Z¹, and each L¹² is connected to F² or Z², wherein L¹¹ and L¹² each individually consist of one or more building blocks selected from C(R¹³)₂, C(R¹³)=C(R¹³), C≡C, C(O), NR¹³, O, S, S(O), S(O)₂, PR¹³ and P(O)R¹³, preferably the building blocks are selected from CH₂, CH=CH, C(O), NR¹³, O, S, S(O) and S(O)₂,
- each R¹³ is as defined below for structure (**23**), preferably R¹³ is H; and
- BM is a branching moiety. The branching moiety is further defined below.

In a more preferred embodiment, L^{A} is a heterobifunctional trivalent linker of structure

-(L¹¹)-BM(L¹²-)(L¹³-)

wherein:
- L¹¹ is connected to Q¹ or Z¹, and L¹² and L¹³ are connected to F² or Z², wherein L¹¹, L¹² and L¹³ each individually consist of one or more building blocks selected from C(R¹³)₂, C(R¹³)=C(R¹³), C≡C, C(O), NR¹³, O, S, S(O), S(O)₂, PR¹³ and P(O)R¹³, preferably the building blocks are selected from CH₂, CH=CH, C(O), NR¹³, O, S, S(O) and S(O)₂, and
- each R¹³ is as defined below for structure (**23**), preferably R¹³ is H; and
- BM is a branching moiety. The branching moiety is further defined below.

L¹¹, L¹² and L¹³ may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups. In one embodiment, the optional substituents may be selected from polar groups, such as oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In the context of the present invention, a linker construct of structure Q¹L^{A}(F²)_{y} reacts with structure Ab(F¹)₂, thereby performing two click reactions between F¹ and Q¹ per antibody Ab, and thus forming two connecting groups Z¹ per antibody Ab. Upon completion of the reaction, an antibody-linker construct is formed containing 2 × y click probes F² per antibody. The antibody-linker construct thus formed has a structure Ab(Z¹L^{A}(F²)_{y})₂.

### The branching moiety BM

A "branching moiety" in the context of the present invention refers to a moiety that is embedded in a linker connecting three moieties. In other words, the branching moiety comprises at least three bonds to other moieties.

Any moiety that contains at least three bonds to other moieties is suitable as branching moiety in the context of the present invention. Suitable branching moieties include a carbon atom (**BM-1**), a nitrogen atom (**BM-3**), a phosphorus atom (phosphine (**BM-5**) and phosphine oxide (**BM-6**)), aromatic rings such as a phenyl ring (e.g. **BM-7**) or a pyridyl ring (e.g. **BM-9**), a (hetero)cycle (e.g. **BM-11** and **BM-12**) and polycyclic moieties (e.g. **BM-13, BM-14** and **BM-15**). Preferably, BM is selected from a carbon atom, a nitrogen atom, a phosphorus atom, a (hetero)aromatic ring, a (hetero)cycle or a polycyclic moiety, more preferably BM is a carbon atom or a nitrogen atom. In case BM is a nitrogen atom, the branching nitrogen may be part of an amide group, for example with the connection to Z¹ or Q¹ via the C=O group and the connection to two occurrences of Z² or F² via the two substituents on nitrogen. In case BM is a carbon atom, the carbon atom is preferably part of a trivalent amino acid, such as lysine, aspartic acid or glutamic acid.

Suitable branching moieties BM are selected from structures **(BM-1)** to **(BM-15)** depicted here below, wherein the three branches, i.e. bonds to other moieties as defined above, are indicated by * (a bond labelled with *).

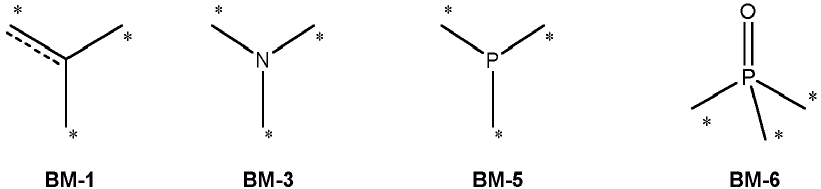

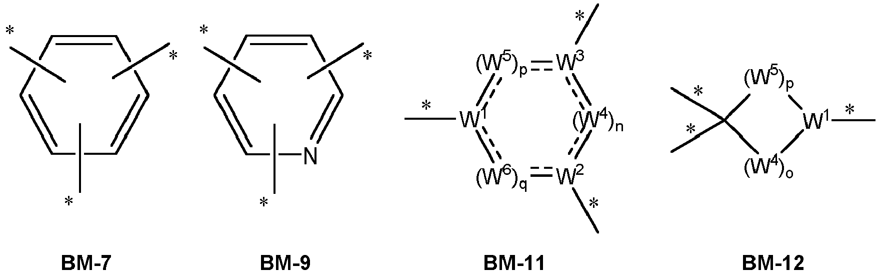

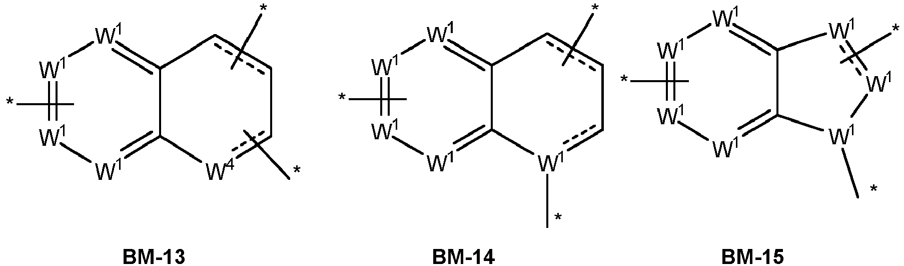

In **(BM-1),** one of the branches labelled with * may be a single or a double bond, indicated with - - - -. In (**BM-11**) to (**BM-15**), the following applies:
- each of n, p, q and q is individually an integer in the range of 0 - 5, preferably 0 or 1, most preferably 1;
- each of W¹, W² and W³ is independently selected from C(R²¹)_{w} and N;
- each of W⁴, W⁵ and W⁶ is independently selected from C(R²¹)_{w+1}, N(R²²)_{w}, O and S;
- each - - - - represents a single or double bond;
- w is 0 or 1 or 2, preferably 0 or 1;
- each R²¹ is independently selected from the group consisting of hydrogen, OH, C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, wherein the C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups are optionally substituted and optionally interrupted by one or more heteroatoms selected from O, S and NR³ wherein R³ is independently selected from the group consisting of hydrogen and C₁ - C₄ alkyl groups; and
- each R²² is independently selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, wherein the C₁ - C₂₄ alkyl groups, C₁ - C₂₄ alkoxy groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups are optionally substituted and optionally interrupted by one or more heteroatoms selected from O, S and NR³ wherein R³ is independently selected from the group consisting of hydrogen and C₁ - C₄ alkyl groups.

The skilled person appreciates that the values of w and the bond order of the bonds represented by - - - - are interdependent. Thus, whenever an occurrence of W is bonded to an endocyclic double bond, w = 1 for that occurrence of W, while whenever an occurrence of W is bonded to two endocyclic single bonds, w = 0 for that occurrence of W. For **BM-12,** at least one of o and p is not 0.

Representative examples of branching moieties according to structure (**BM-11**) and (**BM-12)** include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl, aziridine, azetidine, diazetidine, oxetane, thietane, pyrrolidine, dihydropyrrolyl, tetrahydrofuranyl, dihydrofuranyl, thiolanyl, imidazolinyl, pyrazolidinyl, oxazolidinyl, isoxazolidinyl, thiazolidinyl, isothiazolidinyl, dioxolanyl, dithiolanyl, piperidinyl, oxanyl, thianyl, piperazinyl, morpholino, thiomorpholino, dioxanyl, trioxanyl, dithyanyl, trithianyl, azepanyl, oxepanyl and thiepanyl. Preferred cyclic moieties for use as branching moiety include cyclopropenyl, cyclohexyl, oxanyl (tetrahydropyran) and dioxanyl. The substitution pattern of the three branches determines whether the branching moiety is of structure (**BM-11**) or of structure (**BM-12**).

Representative examples of branching moieties according to structure (**BM-13**) to **(BM-15)** include decalin, tetralin, dialin, naphthalene, indene, indane, isoindene, indole, isoindole, indoline, isoindoline, and the like.

In a preferred embodiment, BM is a carbon atom. In case the carbon atom is according to structure (**BM-1**) and has all four bonds to distinct moieties, the carbon atom is chiral. The stereochemistry of the carbon atom is not crucial for the present invention, and may be *S* or *R*. The same holds for the phosphine (**BM-6**). Most preferably, the carbon atom is according to structure (**BM-1**). One of the branches indicated with * in the carbon atom according to structure (**BM-1**) may be a double bond, in which case the carbon atom may be part of an alkene or imine. In case BM is a carbon atom, the carbon atom may be part of a larger functional group, such as an acetal, a ketal, a hemiketal, an orthoester, an orthocarbonate ester, an amino acid and the like. Preferred amino acids in this respect are Asp, Gly, Lys and iGlu. This also holds in case BM is a nitrogen or phosphorus atom, in which case it may be part of an amide, an imide, an imine, a phosphine oxide (as in **BM-6**) or a phosphotriester.

In a preferred embodiment, BM is a phenyl ring. Most preferably, the phenyl ring is according to structure (**BM-7**). The substitution pattern of the phenyl ring may be of any regiochemistry, such as 1,2,3-substituted phenyl rings, 1,2,4-substituted phenyl rings, or 1,3,5-substituted phenyl rings. To allow optimal flexibility and conformational freedom, it is preferred that the phenyl ring is according to structure (**BM-7**), most preferably the phenyl ring is 1,3,5-substituted. The same holds for the pyridine ring of (**BM-9**).

In the preferred structures for BM defined above, BM typically contains three connection points. The skilled person is capable to determine corresponding BMs with four or five connection points. For example **(BM-1)** and **(BM-7)** - **(BM-15)** would also be suitable as BM with more connection points. Alternatively, the linker may contain more than one BM to create four or five connection points.

In a preferred embodiment, the branching moiety BM is selected from a carbon atom, a nitrogen atom, a phosphorus atom, a (hetero)aromatic ring, a (hetero)cycle or a polycyclic moiety, more preferably a carbon atom or a nitrogen atom.

### Linker L^{B}

Linker L^{B} connects payload D with Ab, via connecting groups Z² and linker L^{A} (in the conjugates according to the invention) or connects payload D with reactive group Q² (in the payload-linker construct). Linkers are known in the art and may be cleavable or non-cleavable. Linker L^{B} preferably is cleavable linker, while linker L^{A} preferably is not cleavable.

Linker L^{B} is connected to one occurrence of Z² (or Q²) and x occurrences of D. Thus, the valency of linker L^{B} is x + 1 or "(x + 1)-valent". Herein, "(x + 1)-valent" refers to the number of connecting points, being either a payload or reactive group Q² (before reaction) or a connecting group Z² (after reaction). Thus, in case x = 1, the linker is connected to one Z²/Q² and one payload D, and the linker has a valency of 1 + 1 = 2. Thus, when x = 1, the L^{B} is divalent. Thus, in case x = 2, the linker is connected to one Z²/Q² and two payloads D, and the linker has a valency of 2 + 1 = 3. Thus, when x = 2, the L^{B} is trivalent. Thus, in case x = 3, the linker is connected to one Z²/Q² and three payloads D, and the linker has a valency of 3 + 1 = 4. Thus, when x = 3, the L^{B} is tetravalent. Thus, in case x = 4, the linker is connected to one Z²/Q² and four payloads D, and the linker has a valency of 4 + 1 = 5. Thus, when x = 4, the L^{B} is pentavalent.

Linker L^{B} may be referred to as "hetereobifunctional", which means that is contains two different functionalities, referring to the chemically different connectivities to Z²/Q² and D.

In a preferred embodiment, L^{B} is a heterobifunctional linker of structure

-(L¹)-(BM)_{r'}-[(L²)ₒ-(L³)ₚ-(L⁴)_{q}-]ₓ

wherein:
- L¹ is connected to Q² or Z², and all occurrences of L⁴ are connected to D;
- L¹, L², L³ and L⁴ are each individually linkers that together link Q² or Z² to D;
- o, p and q are each individually 0 or 1, preferably wherein o = p = 1;
- r' is 0 or 1, wherein r' is 0 when x is 1, or r'1 is 1 when x is 2, 3 or 4;
- BM is a branching moiety. The branching moiety is further defined above.

In an alternative preferred embodiment, x = 1 and L^{B} is a heterobifunctional divalent linker of structure

-(L¹)-(L²)ₒ-(L³)ₚ-(L⁴)_{q}-

wherein:
- L¹ is connected to Q² or Z², and L⁴ is connected to D;
- L¹, L², L³ and L⁴ are each individually linkers that together link Q² or Z² to D;
- o, p and q are each individually 0 or 1, preferably wherein o = p = 1;
- BM is a branching moiety. The branching moiety is further defined above.

In a more preferred embodiment, x = 2 and L^{B} is a heterobifunctional trivalent linker of structure

-(L¹)-(BM)-[(L²)ₒ-(L³)ₚ-(L⁴)_{q}-]₂

wherein:
- L¹ is connected to Q² or Z², and L⁴ is connected to D;
- L¹, L², L³ and L⁴ are each individually linkers that together link Q² or Z² to D;
- o, p and q are each individually 0 or 1, preferably wherein o = p = 1;
- BM is a branching moiety. The branching moiety is further defined above.

Alternatively, L^{B} is a heterobifunctional linker of structure

-(L¹)-[(L²)ₒ-(L³)ₚ-(L⁴)_{q}-]ₓ

wherein BM is embedded with linker L¹ in case x = 2, 3 or 4.

In the context of the present invention, a payload-linker construct of structure Q²(L^{B})Dₓ reacts with an antibody-linker construct of structure Ab(Z¹L^{A}(F²)_{y})₂, thereby performing 2 × y click reactions between F² and Q² per antibody Ab, and thus forming 2 × y connecting groups Z² per antibody Ab. Upon completion of the reaction, an antibody-conjugate is formed containing 2 × y × x payloads D per antibody. The antibody-conjugate thus formed has a structure Ab(Z¹L^{A}(Z²(L^{B})Dₓ)_{y})₂.

L¹, L², L³ and L⁴ are linkers or linking units and each of o, p and q are individually 0 or 1, preferably wherein o = p = 1. In a preferred embodiment, at least linkers L¹ and L² are present (i.e. o = 1; p = 0 or 1; q = 0 or 1), more preferably linkers L¹, L² and L³ are present (i.e. o = 1; p = 1; q = 0 or 1). In one embodiment, L¹, L², L³ and L⁴ each individually consist of one or more building blocks selected from (hetero)aryl, CH₂, CH=CH, C≡C, C(O), NR¹³, O, S, S(O), S(O)₂, PR¹³ and P(O)R¹³, preferably the building blocks are selected from aryl, CH₂, CH=CH, C(O), NR¹³, O, S, S(O) and S(O)₂. Preferred embodiments for each of L¹, L², L³ and L⁴ are provided below.

A linker, especially linker L¹, may contain one or more branch-points for attachment of multiple payloads to a single connecting group. In a preferred embodiment, the linker of the conjugate according to the invention contains a branching moiety. A "branching moiety" in the context of the present invention refers to a moiety that is embedded in a linker connecting three moieties. In other words, the branching moiety comprises at least three bonds to other moieties, typically one bond connecting to Z¹ or Q, one bond to the payload D and one bond to a second payload D. The branching moiety, if present, is preferably embedded in linker L¹, more preferably part of Sp³ or as the nitrogen atom of NR¹³. Any moiety that contains at least three bonds to other moieties is suitable as branching moiety in the context of the present invention. In a preferred embodiment, the branching moiety is selected from a carbon atom, a nitrogen atom, a phosphorus atom, a (hetero)aromatic ring, a (hetero)cycle or a polycyclic moiety. Most preferably, the branching moiety is a nitrogen atom.

### Linker L¹

L¹ may for example be selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups, C₉-C₂₀₀ arylalkynylene groups. Optionally the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups may be substituted, and optionally said groups may be interrupted by one or more heteroatoms, preferably 1 to 100 heteroatoms, said heteroatoms preferably being selected from the group consisting of O, S(O)_{y'} and NR²¹, wherein y' is 0, 1 or 2, preferably y' = 2, and R²¹ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups. In one embodiment, the optional substituents may be selected from polar groups, such as oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In a preferred embodiment, linker L¹ contains a polar group, which may also be present in the chain of L¹. Such a polar group may be selected from (poly)ethylene glycol diamines (e.g. 1,8-diamino-3,6-dioxaoctane or equivalents comprising longer ethylene glycol chains), (poly)ethylene glycol or (poly)ethylene oxide chains, (poly)propylene glycol or (poly)propylene oxide chains and 1,x'-diaminoalkanes (wherein x' is the number of carbon atoms in the alkane, preferably x' = 1 - 10), -(O)ₐ-C(O)-NH-S(O)₂-NR¹³- (as further defined below, see structure (**23**)), -C(S(O)₃⁽⁻⁾)-, -C(C(O)₂⁽⁻⁾)-, -S(O)₂-, -P(O)₂⁽⁻⁾-, -O(CH₂CH₂O)ₜ-, -NR³⁰(CH₂CH₂NR³⁰)ₜ-, and the following two structures:

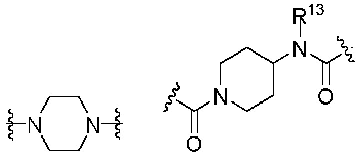

For the polar groups defined here above, it is irrelevant which end is connected to Z¹ and which end to (L²)ₒ.

The polar group may also contain an amino acid, preferably selected from Arg, Glu, Asp, Ser and Thr. Herein, R¹³ is further defined below for structure **(23)**. t is an integer in the range of integer in the range of 0 - 15, preferably 1 - 10, more preferably 2-5, most preferably t = 2 or 4. Each R³⁰ is individually H, C₁₋₁₂ alkyl, C₁₋₁₂ aryl, C₁₋₁₂ alkaryl or C₁₋₁₂ aralkyl. Linker L¹ may contain more than one such polar group, such as at least two polar groups. The polar group may also be present in a branch of linker L¹, which branches off a branching moiety as defined elsewhere. In the context of L¹, a nitrogen or carbon atom is preferably used as branching moiety. It is especially preferred to have a -O(CH₂CH₂O)ₜ- polar group present in a branch.

In a preferred embodiment, Linker L¹ is or comprises a sulfamide group, preferably a sulfamide group according to structure (**23**):

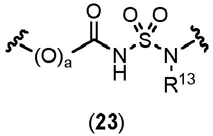

The wavy lines represent the connection to the remainder of the compound, typically to Q² or Z² and to L², L³, L⁴ or D. Preferably, the (O)ₐC(O) moiety is connected to Q² or Z² and the NR¹³ moiety to L², L³, L⁴ or D, preferably to L².

In structure (**23**), a = 0 or 1, preferably a = 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups. Alternatively, R¹³ is D connected to N optionally via a spacer moiety, preferably via Sp² as defined below, in one embodiment D is connected to N via -(B)ₑ-(A)_{f}-(B)_{g}-C(O)-. Alternatively, R¹³ is connected to elsewhere in the linker, optionally via a spacer moiety, to form a cyclic structure. For example, R¹³ may be connected to the linker via a CH₂CH₂ spacer moiety to form a piperazinyl ring, where the connection to D is via the second nitrogen of the piperazinyl ring.

In a preferred embodiment, R¹³ is hydrogen, a C₁ - C₂₀ alkyl group, preferably a C₁-- C₁₆ alkyl group, more preferably a C₁ - C₁₀ alkyl group, or connected to a further occurrence of D or to elsewhere in the linker optionally via a spacer moiety. Herein, the alkyl group is optionally substituted and optionally interrupted by one or more heteroatoms selected from O, S and NR¹⁴, preferably O, wherein R¹⁴ is independently selected from the group consisting of hydrogen and C₁ - C₄ alkyl groups. In another preferred embodiment, R¹³ is a C₁ - C₂₀ alkyl group, more preferably a C₁ -C₁₆ alkyl group, even more preferably a C₁ - C₁₀ alkyl group, wherein the alkyl group is optionally interrupted by one or more O-atoms, and wherein the alkyl group is optionally substituted with an -OH group, preferably a terminal -OH group. In this embodiment it is further preferred that R¹³ is a (poly)ethylene glycol chain comprising a terminal -OH group. In another preferred embodiment, R¹³ is selected from the group consisting of hydrogen, methyl, ethyl, n-propyl, i-propyl, n-butyl, s-butyl and t-butyl, or connected to a further occurrence of D or to elsewhere in the linker optionally via a spacer moiety, more preferably from the group consisting of hydrogen, methyl, ethyl, n-propyl and i-propyl, or connected to a further occurrence of D or to elsewhere in the linker optionally via a spacer moiety, and even more preferably from the group consisting of hydrogen, methyl and ethyl, or connected to a further occurrence of D or to elsewhere in the linker optionally via a spacer moiety. Yet even more preferably, R¹³ is hydrogen or connected to a further occurrence of D or to elsewhere in the linker optionally via a spacer moiety, and most preferably R¹³ is hydrogen.

In a preferred embodiment, L¹ is according to structure (**24**):

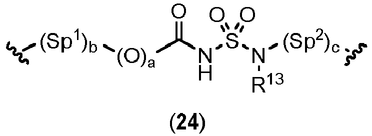

Herein, a and R¹³ are as defined above, Sp¹ and Sp² are independently spacer moieties and b and c are independently 0 or 1. Preferably, b = 0 or 1 and c = 1, more preferably b = 0 and c = 1. In one embodiment, spacers Sp¹ and Sp² are independently selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₇-C₂₀₀ alkylarylene groups, C₇-C₂₀₀ arylalkylene groups, C₈-C₂₀₀ arylalkenylene groups and C₉-C₂₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. When the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are interrupted by one or more heteroatoms as defined above, it is preferred that said groups are interrupted by one or more O-atoms, and/or by one or more S-S groups.

More preferably, spacer moieties Sp¹ and Sp², if present, are independently selected from the group consisting of linear or branched C₁-C₁₀₀ alkylene groups, C₂-C₁₀₀ alkenylene groups, C₂-C₁₀₀ alkynylene groups, C₃-C₁₀₀ cycloalkylene groups, C₅-C₁₀₀ cycloalkenylene groups, C₈-C₁₀₀ cycloalkynylene groups, C₇-C₁₀₀ alkylarylene groups, C₇-C₁₀₀ arylalkylene groups, C₈-C₁₀₀ arylalkenylene groups and C₉-C₁₀₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

Even more preferably, spacer moieties Sp¹ and Sp², if present, are independently selected from the group consisting of linear or branched C₁-C₅₀ alkylene groups, C₂-C₅₀ alkenylene groups, C₂-C₅₀ alkynylene groups, C₃-C₅₀ cycloalkylene groups, C₅-C₅₀ cycloalkenylene groups, C₈-C₅₀ cycloalkynylene groups, C₇-C₅₀ alkylarylene groups, C₇-C₅₀ arylalkylene groups, C₈-C₅₀ arylalkenylene groups and C₉-C₅₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

Yet even more preferably, spacer moieties Sp¹ and Sp², if present, are independently selected from the group consisting of linear or branched C₁-C₂₀ alkylene groups, C₂-C₂₀ alkenylene groups, C₂-C₂₀ alkynylene groups, C₃-C₂₀ cycloalkylene groups, C₅-C₂₀ cycloalkenylene groups, C₈-C₂₀ cycloalkynylene groups, C₇-C₂₀ alkylarylene groups, C₇-C₂₀ arylalkylene groups, C₈-C₂₀ arylalkenylene groups and C₉-C₂₀ arylalkynylene groups, the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted.

In these preferred embodiments it is further preferred that the alkylene groups, alkenylene groups, alkynylene groups, cycloalkylene groups, cycloalkenylene groups, cycloalkynylene groups, alkylarylene groups, arylalkylene groups, arylalkenylene groups and arylalkynylene groups are unsubstituted and optionally interrupted by one or more heteroatoms selected from the group of O, S and NR¹⁶, preferably O, wherein R¹⁶ is independently selected from the group consisting of hydrogen and C₁ - C₄ alkyl groups, preferably hydrogen or methyl.

Most preferably, spacer moieties Sp¹ and Sp², if present, are independently selected from the group consisting of linear or branched C₁-C₂₀ alkylene groups, the alkylene groups being optionally substituted and optionally interrupted by one or more heteroatoms selected from the group of O, S and NR¹⁶, wherein R¹⁶ is independently selected from the group consisting of hydrogen, C₁ - C₂₄ alkyl groups, C₂ - C₂₄ alkenyl groups, C₂ - C₂₄ alkynyl groups and C₃ - C₂₄ cycloalkyl groups, the alkyl groups, alkenyl groups, alkynyl groups and cycloalkyl groups being optionally substituted. In this embodiment, it is further preferred that the alkylene groups are unsubstituted and optionally interrupted by one or more heteroatoms selected from the group of O, S and NR¹⁶, preferably O and/or S-S, wherein R¹⁶ is independently selected from the group consisting of hydrogen and C₁ - C₄ alkyl groups, preferably hydrogen or methyl.

Preferred spacer moieties Sp¹ and Sp² thus include -(CH₂)ᵣ-, -(CH₂CH₂)ᵣ-, -(CH₂CH₂O)ᵣ-, -(OCH₂CH₂)ᵣ-, -(CH₂CH₂O)ᵣCH₂CH₂-, -CH₂CH₂(OCH₂CH₂)ᵣ-, -(CH₂CH₂CH₂O)ᵣ-, -(OCH₂CH₂CH₂)ᵣ-, -(CH₂CH₂CH₂O)ᵣCH₂CH₂CH₂- and -CH₂CH₂CH₂(OCH₂CH₂CH₂)ᵣ-, wherein r is an integer in the range of 1 to 50, preferably in the range of 1 to 40, more preferably in the range of 1 to 30, even more preferably in the range of 1 to 20 and yet even more preferably in the range of 1 to 15. More preferably n is 1, 2, 3, 4, 5, 6, 7, 8, 9 or 10, more preferably 1, 2, 3, 4, 5, 6, 7 or 8, even more preferably 1, 2, 3, 4, 5 or 6, yet even more preferably 1, 2, 3 or 4.

Alternatively, preferred linkers L¹ may be represented by -(W)ₖ-(A)_{d}-(B)ₑ-(A)_{f}-(C(O))_{g}-, wherein:
- d = 0 or 1, preferably d = 1;
- e = an integer in the range 0 - 10, preferably e = 0, 1, 2, 3, 4, 5 or 6, preferably an integer in the range 1 - 10, most preferably e = 1, 2, 3 or 4;
- f = 0 or 1, preferably f = 0;
- wherein d + e + f is at least 1, preferably in the range 1 - 5; and preferably wherein d + f is at least 1, preferably d + f = 1.
- g = 0 or 1, preferably g = 1;
- k = 0 or 1, preferably k = 1;
- A is a sulfamide group according to structure (**23**);
- B is a -CH₂-CH₂-O- or a -O-CH₂-CH₂- moiety, or (B)ₑ is a -(CH₂-CH₂-O)ₑ₁-CH₂-CH₂- or a -(CH₂-CH₂-O)ₑ₁-CH₂- moiety, wherein e1 is defined the same way as e;
- W is -OC(O)-, -C(O)O-, -C(O)NH-, -NHC(O)-, -OC(O)NH-, -NHC(O)O-, -C(O)(CH₂)ₘC(O)-, -C(O)(CH₂)ₘC(O)NH- or -(4-Ph)CH₂NHC(O)(CH₂)ₘC(O)NH-, preferably wherein W is -OC(O)NH-, -C(O)(CH₂)ₘC(O)NH- or -C(O)NH-, and wherein m is an integer in the range 0 - 10, preferably m = 0, 1, 2, 3, 4, 5 or 6, most preferably m = 2 or 3;
- preferably wherein L¹ is connected to Q via (W)ₖ and to L², L³ or D, preferably to L², via (C(O))_{g}, preferably via C(O).

In the context of the present embodiment, the wavy lines in structure (**23**) represent the connection to the adjacent groups such as (W)ₖ, (B)ₑ and (C(O))_{g}. It is preferred that A is according to structure (**23**), wherein a = 1 and R¹³ = H or a C₁ - C₂₀ alkyl group, more preferably R¹³ = H or methyl, most preferably R¹³ = H.

Preferred linkers L¹ have structure -(W)ₖ-(A)_{d}-(B)ₑ-(A)_{f}-(C(O))_{g}-, wherein:
(a) k = 0; d = 1; g = 1; f = 0; B = -CH₂-CH₂-O-; e = 1, 2, 3 or 4, preferably e = 2.
(b) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e = 1.
(c) k = 1; W = -OC(O)NH-; d = 0; B = -CH₂-CH₂-O-; g = 1; f = 0; e = 1, 2, 3 or 4, preferably e = 2.
(d) k = 1; W = -C(O)(CH₂)ₘC(O)NH-; m = 2; d = 0; (B)ₑ = -(CH₂-CH₂-O)ₑ₁-CH₂-CH₂-; f = 0; g = 1; e1 = 1, 2, 3 or 4, preferably e1 = 4.
(e) k = 1; W = -OC(O)NH-; d = 0; (B)ₑ = -(CH₂-CH₂-O)ₑ₁-CH₂-CH₂-; g = 1; f = 0; e1 = 1, 2, 3 or 4, preferably e1 = 4.
(f) k = 1; W = -(4-Ph)CH₂NHC(O)(CH₂)ₘC(O)NH-, m = 3; d = 0; (B)ₑ = -(CH₂-CH₂-O)ₑ₁-CH₂-CH₂-; g = 1; f = 0; e1 = 1, 2, 3 or 4, preferably e1 = 4.
(g) k = 0; d = 0; g = 1; f = 0; B = -CH₂-CH₂-O-; e = 1, 2, 3 or 4, preferably e = 2.
(h) k = 1; W = -C(O)NH-; d = 0; g = 1; f = 0; B = -CH₂-CH₂-O-; e = 1, 2, 3 or 4, preferably e = 2.

Herein, it is preferred that when d and/or f = 1, than a = 1 and R¹³ = H. Most preferred is the linker is structure (a).

In a preferred embodiment, linker L¹ comprises a branching nitrogen atom, which is located in the backbone between Q or Z and (L²)ₒ and which contains a further moiety D as substituent, which is preferably linked to the branching nitrogen atom via a linker. An example of a branching nitrogen atom is the nitrogen atom NR¹³ in structure (**23**), wherein R¹³ is connected to a second occurrence of D via a spacer moiety. Alternatively, a branching nitrogen atoms may be located within L¹ according to structure -(W)ₖ-(A)_{d}-(B)ₑ-(A)_{f}-(C(O))_{g}- In one embodiment, L¹ is represented by -(W)ₖ-(A)_{d}-(B)ₑ-(A)_{f}-(C(O))_{g}-BM[-(A)_{d}-(B)ₑ-(A)_{f}-(C(O))_{g}-]₂, wherein A, B, W, d, e, f, g and k are as defined above and individually selected for each occurrence, and BM is a branching moiety, preferably a branching nitrogen atom, to which two instances of -(A)_{d}-(B)ₑ-(A)_{f}-(C(O))_{g}- are connected. Herein, both (C(O))_{g} moieties are connected to -(L²)ₒ-(L³)ₚ-(L⁴)_{q}-D, wherein L², L³, L⁴, o, p, q and D are as defined above and are each selected individually. In a preferred embodiment, each of L², L³, L⁴, o, p, q and D are the same for both moieties connected to (C(O))_{g}.

Preferred linkers L¹ comprising a branching nitrogen atom have structure -(W)ₖ-(A)_{d}-(B)ₑ-(A)_{f}-(C(O))_{g'}-N[-(A')_{d'}-(B')_{e'}-(A')_{f'}-(C(O))_{g'}-]₂ wherein:
(i) k = d = g = e' = 1; f = d' = g' = 0; W = -C(O)-; B = B' = -CH₂-CH₂-O-; A is according to structure (**23**) with a = 0 and R¹³ = H; e = 1, 2, 3 or 4, preferably e = 2.
(j) k = d = g = e' = g' = 1; f = d' = 0; W = -C(O)-; B = B' = -CH₂-CH₂-O-; A is according to structure (**23**) with a = 0 and R¹³ = H; e = 1, 2, 3 or 4, preferably e = 2.

### Linker L²

Linker L² is a peptide spacer. Linker L² is either absent (o = 0) or present (o = 1). Preferably, linker L² is present and o = 1. The combination of a peptide spacer L² and a cleavable linker L³ is well-known in the art. Linker L² functions as recognition and cleave site for cleaving-enzymes, more preferably the peptides are recognized by specific cleaving enzymes. This allows for cleavage at specific environments in which these cleaving enzymes are expressed such as specific tumors. Since different peptide sequences are cleaved by different enzymes, the L² group also allows customizing the conjugate for specific treatments. The peptide sequences may be cleaved by intracellular enzymes and/or extracellular enzymes.

The peptide spacer may also be defined by (NH-CR¹⁷-CO)ₙ, wherein R¹⁷ represents an amino acid side chain as known in the art. Also covered within this definition is proline, which has R¹⁷ joined with the nitrogen atom to form a cyclic moiety. Herein, the amino acid may be a natural or a synthetic amino acid. Preferably, the amino acid(s) are all in their L-configuration. n is an integer in the range of 1 - 5, preferably in the range of 2 - 4. Thus, the peptide spacer contains 1 - 5 amino acids. Preferably, the peptide is a dipeptide (n = 2), tripeptide (n = 3) or tetrapeptide (n = 4), most preferably the peptide spacer is a dipeptide. R¹⁷ represents the amino acid side chain, preferably selected from the side chains of alanine, cysteine, aspartic acid, glutamic acid, phenylalanine, glycine, histidine, isoleucine, lysine, acetyllysine, leucine, methionine, asparagine, pyrrolysine, proline, glutamine, arginine, serine, threonine, selenocysteine, valine, tryptophan, tyrosine and citrulline. Preferred amino acid side chains are those of Val, Cit, Ala, Lys, Arg, AcLys, Phe, Leu, Ile, Trp, Glu, Asp and Asn, more preferably from the side chains of Val, Cit, Ala, Glu and Lys. Alternatively worded, R¹⁷ is preferably selected from CH₃ (Ala), CH₂CH(CH₃)₂ (Leu), CH₂CH₂CH₂NHC(O)NH₂ (Cit), CH₂CH₂CH₂CH₂NH₂ (Lys), CH₂CH₂CH₂NHC(O)CH₃ (AcLys), CH₂CH₂CH₂NHC(=NH)NH₂ (Arg), CH₂Ph (Phe), CH(CH₃)₂ (Val), CH(CH₃)CH₂CH₃ (Ile), CH₂C(O)NH₂ (Asn), CH₂CH₂C(O)OH (Glu), CH₂C(O)OH (Asp) and CH₂(1*H*-indol-3-yl) (Trp). Especially preferred embodiments of R¹⁷ are CH₃ (Ala), CH₂CH₂CH₂NHC(O)NH₂ (Cit), CH₂CH₂CH₂CH₂NH₂ (Lys), CH₂CH₂C(O)OH (Glu) and CH(CH₃)₂ (Val). Most preferably, R¹⁷ is CH₃ (Ala), CH₂CH₂CH₂NHC(O)NH₂ (Cit), CH₂CH₂CH₂CH₂NH₂ (Lys), or CH(CH₃)₂ (Val).

Although any peptide spacer may be used, preferably the peptide spacer is selected from Val-Cit, Val-Ala, Val-Lys, Val-Arg, AcLys-Val-Cit, AcLys-Val-Ala, Glu-Val-Ala, Asp-Val-Ala, iGlu-Val-Ala, Glu-Val-Cit, Glu-Gly-Cit, Glu-Gly-Val, Asp-Val-Cit, iGlu-Val-Cit, Phe-Cit, Phe-Ala, Phe-Lys, Phe-Arg, Ala-Lys, Leu-Cit, Ile-Cit, Trp-Cit, Asn-Asn, Ala-Ala-Asn, Ala-Asn, Asn-Ala, Phe-Phe, Gly, Gly-Gly, Gly-Gly-Gly, Gly-Gly-Gly-Gly, Leu-Gly, Tyr-Gly, Ala-Gly, Pro-Gly, Phe-Gly, Phe-Gly, Ser-Gly, Gly-Phe-Gly, Gly-Gly-Phe-Gly, Gly-Phe-Gly-Gly, Phe-Gly-Gly-Gly, Gly-Gly-Gly-Phe, Phe-Phe-Gly-Gly, Gly-Gly-Phe-Phe, Gly-Gly-Gly-Phe-Gly and Lys, more preferably Val-Cit, Val-Ala, Glu-Val-Ala, Val-Lys, Phe-Cit, Phe-Ala, Phe-Lys, Ala-Ala-Asn, more preferably Glu-Gly-Cit, Val-Cit, Val-Ala, Asn-Asn, Ala-Ala-Asn, Asn-Ala most preferably Glu-Gly-Cit, Val-Cit, Val-Ala or Asn-Ala. Herein, AcLys is e-N-acetyllysine and iGlu is isoglutamate. In one embodiment, L² = Val-Cit. In another embodiment, L² = Val-Ala. In another embodiment, L² = Asn-Ala. In another embodiment, L² = Glu-Gly-Cit.

In one embodiment, the amino acid side chain R¹⁷ is substituted with a polar group, preferably selected from oxo groups, (poly)ethylene glycol diamines, (poly)ethylene glycol or (poly)ethylene oxide chains, (poly)propylene glycol or (poly)propylene oxide chains, carboxylic acid groups, carbonate groups, carbamate groups, cyclodextrins, crown ethers, saccharides (e.g. monosaccharides, oligosaccharides), phosphates or esters thereof, phosphonic acid or ester, phosphinic acid or ester, sulfoxides, sulfones, sulfonic acid or ester, sulfinic acid, or sulfenic acid.

In an especially preferred embodiment, L² comprises the peptide spacer represented by general structure (**25**), preferably L² is represented by general structure (**25**):

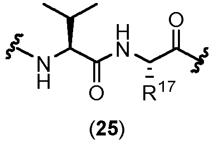

Herein, R¹⁷ is as defined above, preferably R¹⁷ is CH₃ (Ala) or CH₂CH₂CH₂NHC(O)NH₂ (Cit). The wavy lines indicate the connection to (L¹)ₙ and (L³)ₚ, preferably L² according to structure (**25**) is connected to (L¹)ₙ via NH and to (L³)ₚ via C(O).

### Linker L³

Linker L³ is a self-cleavable spacer, also referred to as self-immolative spacer. Linker L³ is either absent (p = 0) or present (p = 1). Preferably, linker L³ is present and p = 1. Cleavage of L² results in 1,6-β elimination in linker L³, resulting in decarboxylation and the release of the payload, D. This advantageously allows for increased probability of release of the payload in regions wherein enzymes that are able to cleave L² are overexpressed. In addition, release of a payload can induce bystander killing which is advantageous for tumours in which not all cancer cells have overexpression of the targeted receptor. Hence, in this embodiment, it is preferred that both linker L² and L³ are present (o = p = 1).

Preferably, L³ is para-aminobenzyloxycarbonyl (PABC) derivative, more preferably a PABC derivative according to structure (**26**):

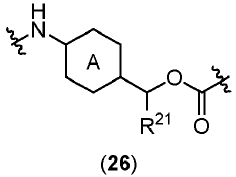

Herein, the wavy lines indicate the connection to L¹ or L², and to L⁴ or D. Typically, the PABC derivative is connected via NH to L¹ or L², preferably to L², and via OC(O) to L⁴ or D.

Ring A is a 5- or 6-membered aromatic or heteroaromatic ring, preferably a 6-membered aromatic or heteroaromatic ring. Suitable 5-membered rings are oxazole, thiazole and furan. Suitable 6-membered rings are phenyl and pyridyl. Ring A may be substituted with a substituent selected from halogen, X²R⁴, N(R⁴)₂, C₁₋₄ alkyl and NO₂. Herein, X² and R⁴ are as defined above, including preferred embodiments thereof. In a preferred embodiment, the optional substituent is selected from F, Cl, Br, OH, OR⁴, SH, NH₂, Et, Me and NO₂. In an especially preferred embodiment, ring A comprises 0 - 2 substituents, more preferably 0 or 1 substituent, most preferably ring A is not substituted. In a preferred embodiment, ring A is 1,4-phenyl, 1,2-phenyl, 2,5-pyridyl or 3,6-pyridyl. Most preferably, A is 1,4-phenyl.

R²¹ is selected from H, R²⁶, C(O)OH and C(O)R²⁶, wherein R²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups. Preferably, R²⁶ is C₃ - C₁₀ (hetero)cycloalkyl or polyalkylene glycol. The polyalkylene glycol is preferably a polyethylene glycol or a polypropylene glycol, more preferably -(CH₂CH₂O)ₛH or -(CH₂CH₂CH₂O)ₛH. The polyalkylene glycol is most preferably a polyethylene glycol, preferably -(CH₂CH₂O)ₛH, wherein s is an integer in the range 1 - 10, preferably 1 - 5, most preferably s = 1, 2, 3 or 4. More preferably, R²¹ is H or C(O)R²⁶, wherein R²⁶ = 4-methyl-piperazine or morpholine. Most preferably, R²¹ is H.

In an alternative embodiment, linker L³ is present (p = 1) and linker L² is not (o = 0) and L³ is a para-glucuronide-meta-amide-benzyloxycarbonyl derivative, preferably a glucuronide derivative according to structure (**27**):

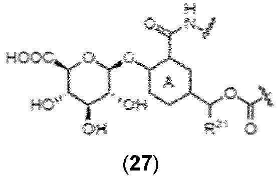

Herein, the wavy lines indicate the connection to L¹, and to L⁴ or D. Typically, the glucuronide derivative is connected via NH to L¹, and via (O)CO to L⁴ or D. Ring A and R²¹ are defined as for the PABC derivative according to structure (**26**). Preferably, ring A a 6-membered aromatic or heteroaromatic ring, such as oxazole, thiazole, furan, phenyl and pyridyl. In a preferred embodiment, ring A is 1 ,3,4-phenyl, 2,4,5-pyridyl or 2,5,6-pyridyl. Most preferably, A is 1,3,4-phenyl. Preferably, R²¹ is H or C(O)R²⁶, wherein R²⁶ = 4-methyl-piperazine or morpholine. Most preferably, R²¹ is H.

Linker L³ according to structure (**27**) is cleavable by β-glucuronidase, similar to the mechanism in PABC, which results in self-immolation of the para-hydroxybenzyloxy group, decarboxylation and the release of the payload. An ADC comprising the glucuronide derivative according to structure (**27**) is especially useful for treating cancers having an overexpression of β-glucuronidase. β Glucuronidase concentrations in many solid tumours, including lung, breast, and gastrointestinal cancers, as well as in the tumour microenvironment, are reported to be higher than those in normal tissues, and the enzyme is not found in the general circulation. Thus, it is preferred that the conjugates according to the invention comprising the glucuronide derivative according to structure (**27**) are used to treat patients suffering from lung, breast, and gastrointestinal cancers.

### Linker L⁴

Linker L⁴ is either absent (q = 0) or present (q = 1). Preferably, linker L⁴ is present and q = 1. Linker L⁴ is selected from:
- an aminoalkanoic acid spacer according to the structure - NR²²-(Cₓ-alkylene)-C(O)-, wherein x is an integer in the range 1 - 20 and R²² is H or C₁ - C₄ alkyl;
- an ethyleneglycol spacer according to the structure -NR²²-(CH₂-CH₂-O)ₑ₆-(CH₂)ₑ₇-C(O)-, wherein e6 is an integer in the range 1 - 10, e7 is an integer in the range 1 - 3 and R²² is H or C₁ - C₄ alky; and
- an diamine spacer according to the structure - NR²²-(Cₓ-alkylene)-NR²²-(C(O))ₕ-, wherein h is 0 or 1, x is an integer in the range 1 - 20 and R²² is H or C₁ - C₄ alkyl.

Linker L⁴ may be an aminoalkanoic acid spacer, i.e. -NR²²-(Cₓ-alkylene)-C(O)-, wherein x is an integer in the range 1 to 20, preferably 1 - 10, most preferably 1 - 6. Herein, the aminoalkanoic acid spacer is typically connected to L³ via the nitrogen atom and to D via the carbonyl moiety. Preferred linkers L⁴ are selected from 6-aminohexanoic acid (Ahx, x = 5), β-alanine (x = 2) and glycine (Gly, x = 1), even more preferably 6-aminohexanoic acid or glycine. In one embodiment, L⁴= 6-aminohexanoic acid. In one embodiment, L⁴= glycine. Herein, R²² is H or C₁ - C₄ alkyl, preferably R²² is H or methyl, most preferably R²² is H.

Alternatively, linker L⁴ may be an ethyleneglycol spacer according to the structure -NR²²-(CH₂-CH₂-O)ₑ₆-(CH₂)ₑ₇-(C(O)-, wherein e6 is an integer in the range 1 - 10, preferably e6 is in the range 2-6, and e7 is an integer in the range 1 - 3, preferably e7 is 2. Herein, R²² is H or C₁ - C₄ alkyl, preferably R²² is H or methyl, most preferably R²² is H.

Alternatively, linker L⁴ may be a diamine spacer according to the structure - NR²²-(Cₓ-alkylene)-NR²²-(C(O))ₕ-, wherein h is 0 or 1, x is an integer in the range 1 - 20, preferably an integer in the range 2-6, even more preferably x = 2 or 5, most preferably x = 2. R²² is H or C₁ - C₄ alkyl. Herein, R²² is H or C₁ - C₄ alkyl, preferably R²² is H or methyl, most preferably R²² is methyl. Herein, h is preferably 1, in which case linker L⁴ is especially suited for conjugation via a phenolic hydroxyl group present on payload D.

### Payload D

D, also referred to in the art as the "payload", represents the compound that is or is to be connected to antibody Ab. Payload molecules are well-known in the art, especially in the field of antibody-drug conjugates, as the moiety that is covalently attached to the antibody and that is released therefrom upon uptake of the conjugate and/or cleavage of the linker. In a preferred embodiment, the payload is selected from the group consisting of an active substance, a reporter molecule, a polymer, a solid surface, a hydrogel, a nanoparticle, a microparticle and a biomolecule. Especially preferred payloads are active substances and reporter molecules, in particular active substances.

The term "active substance" herein relates to a pharmacological and/or biological substance, i.e. a substance that is biologically and/or pharmaceutically active, for example a drug, a prodrug, a cytotoxin, a diagnostic agent, a protein, a peptide, a polypeptide, a peptide tag, an amino acid, a glycan, a lipid, a vitamin, a steroid, a nucleotide, a nucleoside, a polynucleotide, RNA or DNA. Examples of peptide tags include cell-penetrating peptides like human lactoferrin or polyarginine. An example of a glycan is oligomannose. An example of an amino acid is lysine.

When the payload is an active substance, the active substance is preferably selected from the group consisting of drugs and prodrugs. More preferably, the active substance is selected from the group consisting of pharmaceutically active compounds, in particular low to medium molecular weight compounds (e.g. about 200 to about 2500 Da, preferably about 300 to about 1750 Da). In a further preferred embodiment, the active substance is selected from the group consisting of cytotoxins, antiviral agents, antibacterial agents, peptides and oligonucleotides.

Preferred cytotoxins are selected from the groups of nitrogen mustards, nitrosoureas, alkylsulphonates, triazenes, platinum containing compounds, plant alkaloids, DNA topoisomerase inhibitors, anti-metabolites, hormonal therapies, kinase inhibitors, antibiotics, and further cytotoxins defined here below.

Suitable nitrogen mustards include chlorambucil, chlornaphazine, cyclophosphamide, dacarbazine, estramustine, ifosfamide, mechlorethamine, mechlorethamine oxide hydrochloride, mannomustine, mitobronitol, melphalan, mitolactol, pipobroman, novembichin, phenesterine, prednimustine, thiotepa, trofosfamide, uracil mustard; CC-1065 (including its adozelesin, carzelesin and bizelesin synthetic analogues); duocarmycin (including the synthetic analogues, KW-2189 and CBI-TMI); benzodiazepine monomers or dimers (e.g., pyrrolobenzodiazepine (PBD), tomaymycin, indolinobenzodiazepine, isoindolinebenzodiazepine, imidazobenzothiadiazepine and oxazolidinobenzodiazepine).

Suitable nitrosoureas include carmustine, lomustine, chlorozotocin, fotemustine, nimustine and ranimustine.

Suitable alkylsulphonates include busulfan, treosulfan, improsulfan and piposulfan.

Suitable triazenes include dacarbazine.

Suitable platinum containing compounds include carboplatin, cisplatin, oxaliplatin; aziridines, such as benzodopa, carboquone, meturedopa, and uredopa; ethylenimines and methylamelamines including altretamine, triethylenemelamine, trietylenephosphoramide, triethylenethiophosphaoramide and trimethylolomelamine.

Suitable plant alkaloids include vinca alkaloids (e.g. vincristine, vinblastine, vindesine, vinorelbine, navelbin), toxoids (e.g. paclitaxel, docetaxel and their analogs), maytansinoids and their analogs (e.g. DM1, DM2, DM3, DM4, maytansine and ansamitocins), cryptophycins (particularly cryptophycin 1 and cryptophycin 8), epothilones, eleutherobin, discodermolide, bryostatins, dolostatins, auristatins, tubulysins, cephalostatins, pancratistatin, sarcodictyin and spongistatin.

Suitable DNA topoisomerase inhibitors include any camptothecin, including 9-aminocamptothecin, exatecan, DXd (DX-8951 derivative), crisnatol, daunomycin, etoposide, etoposide phosphate, irinotecan, mitoxantrone, novantrone, retinoic acids (retinols), teniposide, topotecan, 9-nitrocamptothecin (RFS 2000); mitomycins and mitomycin C.

Suitable anti-metabolites include anti-folate, DHFR inhibitors (e.g. methotrexate, trimetrexate, denopterin, pteropterin, aminopterin (4-aminopteroic acid) or other folic acid analogues), IMP dehydrogenase inhibitors (e.g. mycophenolic acid, tiazofurin, ribavirin, EICAR), ribonucleotide reductase inhibitors (e.g. hydroxyurea, deferoxamine), pyrimidine analogs (e.g. uracil analogs such as ancitabine, azacitidine, 6-azauridine, capecitabine (Xeloda), carmofur, cytarabine, dideoxyuridine, doxifluridine, enocitabine, 5-fluorouracil, floxuridine, ratitrexed (Tomudex); cytosine analogs such as cytarabine, cytosine arabinoside, fludarabine; purine analogs such as azathioprine, fludarabine, mercaptopurine, thiamiprine, thioguanine), folic acid replenisher (e.g. frolinic acid).

Suitable hormonal therapies include receptor antagonists (e.g. anti-estrogen such as megestrol, raloxifene, tamoxifen, LHRH agonists such as goscrclin, leuprolide acetate; antiandrogens such as bicalutamide, flutamide, calusterone, dromostanolone propionate, epitiostanol, goserelin, leuprolide, mepitiostane, nilutamide, testolactone, trilostane and other androgens inhibitors), retinoids/deltoids (e.g. Vitamin D3 analogs such as CB 1093, EB 1089 KH 1060, cholecalciferol, ergocalciferol), photodynamic therapies (e.g. verteporfin, phthalocyanine, photosensitizer Pc4, demethoxy-hypocrellin A).

Suitable kinase inhibitors include BIBW 2992 (anti-EGFR/Erb2), imatinib, gefitinib, pegaptanib, sorafenib, dasatinib, sunitinib, erlotinib, nilotinib, lapatinib, axitinib, pazopanib, vandetanib, E7080 (anti-VEGFR2), mubritinib, ponatinib (AP24534), bafetinib (INNO-406), bosutinib (SKI-606), cabozantinib, vismodegib, iniparib, ruxolitinib, CYT387, axitinib, tivozanib, sorafenib and ispinesib.

Suitable antibiotics include the enediyne antibiotics (e.g. calicheamicins, especially calicheamicin .γ1, δ1, α1 and β1), dynemicin (e.g. dynemicin A and deoxydynemicin), esperamicin, kedarcidin, C-1027, maduropeptin, as well as neocarzinostatin chromophore and related chromoprotein enediyne antiobiotic chromomophores, aclacinomysins, actinomycin, authramycin, azaserine, bleomycins, cactinomycin, carabicin, carminomycin, carzinophilin; chromomycins, dactinomycin, daunorubicin, nemorubicin, detorubicin, 6-diazo-5-oxo-L-norleucine, doxorubicin, morpholino-doxorubicin, cyanomorpholino-doxorubicin, 2-pyrrolino-doxorubicin and deoxydoxorubicin, epirubicin, esorubicin, idarubicin, marcellomycin, nitomycins, mycophenolic acid, nogalamycin, olivomycins, peplomycin, potfiromycin, puromycin, quelamycin, rodorubicin, streptonigrin, streptozocin, tubercidin, ubenimex, zinostatin and zorubicin.

Suitable polyketides include acetogenins (in particualr bullatacin and bullatacinone), gemcitabine, epoxomicins (e. g. carfilzomib), bortezomib, thalidomide, lenalidomide, pomalidomide, tosedostat, zybrestat, PLX4032, STA-9090, Stimuvax, allovectin-7, Xegeva and Provenge.

The cytotoxin may further be selected from isoprenylation inhibitors (such as lovastatin), dopaminergic neurotoxins (such as 1-methyl-4-phenylpyridinium ion), cell cycle inhibitors (such as staurosporine), actinomycins (such as actinomycin D and dactinomycin), bleomycins (such as bleomycin A2, bleomycin B2, peplomycin), anthracyclines (e.g. daunorubicin, doxorubicin (adriamycin), idarubicin, epirubicin, pirarubicin, zorubicin, mitoxantrone), MDR inhibitors (e.g. verapamil), Ca2+ ATPase inhibitors (e.g. thapsigargin), histone deacetylase inhibitors (e.g. Vorinostat, Romidepsin, Panobinostat, Valproic acid, Mocetinostat (MGCD0103), Belinostat, PCI-24781, Entinostat, SB939, Resminostat, Givinostat, AR-42, CUDC-101, sulforaphane, Trichostatin A), thapsigargin, Celecoxib, glitazones, epigallocatechin gallate, disulfiram, salinosporamide A.; anti-adrenals (e.g. aminoglutethimide, mitotane, trilostane; aceglatone; aldophosphamide glycoside; aminolevulinic acid; amsacrine; arabinoside, bestrabucil; bisantrene; edatraxate; defofamine; demecolcine; diaziquone; eflornithine (DFMO), elfomithine; elliptinium acetate, etoglucid; gallium nitrate; gacytosine, hydroxyurea; ibandronate, lentinan; lonidamine; mitoguazone; mitoxantrone; mopidamol; nitracrine; pentostatin; phenamet; pirarubicin; podophyllinic acid; 2-ethylhydrazide; procarbazine; PSK^{®}; razoxane; rhizoxin; sizofiran; spirogermanium; tenuazonic acid; triaziquone; 2, 2',2"-trichlorotriethylamine; trichothecenes (especially T-2 toxin, verrucarin A, roridin A and anguidine).

The term "reporter molecule" herein refers to a molecule whose presence is readily detected, for example a diagnostic agent, a dye, a fluorophore, a radioactive isotope label, a contrast agent, a magnetic resonance imaging agent or a mass label.

A wide variety of fluorophores, also referred to as fluorescent probes, is known to a person skilled in the art. Several fluorophores are described in more detail in e.g. G.T. Hermanson, "Bioconjugate Techniques", Elsevier, 3rd Ed. 2013, Chapter 10: "Fluorescent probes", p. 395 - 463, incorporated by reference. Examples of a fluorophore include all kinds of Alexa Fluor (e.g. Alexa Fluor 555), cyanine dyes (e.g. Cy3 or Cy5) and cyanine dye derivatives, coumarin derivatives, fluorescein and fluorescein derivatives, rhodamine and rhodamine derivatives, boron dipyrromethene derivatives, pyrene derivatives, naphthalimide derivatives, phycobiliprotein derivatives (e.g. allophycocyanin), chromomycin, lanthanide chelates and quantum dot nanocrystals.

Examples of a radioactive isotope label include ^{99m}Tc, ¹¹¹In, ^{114m}In, ¹¹⁵In, ¹⁸F, ¹⁴C, ⁶⁴Cu, ¹³¹I, ¹²⁵I, ¹²³I, ²¹²Bi, ⁸⁸Y, ⁹⁰Y, ⁶⁷Cu, ¹⁸⁶Rh, ¹⁸⁸Rh, ⁶⁶Ga, ⁶⁷Ga and ¹⁰B, which is optionally connected via a chelating moiety such as e.g. DTPA (diethylenetriaminepentaacetic anhydride), DOTA (1,4,7,10-tetraazacyclododecane-*N,N',N",N‴*-tetraacetic acid), NOTA (1,4,7-triazacyclononane *N,N',N"-*triacetic acid), TETA (1,4,8,11-tetraazacyclotetradecane-*N,N',N",N*‴-tetraacetic acid), DTTA (*N¹-*(*p-*isothiocyanatobenzyl)-diethylenetriamine-*N¹,N²,N³,N³*-tetraacetic acid), deferoxamine or DFA (*N'-*[5-[[4-[[5-(acetylhydroxyamino)pentyl]amino]-1,4-dioxobutyl]hydroxyamino]pentyl]-*N*-(5-aminopentyl)-*N*-hydroxybutanediamide) or HYNIC (hydrazinonicotinamide). Isotopic labelling techniques are known to a person skilled in the art, and are described in more detail in e.g. G.T. Hermanson, "Bioconjugate Techniques", Elsevier, 3rd Ed. 2013, Chapter 12: "Isotopic labelling techniques", p. 507 - 534, incorporated by reference.

Polymers suitable for use as a payload D in the compound according to the invention are known to a person skilled in the art, and several examples are described in more detail in e.g. G.T. Hermanson, "Bioconjugate Techniques", Elsevier, 3rd Ed. 2013, Chapter 18: "PEGylation and synthetic polymer modification", p. 787 - 838, incorporated by reference. When payload D is a polymer, payload D is preferably independently selected from the group consisting of a poly(ethyleneglycol) (PEG), a polyethylene oxide (PEG), a polypropylene glycol (PPG), a polypropylene oxide (PPO), a 1 ,q-diaminoalkane polymer (wherein q is the number of carbon atoms in the alkane, and preferably q is an integer in the range of 2 to 200, preferably 2 to 10), a (poly)ethylene glycol diamine (e.g. 1,8-diamino-3,6-dioxaoctane and equivalents comprising longer ethylene glycol chains), a polysaccharide (e.g. dextran), a poly(amino acid) (e.g. a poly(L-lysine)) and a poly(vinyl alcohol).

Solid surfaces suitable for use as a payload D are known to a person skilled in the art. A solid surface is for example a functional surface (e.g. a surface of a nanomaterial, a carbon nanotube, a fullerene or a virus capsid), a metal surface (e.g. a titanium, gold, silver, copper, nickel, tin, rhodium or zinc surface), a metal alloy surface (wherein the alloy is from e.g. aluminum, bismuth, chromium, cobalt, copper, gallium, gold, indium, iron, lead, magnesium, mercury, nickel, potassium, plutonium, rhodium, scandium, silver, sodium, titanium, tin, uranium, zinc and/or zirconium), a polymer surface (wherein the polymer is e.g. polystyrene, polyvinylchloride, polyethylene, polypropylene, poly(dimethylsiloxane) or polymethylmethacrylate, polyacrylamide), a glass surface, a silicone surface, a chromatography support surface (wherein the chromatography support is e.g. a silica support, an agarose support, a cellulose support or an alumina support), etc. When payload D is a solid surface, it is preferred that D is independently selected from the group consisting of a functional surface or a polymer surface.

Hydrogels are known to the person skilled in the art. Hydrogels are water-swollen networks, formed by cross-links between the polymeric constituents. See for example A. S. Hoffman, Adv. Drug Delivery Rev. 2012, 64, 18, incorporated by reference. When the payload is a hydrogel, it is preferred that the hydrogel is composed of poly(ethylene)glycol (PEG) as the polymeric basis.

Micro- and nanoparticles suitable for use as a payload D are known to a person skilled in the art. A variety of suitable micro- and nanoparticles is described in e.g. G.T. Hermanson, "Bioconjugate Techniques", Elsevier, 3rd Ed. 2013, Chapter 14: "Microparticles and nanoparticles", p. 549 - 587, incorporated by reference. The micro- or nanoparticles may be of any shape, e.g. spheres, rods, tubes, cubes, triangles and cones. Preferably, the micro- or nanoparticles are of a spherical shape. The chemical composition of the micro- and nanoparticles may vary. When payload D is a micro- or a nanoparticle, the micro- or nanoparticle is for example a polymeric micro- or nanoparticle, a silica micro- or nanoparticle or a gold micro- or nanoparticle. When the particle is a polymeric micro- or nanoparticle, the polymer is preferably polystyrene or a copolymer of styrene (e.g. a copolymer of styrene and divinylbenzene, butadiene, acrylate and/or vinyltoluene), polymethylmethacrylate (PMMA), polyvinyltoluene, poly(hydroxyethyl methacrylate (pHEMA) or polyethylene glycol dimethacrylate/2-hydroxyethylmetacrylae) [poly(EDGMA/HEMA)]. Optionally, the surface of the micro- or nanoparticles is modified, e.g. with detergents, by graft polymerization of secondary polymers or by covalent attachment of another polymer or of spacer moieties, etc.

Payload D may also be a biomolecule. Biomolecules, and preferred embodiments thereof, are described in more detail below. When payload D is a biomolecule, it is preferred that the biomolecule is selected from the group consisting of proteins (including glycoproteins such as antibodies), polypeptides, peptides, glycans, lipids, nucleic acids, oligonucleotides, polysaccharides, oligosaccharides, enzymes, hormones, amino acids and monosaccharides.

In the context of the present invention, cytotoxic payloads are especially preferred. Thus, D is preferably, a cytotoxin, more preferably selected from the group consisting of colchicine, vinca alkaloids, anthracyclines, camptothecins, doxorubicin, daunorubicin, taxanes, calicheamicins, tubulysins, irinotecans, an inhibitory peptide, amanitins, amatoxins, duocarmycins, epothilones, mytomycins, combretastatins, maytansines, auristatins, enediynes, pyrrolobenzodiazepines (PBDs) or indolinobenzodiazepine dimers (IGN). The present invention is especially suitable for cytotoxic payloads with a moderate cytotoxicity. Normally, conjugates of such payloads need to be administered in a higher dose, but in view of the high DAR (high payload loading) of the conjugates of the present invention, even conjugates of moderately toxic payloads can be administered in a desirably low dose. In other words, D is preferably a payload that enables a moderate to high administered dose in patients (3-20 mg/kg) even at high DAR (DAR >> 6). In view of the high payload loading in the conjugates of the present invention, it is especially preferred that the payload (in unconjugated form) has an average cytotoxicity (IC₅₀) of >50 nM. As such, even payloads with a lower cytotoxicity can be employed in antibody-conjugates with high efficacy.

### Preferred linkers L^{A}

Especially preferred linkers L^{A} have the structure -(L¹¹)-BM(L¹²-)(L¹³-), wherein L¹¹ is according to any one of (i) - (x):
(i) L¹¹ is *-C(O)-(CH₂)_{a'}-C(O)-**, wherein the bond labelled * is connected to Q¹ or Z¹ and the bond labelled ** is connected to BM, wherein a' is an integer in the range 1 - 5, preferably a'= 3. Further, it is preferred that BM is a nitrogen atom (for y = 2) or carbon atom (for y = 3). Most preferably, BM = N.
(ii) L¹¹ is *-NH-C(O)-(CH₂)_{a'}-**, wherein the bond labelled * is connected to Q¹ or Z¹ and the bond labelled ** is connected to BM, wherein a' is an integer in the range 0 - 5, preferably a'= 0. Further, it is preferred that BM is a nitrogen atom (for y = 2) or carbon atom (for y = 3). Most preferably, BM = N.
(iii) L¹¹ is *=N-C(O)-(CH₂)_{a'}-**, wherein the double bond labelled * is connected to Q¹ or Z¹ and the bond labelled ** is connected to BM, wherein a' is an integer in the range 0 - 5, preferably a'= 0. Further, it is preferred that BM is a nitrogen atom (for y = 2) or carbon atom (for y = 3). Most preferably, BM = N.
(iv) L¹¹ is *-NH-C(O)-(CH₂)_{a'}-C(O)-**, wherein the bond labelled * is connected to Q¹ or Z¹ and the bond labelled ** is connected to BM, wherein a' is an integer in the range 1 - 5, preferably a'= 3. Further, it is preferred that BM is a nitrogen atom (for y = 2) or carbon atom (for y = 3). Most preferably, BM = N.
(v) L¹¹ is *=N-C(O)-(CH₂)_{a'}-C(O)-**, wherein the double bond labelled * is connected to Q¹ or Z¹ and the bond labelled ** is connected to BM, wherein a' is an integer in the range 1 - 5, preferably a'= 3. Further, it is preferred that BM is a nitrogen atom (for y = 2) or carbon atom (for y = 3). Most preferably, BM = N.
(vi) L¹¹ is *-C(O)-**, wherein the bond labelled * is connected to Q¹ or Z¹ and the bond labelled ** is connected to BM, wherein it is preferred that BM is a nitrogen atom (for y = 2) or carbon atom (for y = 3). Most preferably, BM = N.
(vii) L¹¹ is *-(**23**)-(CH₂CH₂O)_{a'}-(C(O))_{a"}-**, wherein the bond labelled * is connected to Q¹ or Z¹ and the bond labelled ** is connected to BM, and wherein (**23**) refers to a group according to structure (**23**). Further, a' is an integer in the range 0 - 5, preferably a'= 1 - 4, most preferably a' = 2, and a" is 0 or 1, preferably a" is 1. Further, it is preferred that BM is a nitrogen atom (for y = 2) or carbon atom (for y = 3). Most preferably, BM = N.
(viii) L¹¹ is *-(**23**)-(CH₂CH₂O)_{a'}-(**23**)-**, wherein the bond labelled * is connected to Q¹ or Z¹ and the bond labelled ** is connected to BM, and wherein (**23**) refers to a group according to structure (**23**). Further, a' is an integer in the range 0 - 5, preferably a'= 1 - 4, most preferably a' = 2. Further, it is preferred that BM is a nitrogen atom (for y = 2) or carbon atom (for y = 3). Most preferably, BM = N.
(ix) L¹¹ is *-C(O)-NH-(CH₂CH₂O)_{a'}-C(O)-**, wherein the bond labelled * is connected to Q¹ or Z¹ and the bond labelled ** is connected to BM, wherein a' is an integer in the range 1 - 5, preferably a'= 3. Further, it is preferred that BM is a nitrogen atom (for y = 2) or carbon atom (for y = 3). Most preferably, BM = N.
(x) L¹¹ is *-C(O)-NH-(CH₂CH₂O)_{a'}-O-C(O)-NH-**, wherein the bond labelled * is connected to Q¹ or Z¹ and the bond labelled ** is connected to BM, wherein a' is an integer in the range 1 - 5, preferably a'= 3. Further, it is preferred that BM is a nitrogen atom (for y = 2) or carbon atom (for y = 3). Most preferably, BM = N.

Especially preferred linkers L^{A} have the structure -(L¹¹)-BM(L¹²-)(L¹³-), wherein L¹² and L¹³ are according to any one of (xi) - (xv):
(xi) L¹² and L¹³ both are *-(CH₂)_{a'}-O-C(O)-NH)-(CH₂CH₂O)_{a"}-CH₂-CH₂-N-C(O)-**, wherein the bond labelled * is connected to BM and the bond labelled ** is connected to F², wherein a' is an integer in the range 0 - 4, preferably a'= 2, and a" is an integer in the range 0-4, preferably a" is 2. Further, it is preferred that BM is a nitrogen atom.
(xii) L¹² and L¹³ both are *-(CH₂CH₂O)_{a'}-CH₂-CH₂-C(O)-NH-(CH₂)_{a"}-Ph-**, wherein the bond labelled * is connected to BM and the bond labelled ** is connected to F², wherein a' is an integer in the range 0 - 5, preferably a'= 2, and a" is an integer in the range 0 - 4, preferably a" is 1. Preferably, Ph is 1,4-Ph. Further, it is preferred that BM is a nitrogen atom.
(xiii) L¹² and L¹³ both are *-(CH₂CH₂O)_{a'}-CH₂-CH₂-C(O)-NH-(CH₂)_{a"}-NH-C(O)-Ph-**, wherein the bond labelled * is connected to BM and the bond labelled ** is connected to F², wherein a' is an integer in the range 0 - 5, preferably a'= 2, and a" is an integer in the range 0 - 4, preferably a" is 2 or 3. Preferably, Ph is 1,4-Ph. Further, it is preferred that BM is a nitrogen atom.
(xiv) L¹² and L¹³ both are *-(CH₂CH₂O)_{a'}-CH₂-CH₂-C(O)-NH-(CH₂)_{a"}-O-Ph-**, wherein the bond labelled * is connected to BM and the bond labelled ** is connected to F², wherein a' is an integer in the range 0 - 5, preferably a'= 2, and a" is an integer in the range 0 - 4, preferably a" is 2 or 3. Preferably, Ph is 1,4-Ph. Further, it is preferred that BM is a nitrogen atom.
(xv) L¹² and L¹³ both have the structure -(L¹⁴)-BM'(L¹⁵-)(L¹⁶-), wherein:
   - L¹⁴ is as defined for L¹¹, preferably according to any one of (i) to (x) as defined above or, L¹⁴ is *-(CH₂CH₂O)_{a'}-CH₂-CH₂-(C(O))_{a"}-**, wherein the bond labelled * is connected to BM and the bond labelled ** is connected to BM', and wherein a' is an integer in the range 0 - 5, preferably a'= 1 - 4, most preferably a' = 2, and a" is 0 or 1, preferably a" is 1.
   - BM and BM' are branching moieties, preferably both are nitrogen atoms. With one occurrence of BM and two occurrences of BM' (in each of L¹⁵ and L¹⁶), y = 4.
   - L¹⁵ and L¹⁶ are as defined for L¹² and L¹³. Preferably, according to (xiv) as defined above.

Preferably, linkers L¹² and L¹³ both have the same structure. In an especially preferred embodiment of L^{A}, linker L¹¹ is according to any one of (i) - (x) and linkers L¹² and L¹³ are according to any one of (xi) - (xv).

Such preferred linkers L^{A} can be employed in the linker-construct according to the invention, in the conjugate according to the invention and in the intermediate antibody-linker construct according to the invention. In the context of this preferred embodiment, Q¹ is preferably according to (Q26) or (Q37) and F² is preferably according to (F8a), wherein R²⁹ is hydrogen or methyl. Alternatively, it is preferred that Z¹ is preferably according to (Z26) or (Z37), wherein ring Z is according to (Za), and Z² is preferably according to (Z29), wherein ring Z is according to (Zj), wherein R²⁹ is hydrogen or methyl.

### Process for synthesising the conjugate according to general structure (2)

In a further aspect, the present invention relates to a process for the preparation of the conjugate according to the invention, the process comprising:
(a) providing a modified antibody having the structure Ab(F¹)_{z}, wherein Ab is an antibody, z is 2 or 4, and F¹ is a click probe;
(b) reacting the modified antibody with z equivalents of Q¹L^{A}(F²)_{y}, wherein Q¹ is a click probe that is reactive towards F¹, L^{A} is a heterobifunctional (y + 1)-valent linker, y is 2, 3 or 4, and F² is a click probe that is not reactive towards Q¹, to obtain an antibody-linker construct of structure Ab(Z¹L^{A}(F²)_{y})₂, wherein Z¹ is a connecting group obtained by reaction of F¹ and Q¹;
(c) reacting the antibody-linker construct with z × y equivalents of Q²(L^{B})Dₓ, wherein Q² is a click probe that is reactive towards F², L^{B} is a (x + 1)-valent linker, x is 1, 2, 3 or 4, provided that x + y is at least 4, and D is a payload molecule, to obtain the conjugate of structure Ab(Z¹L^{A}(Z²(L^{B})Dₓ)_{y})_{z}, wherein Z² is a connecting group obtained by reaction of F² and Q².

The reactions performed in steps (b) and (c) are click reactions, wherein click probes F react with click probes Q to form connecting groups Z. This conjugation technique is known to the skilled person. The present reactions occur under conditions such that Q is reacted with F to form covalent connections. In the process according to the invention, Q reacts with F, forming a covalent connection between the antibody and the payloads. Complementary reactive groups Q and reactive groups F are known to the skilled person and are described in more detail below.

Thus, the reactions in the process according to the invention, conjugation is accomplished via a cycloaddition. Preferred cycloadditions are a (4+2)-cycloaddition (e.g. a Diels-Alder reaction) or a [3+2]-cycloaddition (e.g. a 1,3-dipolar cycloaddition). The reaction of step (b) is preferably a [3+2]-cycloaddition, more preferably a 1,3-dipolar cycloaddition. The 1,3-dipolar cycloaddition is preferably the alkyne-azide cycloaddition, and most preferably wherein Q is or comprises an alkyne group and F is an azido group. The reaction of step (c) is a [4+2]-cycloaddition, preferably a Diels-Alder reaction. The preferred Diels-Alder reaction is the inverse-electron demand Diels-Alder cycloaddition. Cycloadditions, such as Diels-Alder reactions and 1,3-dipolar cycloadditions are known in the art, and the skilled person knowns how to perform them.

### Step (a)

In step (a), an antibody having the structure Ab(F¹)_{z} is provided. Typically, two or four click probes F¹ are chemically or enzymatically introduced onto the antibody. Preferably, z = 2 and two click probes are introduced onto the antibody.

In a preferred embodiment, an antibody comprising two or four, preferably two, core N-acetylglucosamine moieties is contacted with a compound of the formula S(F¹)-P in the presence of a catalyst, wherein S(F¹) is a sugar derivative comprising two reactive groups F¹ capable of reacting with a reactive group Q¹, and P is a nucleoside mono- or diphosphate, and wherein the catalyst is capable of transferring the S(F¹) moiety to the core-GIcNAc moiety. Herein, the antibody is typically an antibody that has been trimmed to a core-GIcNAc residue as described further below.

The starting material, i.e. the antibody comprising two or four, preferably two, core-GIcNAc substituents, is known in the art and can be prepared by methods known by the skilled person. In one embodiment, the process according to the invention further comprises the deglycosylation of an antibody glycan having a core N-acetylglucosamine, in the presence of an endoglycosidase, in order to obtain an antibody comprising a core N-acetylglucosamine substituent, wherein said core N-acetylglucosamine and said core N-acetylglucosamine substituent are optionally fucosylated. Depending on the nature of the glycan, a suitable endoglycosidase may be selected. The endoglycosidase is preferably selected from the group consisting of EndoS, EndoA, EndoE, EfEndo18A, EndoF, EndoM, EndoD, EndoH, EndoT and EndoSH and/or a combination thereof, the selection of which depends on the nature of the glycan. EndoSH is described in PCT/EP2017/052792, see Examples 1 - 3, and SEQ. ID No: 1, which is incorporated by reference herein.

Structural feature S is defined above for the conjugate according to the invention, which equally applies to the present aspect. Compounds of the formula S(F¹)-P, wherein a nucleoside monophosphate or a nucleoside diphosphate P is linked to a sugar derivative S(F¹), are known in the art. For example Wang et al., Chem. Eur. J. 2010, 16, 13343-13345, Piller et al., ACS Chem. Biol. 2012, 7, 753, Piller et al., Bioorg. Med. Chem. Lett. 2005, 15, 5459-5462 and WO 2009/102820, all incorporated by reference herein, disclose a number of compounds S(F¹)-P and their syntheses. In a preferred embodiment nucleoside mono- or diphosphate P in S(F¹)-P is selected from the group consisting of uridine diphosphate (UDP), guanosine diphosphate (GDP), thymidine diphosphate (TDP), cytidine diphosphate (CDP) and cytidine monophosphate (CMP), more preferably P is selected from the group consisting of uridine diphosphate (UDP), guanosine diphosphate (GDP) and cytidine diphosphate (CDP), most preferably P = UDP. Preferably, S(F¹)-P is selected from the group consisting of GalNAz-UDP, F₂-GalNAz-UDP (*N*-(azidodifluoro)acetylgalactosamine), 6-AzGal-UDP, 6-AzGaINAc-UDP (6-azido-6-deoxy-N-acetylgalactosamine-UDP), 4-AzGalNAz-UDP, 6-AzGalNAz-UDP, GIcNAz-UDP, 6-AzGlc-UDP, 6-AzGlcNAz-UDP and 2-(but-3-yonic acid amido)-2-deoxy-galactose-UDP. Most preferably, S(F¹)-P is GalNAz-UDP or 6-AzGaINAc-UDP.

Suitable catalyst that are capable of transferring the S(F¹) moiety to the core-GIcNAc moiety are known in the art. A suitable catalyst is a catalyst wherefore the specific sugar derivative nucleotide S(F¹)-P in that specific process is a substrate. More specifically, the catalyst catalyses the formation of a β(1,4)-glycosidic bond. Preferably, the catalyst is selected from the group of galactosyltransferases and N-acetylgalactosaminyltransferases, more preferably from the group of P(1,4)-N-acetylgalactosaminyltransferases (GaINAcT) and β(1,4)-galactosyltransferases (GalT), most preferably from the group of β(1,4)-N-acetylgalactosaminyltransferases having a mutant catalytic domain. Suitable catalysts and mutants thereof are disclosed in WO 2014/065661, WO 2016/022027 and WO 2016/170186, all incorporated herein by reference. In one embodiment, the catalyst is a wild-type galactosyltransferase or *N*-acetylgalactosaminyltransferase, preferably an N-acetylgalactosaminyltransferase. In an alternative embodiment, the catalyst is a mutant galactosyltransferase or *N*-acetylgalactosaminyltransferases, preferably a mutant N-acetylgalactosaminyltransferase. Mutant enzymes described in WO 2016/022027 and WO 2016/170186 are especially preferred. These galactosyltransferase (mutant) enzyme catalysts are able to recognize internal sugars and sugar derivatives as an acceptor. Thus, sugar derivative S(F¹) is linked to the core-GIcNAc substituent in step (a), irrespective of whether said GIcNAc is fucosylated or not.

Step (a) is preferably performed in a suitable buffer solution, such as for example phosphate, buffered saline (e.g. phosphate-buffered saline, tris-buffered saline), citrate, HEPES, tris and glycine. Suitable buffers are known in the art. Preferably, the buffer solution is phosphate-buffered saline (PBS) or tris buffer. Step (i) is preferably performed at a temperature in the range of about 4 to about 50 °C, more preferably in the range of about 10 to about 45 °C, even more preferably in the range of about 20 to about 40 °C, and most preferably in the range of about 30 to about 37 °C. Step (i) is preferably performed a pH in the range of about 5 to about 9, preferably in the range of about 5.5 to about 8.5, more preferably in the range of about 6 to about 8. Most preferably, step (i) is performed at a pH in the range of about 7 to about 8.

### Step (b)

In step (b), the modified antibody Ab(F¹)_{z} is reacted with a linker construct according to the invention, comprising a reactive group Q¹ capable of reacting with reactive group F¹, to obtain an antibody-linker construct, containing connecting group Z¹ resulting from the reaction between Q¹ and F¹. Such reaction occurs under condition such that reactive group Q¹ is reacted with the reactive group F¹ of the antibody to covalently link the antibody to the linker-construct. In step (b), the reaction occurs with z equivalents of Q¹L^{A}(F²)_{y}, although more equivalents of Q¹L^{A}(F²)_{y} may be present in the reaction mixture in order to ensure complete reaction. The skilled person is able to determine the optimal reaction conditions and stoichiometry of the reactants in order to obtain an optimal yield.

In a preferred embodiment, in step (b) an azide on an azide-modified antibody reacts with a benzoannulated or tetramethylated (hetero)cycloalkyne group, preferably wherein Q¹ is according to structure (Q5), (Q6), (Q7), (Q11), (Q17), (Q18), (Q19) or (Q19a), preferably according to structure (Q26), (Q27), (Q28), (Q32), (Q37), (Q38) or (Q38a), more preferably according to structure (Q40), (Q41) or (Q43), via a cycloaddition reaction. Using such benzoannulated or tetramethylated (hetero)cycloalkyne groups, the presence of a catalyst is not required, and the cycloaddition reaction may even occur spontaneously by a reaction called strain-promoted azide-alkyne cycloaddition (SPAAC). This is one of the reactions known in the art as "metal-free click chemistry".

### Step (c)

In step (c), the modified antibody-linker construct Ab(Z¹L^{A}(F²)_{y})_{z} is reacted with a payload-linker, comprising a reactive group Q² capable of reacting with reactive group F², to obtain an antibody-conjugate, containing connecting group Z² resulting from the reaction between Q² and F². Such reaction occurs under condition such that reactive group Q² is reacted with the reactive group F² to covalently link the antibody to the payloads. Step (c) may also be referred to as the conjugation reaction. In step (c), the reaction occurs with z × y equivalents of Q²(L^{B})Dₓ, although more equivalents of Q²(L^{B})Dₓ may be present in the reaction mixture in order to ensure complete reaction. The skilled person is able to determine the optimal reaction conditions and stoichiometry of the reactants in order to obtain an optimal yield.

In a preferred embodiment, in step (c) a 1 ,2,4,5-tetrazine on the antibody-linker construct reacts with a bicyclononyne group, preferably wherein Q¹ is according to structure (Q8), more preferably according to structure (Q29), most preferably according to structure (Q42), via a cycloaddition reaction. Using such bicyclononyne groups, the presence of a catalyst is not required, and the cycloaddition reaction may even occur spontaneously by a reaction called strain-promoted azide-alkyne cycloaddition (SPAAC). This is one of the reactions known in the art as "metal-free click chemistry".

### Reactive moiety Q

Reactive moieties Q¹ and Q² are click probes. In the context of the present invention, Q refers to Q¹ and Q². In the context of the present invention, the term "reactive moiety" may refer to a chemical moiety that comprises a reactive group, but also to a reactive group itself. For example, a cyclooctynyl group is a reactive group comprising a reactive group, namely a C-C triple bond. However, a reactive group, for example an azido reactive group, may herein also be referred to as a reactive moiety.

Q is reactive towards and complementary to F. Herein, a reactive group is denoted as "complementary" to a reactive group when said reactive group reacts with said reactive group selectively, optionally in the presence of other functional groups. Complementary reactive click probes are known to a person skilled in the art, and are described in more detail below. The exact nature of Q, and F, depends on the type of click reaction that is employed. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, click probe Q comprises or is an alkene moiety or an alkyne moiety, more preferably wherein the alkene is a (hetero)cycloalkene and/or the alkyne is a terminal alkyne or a (hetero)cycloalkyne.

In an especially preferred embodiment, Q comprises a cyclic (hetero)alkyne moiety. The alkynyl group may also be referred to as a (hetero)cycloalkynyl group, i.e. a heterocycloalkynyl group or a cycloalkynyl group, wherein the (hetero)cycloalkynyl group is optionally substituted. Preferably, the (hetero)cycloalkynyl group is a (hetero)cycloheptynyl group, a (hetero)cyclooctynyl group, a (hetero)cyclononynyl group or a (hetero)cyclodecynyl group. Herein, the (hetero)cycloalkynes may optionally be substituted. Preferably, the (hetero)cycloalkynyl group is an optionally substituted (hetero)cycloheptynyl group or an optionally substituted (hetero)cyclooctynyl group. Most preferably, the (hetero)cycloalkynyl group is a (hetero)cyclooctynyl group, wherein the (hetero)cyclooctynyl group is optionally substituted.

In an especially preferred embodiment, Q comprises a (hetero)cycloalkynyl or (hetero)cycloalkenyl group and is according to structure (Q1): 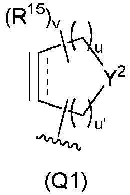 Herein:
- the bond depicted as - - - is a double bond or a triple bond;
- R¹⁵ is independently selected from the group consisting of hydrogen, halogen, -OR¹⁶, -NOz, -CN, -S(O)₂R¹⁶, -S(O)₃(-), C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y² is C(R³¹)₂, O, S, S⁽⁺⁾R³¹, S(O)R³¹, S(O)=NR³¹ or NR³¹, wherein S⁽⁺⁾ is a cationic sulphur atom counterbalanced by B⁽⁻⁾, wherein B⁽⁻⁾ is an anion, and wherein each R³¹ individually is R¹⁵ or a connection with D, connected via L;
- u is 0, 1, 2, 3, 4 or 5;
- u' is 0, 1, 2, 3, 4 or 5, wherein u + u' = 0, 1, 2, 3, 4, 5, 6, 7 or 8;
- v = an integer in the range 0-16;

Typically, v = (u + u') × 2 (when the connection to L, depicted by the wavy bond, is via Y²) or [(u + u') × 2] - 1 (when the connection to L, depicted by the wavy bond, is via one of the carbon atoms of u and u').

In a preferred embodiment of structure (Q1), reactive group Q comprises a (hetero)cycloalkynyl group and is according to structure (Q1a): 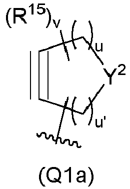 Herein,
- R¹⁵ and Y² are as defined above
- u is 0, 1, 2, 3, 4 or 5;
- u' is 0, 1, 2, 3, 4 or 5, wherein u + u' = 4, 5, 6, 7 or 8;
- v = an integer in the range 8-16.

In a preferred embodiment, u + u' = 4, 5 or 6, more preferably u + u' = 5.

In a preferred embodiment, v = 8, 9 or 10, more preferably v = 9 or 10, most preferably v = 10.

In a preferred embodiment, Q is a (hetero)cycloalkynyl group selected from the group consisting of (Q2) - (Q20) depicted here below.

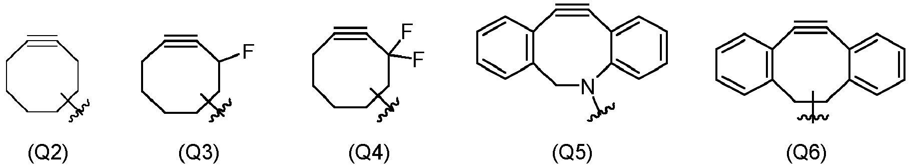

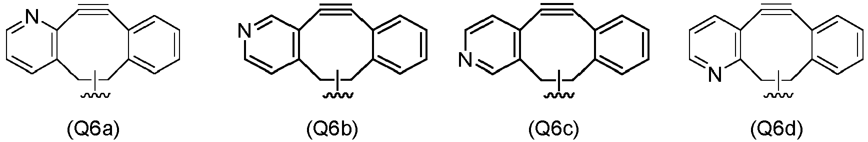

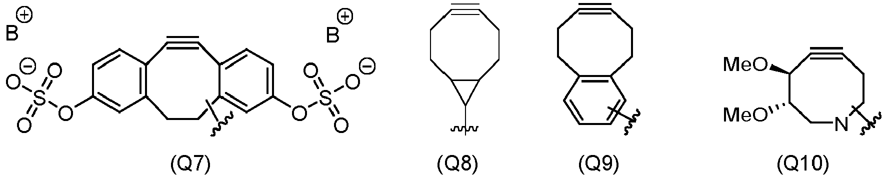

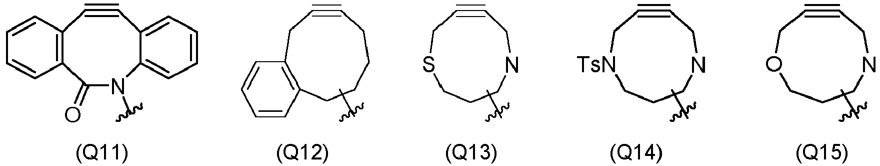

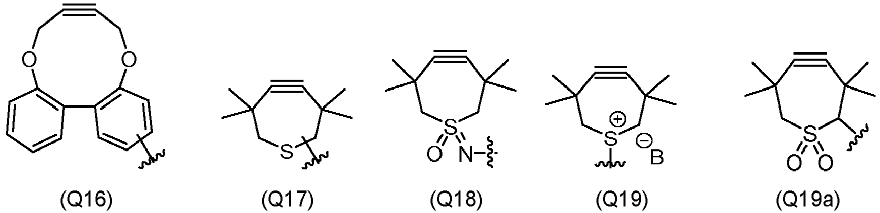

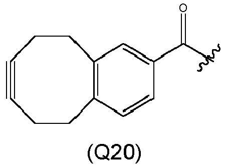

Herein, the connection to L, depicted with the wavy bond, may be to any available carbon or nitrogen atom of Q. The nitrogen atom of (Q10), (Q13), (Q14) and (Q15) may bear the connection to L, or may contain a hydrogen atom or be optionally functionalized. B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf. B⁽⁺⁾ is a cation, preferably a pharmaceutically acceptable cation. In the conjugation reaction, B⁽⁻⁾ does not need to be a pharmaceutically acceptable anion, since B⁽⁻⁾ will exchange with the anions present in the reaction mixture anyway. In case (Q19) is used for Q, the negatively charged counter-ion is preferably pharmaceutically acceptable upon isolation of the conjugate according to the invention, such that the conjugate is readily useable as medicament.

In a further preferred embodiment, Q is a (hetero)cycloalkynyl group selected from the group consisting of (Q21) - (Q38a) depicted here below.

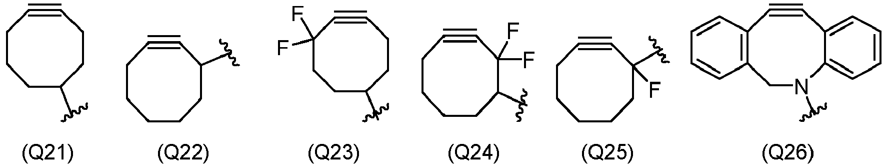

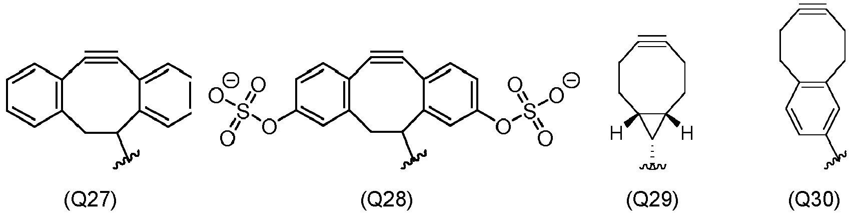

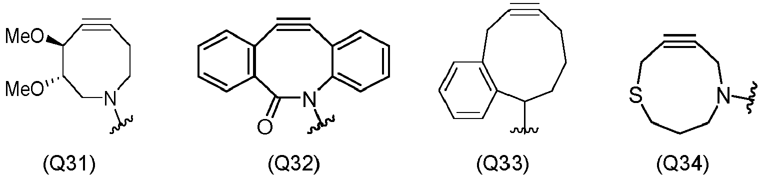

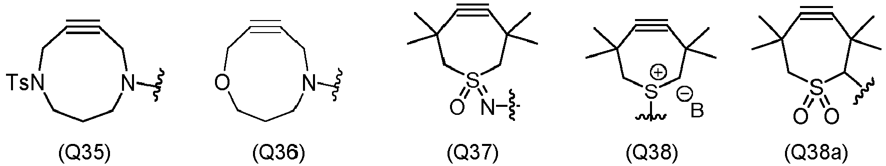

In structure (Q38), B⁽⁻⁾ is an anion, which is preferably selected from ⁽⁻⁾OTf, Cl⁽⁻⁾, Br⁽⁻⁾ or I⁽⁻⁾, most preferably B⁽⁻⁾ is ⁽⁻⁾OTf.

In a preferred embodiment, Q comprises a (hetero)cyclooctyne moiety or a (hetero)cycloheptyne moiety, preferably according to structure (Q8), (Q26), (Q27), (Q28), (Q37) or (Q38a), which are optionally substituted. Each of these preferred options for Q are further defined here below.

Thus, in a preferred embodiment, Q comprises a heterocycloheptyne moiety according to structure (Q37), also referred to as a TMTHSI, which is optionally substituted. Preferably, the heterocycloheptyne moiety according to structure (Q37) is not substituted.

In an alternative preferred embodiment, Q comprises a cyclooctyne moiety according to structure (Q8), more preferably according to (Q29), also referred to as a bicyclo[6.1.0]non-4-yn-9-yl] group (BCN group), which is optionally substituted. Preferably, the cyclooctyne moiety according to structure (Q8) or (Q29) is not substituted. In the context of the present embodiment, Q preferably is a (hetero)cyclooctyne moiety according to structure (Q39) as shown below, wherein V is (CH₂)_{I} and I is an integer in the range of 0 to 10, preferably in the range of 0 to 6. More preferably, I is 0, 1, 2, 3 or 4, more preferably I is 0, 1 or 2 and most preferably I is 0 or 1. In the context of group (Q39), I is most preferably 1. Most preferably, Q is according to structure (Q42), defined further below.

In an alternative preferred embodiment, Q comprises a (hetero)cyclooctyne moiety according to structure (Q26), (Q27) or (Q28), also referred to as a DIBO, DIBAC, DBCO or ADIBO group, which are optionally substituted. In the context of the present embodiment, Q preferably is a (hetero)cyclooctyne moiety according to structure (Q40) or (Q41) as shown below, wherein Y¹ is O or NR¹¹, wherein R¹¹ is independently selected from the group consisting of hydrogen, a linear or branched C₁ - C₁₂ alkyl group or a C₄ - C₁₂ (hetero)aryl group. The aromatic rings in (Q40) are optionally O-sulfonylated at one or more positions, whereas the rings of (Q41) may be halogenated at one or more positions. Preferably, the (hetero)cyclooctyne moiety according to structure (Q40) or (Q41) is not further substituted. Most preferably, Q is according to structure (Q43), defined further below.

In an alternative preferred embodiment, Q comprises a heterocycloheptynyl group and is according to structure (Q37).

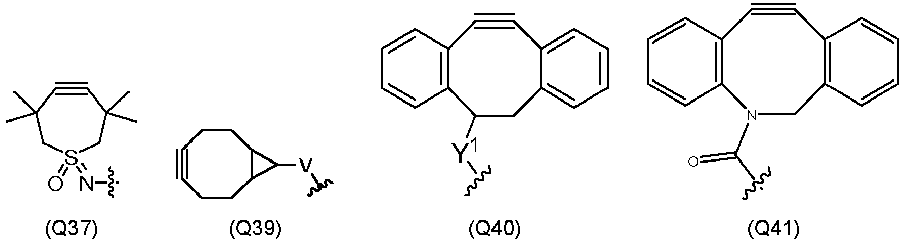

In an especially preferred embodiment, Q comprises a cyclooctynyl group and is according to structure (Q42): 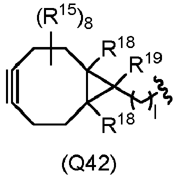 Herein:
- R¹⁵ is independently selected from the group consisting of hydrogen, halogen, -OR¹⁶, -NOz, -CN, -S(O)₂R¹⁶, -S(O)₃⁽⁻⁾,C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁸ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- R¹⁹ is selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups, the alkyl groups optionally being interrupted by one of more hetero-atoms selected from the group consisting of O, N and S, wherein the alkyl groups, (hetero)aryl groups, alkyl(hetero)aryl groups and (hetero)arylalkyl groups are independently optionally substituted, or R¹⁹ is a second occurrence of Q or D connected via a spacer moiety; and
- I is an integer in the range 0 to 10.

In a preferred embodiment of the reactive group according to structure (Q42), R¹⁵ is independently selected from the group consisting of hydrogen, halogen, -OR¹⁶, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and C₁ - C₆ alkyl, most preferably all R¹⁵ are H. In a preferred embodiment of the reactive group according to structure (Q42), R¹⁸ is independently selected from the group consisting of hydrogen, C₁ - C₆ alkyl groups, most preferably both R¹⁸ are H. In a preferred embodiment of the reactive group according to structure (Q42), R¹⁹ is H. In a preferred embodiment of the reactive group according to structure (Q42), I is 0 or 1, more preferably I is 1.

In an especially preferred embodiment, Q comprises a (hetero)cyclooctynyl group and is according to structure (Q43): 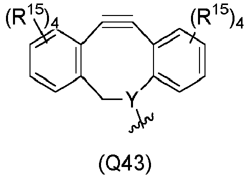 Herein:
- R¹⁵ is independently selected from the group consisting of hydrogen, halogen, -OR¹⁶, -NOz, - CN, -S(O)₂R¹⁶, -S(O)₃⁽⁻⁾, C₁ - C₂₄ alkyl groups, C₅ - C₂₄ (hetero)aryl groups, C₇ - C₂₄ alkyl(hetero)aryl groups and C₇ - C₂₄ (hetero)arylalkyl groups and wherein the alkyl groups, (hetero)aryl groups, alkyl(hetero)aryl groups and (hetero)arylalkyl groups are optionally substituted, wherein two substituents R¹⁵ may be linked together to form an optionally substituted annulated cycloalkyl or an optionally substituted annulated (hetero)arene substituent, and wherein R¹⁶ is independently selected from the group consisting of hydrogen, halogen, C₁ - C₂₄ alkyl groups, C₆ - C₂₄ (hetero)aryl groups, C₇ - C₂₄ alkyl(hetero)aryl groups and C₇ - C₂₄ (hetero)arylalkyl groups;
- Y is N or CR¹⁵;
- a carbon atom in the fused aromatic rings may be replaced by a nitrogen atom, as in (Q6a) - (Q6d), preferably wherein Y is CR¹⁵.

In a preferred embodiment of the reactive group according to structure (Q43), R¹⁵ is independently selected from the group consisting of hydrogen, halogen, -OR¹⁶, -S(O)₃⁽⁻⁾, C₁ - C₆ alkyl groups, C₅ - C₆ (hetero)aryl groups, wherein R¹⁶ is hydrogen or C₁ - C₆ alkyl, more preferably R¹⁵ is independently selected from the group consisting of hydrogen and -S(O)₃⁽⁻⁾. In a preferred embodiment of the reactive group according to structure (Q43), Y is N or CH, more preferably Y = N.

In an alternative preferred embodiment, Q comprises a cyclic alkene moiety. The alkenyl group Q may also be referred to as a (hetero)cycloalkenyl group, i.e. a heterocycloalkenyl group or a cycloalkenyl group, preferably a cycloalkenyl group, wherein the (hetero)cycloalkenyl group is optionally substituted. Preferably, the (hetero)cycloalkenyl group is a (hetero)cyclopropenyl group, a (hetero)cyclobutenyl group, a norbornene group, a norbornadiene group, a *trans-*(hetero)cycloheptenyl group, a *trans*-(hetero)cyclooctenyl group, a *trans*-(hetero)cyclononenyl group or a *trans*-(hetero)cyclodecenyl group, which may all optionally be substituted. Especially preferred are (hetero)cyclopropenyl groups, *trans*-(hetero)cycloheptenyl group or *trans-*(hetero)cyclooctenyl groups, wherein the (hetero)cyclopropenyl group, the *trans-*(hetero)cycloheptenyl group or the *trans*-(hetero)cyclooctenyl group is optionally substituted. Preferably, Q comprises a cyclopropenyl moiety according to structure (Q44), a hetereocyclobutene moiety according to structure (Q45), a norbornene or norbornadiene group according to structure (Q46), a *trans*-(hetero)cycloheptenyl moiety according to structure (Q47) or a *trans-*(hetero)cyclooctenyl moiety according to structure (Q48). Herein, Y³ is selected from C(R²³)₂, NR²³ or O, wherein each R²³ is individually hydrogen, C₁ - C₆ alkyl or is connected to L, optionally via a spacer, and the bond labelled - - - is a single or double bond. In a further preferred embodiment, the cyclopropenyl group is according to structure (Q49). In another preferred embodiment, the trans-(hetero)cycloheptene group is according to structure (Q50) or (Q51). In another preferred embodiment, the *trans*-(hetero)cyclooctene group is according to structure (Q52), (Q53), (Q54), (Q55) or (Q56).

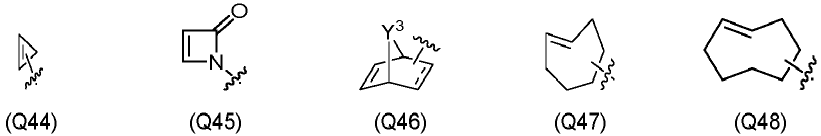

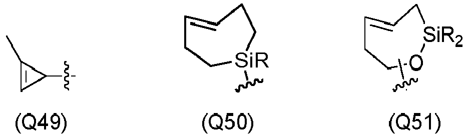

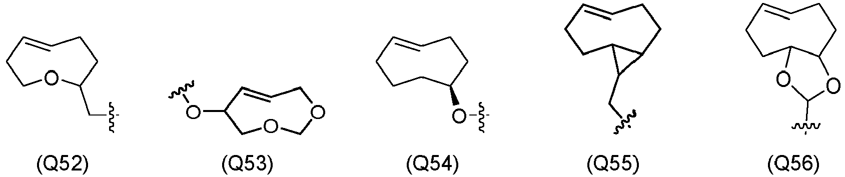

Herein, the R group(s) on Si in (Q50) and (Q51) are typically alkyl or aryl, preferably C₁-C₆ alkyl.

In an alternative preferred embodiment, Q is a thiol-reactive probe. In this embodiment, Q is a reactive group compatible with cysteine conjugation. Such probes are known in the art and may be selected from the group consisting of a maleimide moiety, a haloacetamide moiety, an allenamide moiety, a phosphonamidite moiety, a cyanoethynyl moiety, a vinylsulfone, a vinylpyridine moiety or a methylsulfonylphenyloxadiazole moiety. Most preferably, Q comprises a maleimide moiety. Reagents may be monoalkylation type or may be a cross-linker for reaction with two cysteine side-chains.

In a preferred embodiment, click probe QZ comprise a moiety selected from (Q1) - (Q56), more preferably is a moiety selected from (Q1) - (Q56).

In the present invention, the exact structure of Q¹ and Q² should differ, as Q¹ is not reactive towards F², whereas Q² is reactive towards F².

In a preferred embodiment, F¹ is azide and Q¹ is an benzoannulated or tetramethylated (hetero)cycloalkyne, while F² is tetrazine and Q² is bicyclononyne. Herein, Q¹ is preferably according to structure (Q5), (Q6), (Q7), (Q11), (Q17), (Q18), (Q19) or (Q19a), more preferably according to structure (Q26), (Q27), (Q28), (Q32), (Q37), (Q38) or (Q38a), most preferably according to structure (Q40), (Q41) or (Q43). Herein, Q² is preferably according to structure (Q8), more preferably according to structure (Q29), most preferably according to structure (Q42).

### Reactive moiety F

Reactive moieties F¹ and F² are click probes. In the context of the present invention, F refers to F¹ and F². F is reactive towards and complementary to Q. Herein, a reactive group is denoted as "complementary" to a reactive group when said reactive group reacts with said reactive group selectively, optionally in the presence of other functional groups. Complementary reactive click probes are known to a person skilled in the art, and are described in more detail below. The exact nature of Q, and F, depends on the type of click reaction that is employed. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, click probe F comprises or is an azide moiety or a tetrazine moiety.

F is reactive towards Q in the conjugation reaction defined below, preferably wherein the conjugation reaction is a cycloaddition or a nucleophilic reaction. As the skilled person will understand, the options for F are the same as those for Q, provided that F and Q are reactive towards each other. Thus, F preferably comprises a click probe, a thiol, a thiol-reactive moiety, an amine or an amine-reactive moiety, more preferably F is a click probe, a thiol or an amine, most preferably F is a click probe. The click probe is reactive in a cycloaddition (click reaction) and is preferably selected from an azide, a tetrazine, a triazine, a nitrone, a nitrile oxide, a nitrile imine, a diazo compound, an *ortho*-quinone, a dioxothiophene, a sydnone, an alkene moiety and an alkyne moiety. Preferably, the click probe comprises or is an azide, a tetrazine, a triazine, a nitrone, a nitrile oxide, a nitrile imine, a diazo compound, an *ortho*-quinone, a dioxothiophene or a sydnone, most preferably an azide. Typical thiol-reactive moieties are selected from maleimide moiety, a haloacetamide moiety, an allenamide moiety, a phosphonamidite moiety, a cyanoethynyl moiety, an *ortho*-quinone moiety, a vinylsulfone, a vinylpyridine moiety or a methylsulfonylphenyloxadiazole moiety. Most preferably, the thiol-reactive moiety comprises or is a maleimide moiety. Typical amine-reactive moieties are selected from N-hydroxysuccinimidyl esters, *p*-nitrophenyl carbonates, pentafluorophenyl carbonates, isocyanates, isothiocyanates and benzoyl halides. In a preferred embodiment, F is a click probe or a thiol, more preferably F is an azide or a thiol, most preferably F is an azide.

The reactive group F on the antibody are typically introduced by a specific technique, for example a (bio)chemical or a genetic technique. The reactive group that is placed in the antibody is prepared by chemical synthesis, for example an azide or a terminal alkyne. Methods of preparing modified antibodies are known in the art, e.g. from WO 2014/065661, WO 2016/170186 and WO 2016/053107, which are incorporated herein by reference. From the same documents, the conjugation reaction between the modified antibody and a linker-toxin-construct is known to the skilled person.

Preferably, F is a click probe reactive towards a (hetero)cycloalkene and/or a (hetero)cycloalkyne, and is typically selected from the group consisting of azide, tetrazine, triazine, nitrone, nitrile oxide, nitrile imine, diazo compound, *ortho*-quinone, dioxothiophene and sydnone. Preferred structures for the reactive group are structures (F1) - (F10) depicted here below.

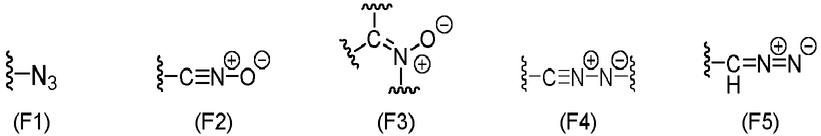

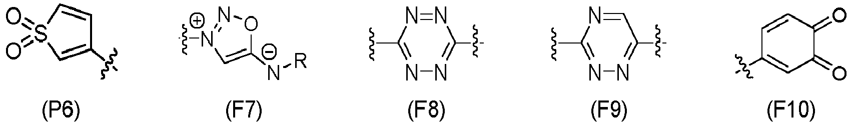

Herein, the wavy bond represents the connection to Ab or L^{A}. For (F3), (F4), (F8) and (F9), the payload can be connected to any one of the wavy bonds. The other wavy bond may then be connected to an R group selected from hydrogen, C₁ - C₂₄ alkyl groups, C₂ - C₂₄ acyl groups, C₃ - C₂₄ cycloalkyl groups, C₂ - C₂₄ (hetero)aryl groups, C₃ - C₂₄ alkyl(hetero)aryl groups, C₃ - C₂₄ (hetero)arylalkyl groups and C₁ - C₂₄ sulfonyl groups, each of which (except hydrogen) may optionally be substituted and optionally interrupted by one or more heteroatoms selected from O, S and NR³² wherein R³² is independently selected from the group consisting of hydrogen and C₁ - C₄ alkyl groups. The skilled person understands which R groups may be applied for each of the groups F. For example, the R group connected to the nitrogen atom of (F3) may be selected from alkyl and aryl, and the R group connected to the carbon atom of (F3) may be selected from hydrogen, alkyl, aryl, acyl and sulfonyl. Preferably, the reactive moiety F is selected from azides or tetrazines.

In an especially preferred embodiment, F is a tetrazine according to structure (F8a):

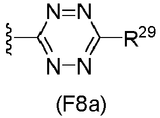

Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C₁₋₆ alkyl and C(O)-NR³³-aryl, wherein R³³ is H or C₁₋₄ alkyl. Preferably, R²⁹ is selected from hydrogen, methyl, phenyl, pyridyl, pyridinyl and pyrimidinyl. It was found that R²⁹ is hydrogen gave optimal results in reactivity in the cycloaddition reaction. Thus, in a preferred embodiment, ring F, in particular F², is (F8a) wherein R²⁹ is selected from hydrogen, methyl, phenyl, pyridyl, pyridinyl and pyrimidinyl, more preferably R²⁹ is hydrogen.

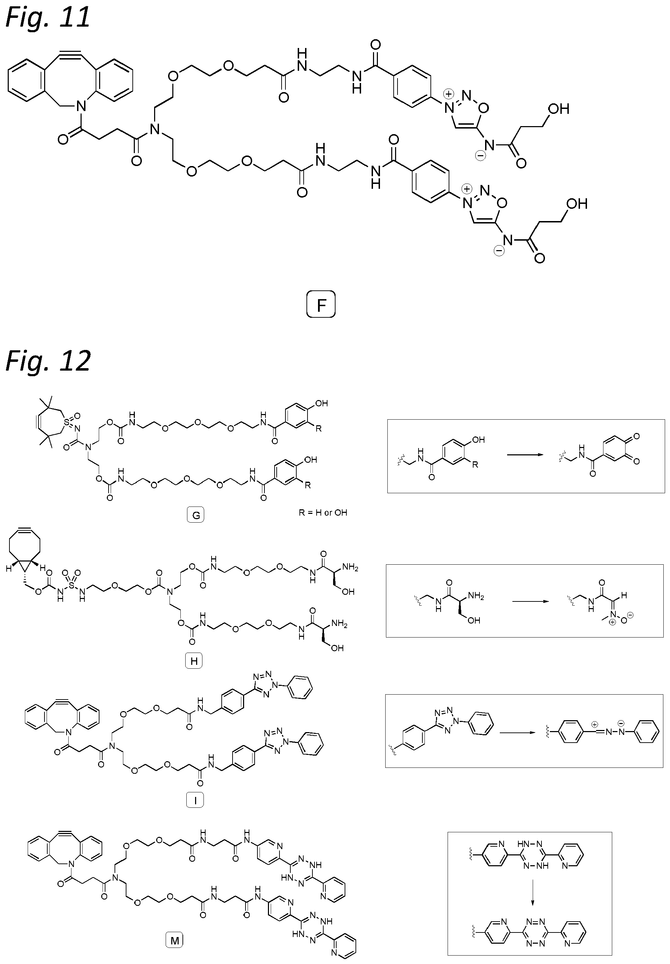

In a preferred embodiment, click probes F¹ and F² are selected from the group consisting of azide, tetrazine, triazine, nitrone, nitrile oxide, nitrile imine, diazo compound, dioxothiophene, sydnone, iminosydnone and catechol. Note that catechol in situ oxidized to an ortho-quinone group, which is reactive as click probe. Likewise, the term "tetrazine" also encompasses "hydrotetrazine", a known precursor that forms tetrazine upon in situ oxidation. Such precursors of click probes, which in situ form reactive groups, are also covered in the present invention. Figure 12 gives some known examples of in situ formed click probes by oxidation. In a preferred embodiment, F¹ is an azide and F² is iminosydnone, catechol, which forms a *ortho*-quinone group in situ, or tetrazine. More preferably, F² is iminosydnone according to structure (F7), catechol, which forms structure (F10) in situ, or tetrazine according to structure (F8). Most preferably, F¹ is an azide according to structure (F1) and F² is a tetrazine according to structure (F8a).

### Linker-construct according to general structure (4)

In a further aspect, the invention concerns a linker-construct. The linker-construct according to the invention is a heterobifunctional (y + 1)-valent linker of structure (4):

Q¹L^{A}(F²)_{y} (**4**)

Herein, Q¹ is a click probe, L^{A} is a linker, y is 2, 3 or 4, and F² is a click probe that is not reactive towards Q¹. Q¹, L^{A}, y and F² are defined elsewhere, and those definitions and preferred embodiments thereof are equally applicable to the linker-construct according to the invention.

In a preferred embodiment of the linker-construct according to the present aspect, Q¹ is a benzoannulated or tetramethylated (hetero)cycloalkyne. Such a click probe Q¹ is preferably combined with a tetrazine as click probe F². As such, both Q¹ and F² are click probes reactive in a cycloaddition reaction, but are not reactive towards each other.

Q¹ is preferably selected from (Q5), (Q6), (Q6a), (Q6b), (Q6c), (Q6d), (Q7), (Q18) and (Q19a) as defined above. F² is preferably a tetrazine according to structure (F8a): 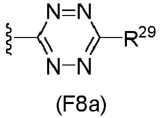 Herein, R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C¹⁻⁶ alkyl and C(O)-NR³³-aryl, wherein R³³ is H or C₁₋₄ alkyl, preferably wherein R²⁹ is selected from hydrogen, methyl, phenyl, pyridyl, pyridinyl and pyrimidinyl.

Integer y is 2, 3 or 4, preferably y is 2 or 3, most preferably y is 2.

### Application

The conjugates of the present invention are especially suitable in the treatment of cancer. In that light, the invention further concerns a method for the treatment of cancer, comprising administering to a subject in need thereof the conjugate according to the invention. The subject in need thereof is typically a cancer patient. The use of conjugates, such as antibody-drug conjugates, is well-known in the field of cancer treatment, and the conjugates according to the invention are especially suited in this respect. The method as described is typically suited for the treatment of cancer. In the method according to this aspect, the antibody-conjugate is typically administered in a therapeutically effective dose. A preferred dose for administration of the conjugates according to the invention is in the range of 3 -20 mg per kg bodyweight, every three weeks, or every two weeks, or every week, preferably every three weeks. The present aspect of the invention can also be worded as a conjugate according to the invention for use in the treatment of cancer. In other words, this aspect concerns the use of a conjugate according to the invention for the preparation of a medicament or pharmaceutical composition for use in the treatment of cancer. In the present context, treatment of cancer is envisioned to encompass treating, imaging, diagnosing, preventing the proliferation of, containing and reducing tumours.

This aspect of the present invention may also be worded as a method for targeting a tumour cell expressing a specific extracellular receptor, comprising contacting the conjugate according to the invention with cells that may possibly express the extracellular receptor, and wherein the antibody specifically targets the extracellular receptor. The method according to this aspect is thus suitable to determine whether the cells are expressing the desired extracellular receptor. These tumour cells may be present in a subject, in which case the method comprises administering to a subject in need thereof the conjugate according to the invention. Alternatively, the method occurs *ex vivo* or *in vitro.* In a preferred embodiment, the cells that may possibly express the extracellular receptor are cells that express the extracellular receptor. The targeting of tumour cells preferably includes one or more of treating, imaging, diagnosing, preventing the proliferation of, containing and reducing the tumour cells.

In the context of diagnosis, it is typically unknown whether the cells that are being contacted in fact express the specific extracellular receptor that is being investigated. For example, in the diagnosis of HER2-positive breast cancer, a conjugate containing an antibody that targets HER2, such as trastuzumab, may be contacted with the cells. In case the tumour cells are in fact HER2-expressing, the conjugate will target the cells, while in case the tumour cells are not HER2-expressing, the conjugate will not target the cells. Likewise, in the treatment of a cancer cells specifically expressing an extracellular receptor, the skilled person will understand that a cell-binding agent, such as an antibody, is to be used that targets that specific extracellular receptor.

In the methods of the present invention, it is preferred that the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, Brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha, Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSCA hlg, PSMA, PTK7, RET, RNF43, RON, ROR1, ROR2, Sema 5b, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin. Likewise, it is preferred that the tumour cells express an extracellular receptor selected from the same group. The skilled person is capable of matching the desired extracellular receptor with a suitable cell-binding agent capable of targeting that extracellular receptor.

The conjugates of the present invention are also especially suitable as antibiotic, antiviral, anti-inflammatory and anti-autoimmune agent. Thus, in an alternative embodiment, the invention concerns a method for the treatment of infection, inflammation of an autoimmune disorder, comprising administering to a subject in need thereof the conjugate according to the invention. In the method according to this aspect, the antibody-conjugate is typically administered in a therapeutically effective dose. The present aspect of the invention can also be worded as a conjugate according to the invention for use in the treatment of infection, inflammation of an autoimmune disorder. In other words, this aspect concerns the use of a conjugate according to the invention for the preparation of a medicament or pharmaceutical composition for use in the treatment of infection, inflammation of an autoimmune disorder. Herein, the infection may be bacterial or viral.

In the present embodiment, the antibody is preferably specific to an extracellular protein resulting from a viral infection and/or tumour-associated carbohydrate antigen. Herein, the extracellular protein resulting from a viral infection may be human polio virus (HPV), human cytomegalovirus (HCMV) or human papillomavirus (HPV). The tumour-associated carbohydrate antigen (TACA) may be selected from the group of Tn, STn, T-antigen, LDN, Lewis^{c} (Le^{c}), Sialyl-Lewis^{c} (SLe^{c}), 6-Sialyl-Lewis^{c} (6SLe^{c}), LN, alpha-Gal, 3SLN, 6SLN, H-antigen, A-antigen, B-antigen, Lewis^{a} (Le^{a}), Sialyl-Lewis^{a} (SLe^{a}), 6-Sialyl-Lewis^{a} (6SLe^{a}), Lewis^{b} (Le^{b}), Sialyl-Lewis^{b} (SLe^{b}), 6-Sialyl-Lewis^{b} (6SLe^{b}), Lewis" (Le^{x}), Sialyl-Lewis^{x} (SLe^{x}), 6-Sialyl-Lewis^{x} (6SLe^{x}), Lewis^{y} (Le^{y}), Sialyl-Lewis^{Y} (SLe^{y}), 6-Sialyl-Lewis^{y} (6SLe^{y}) and or combinations thereof. The antibody may also be specific to both an extracellular protein and a TACA at the same time.

In this light, the invention also concerns a pharmaceutical composition comprising the conjugate according to the invention and a pharmaceutically acceptable carrier. The pharmaceutical composition typically contains the conjugate according to the invention in a pharmaceutically effective dose.

### Description of the figures

Figure 1 shows the general scheme for preparation of antibody-drug conjugates by reaction of a monoclonal antibody (in most cases a symmetrical dimer) containing an x number of functionalities F. By incubation of antibody-(F)ₓ with excess of a linker-drug construct (Q-spacer-linker-payload) a conjugate is obtained by reaction of F with Q, forming connecting group Z.
Figure 2 depicts a range of reagents suitable for reaction with cysteine side-chains. Reagents may be monoalkylation type (A) or may be a cross-linker (B) for reaction with two cysteine side-chains.
Figure 3 shows the general process for non-genetic conversion of a monoclonal antibody (mAb) into an antibody containing probes for click conjugation (F). The click probe may be on various positions in the antibody, depending on the technology employed. For example, the antibody may be converted into an antibody containing two click probes (structure on the left) or four click probes (bottom structure) or eight probes (structure on the right) for click conjugation.
Figure 4 shows a representative (but not comprehensive) set of functional groups (F) that can be introduced into an antibody by engineering, by chemical modification, or by enzymatic means, which upon metal-free click reaction with a complementary reactive group Q lead to connecting group Z. Functional group F may be artificially introduced (engineered) into an antibody at any position of choice. Some functional groups F (*e*.*g*. nitrile oxide, quinone), may besides strained alkynes also react with strained alkenes, which as an example is depicted for triazine or tetrazine (bottom line). The pyridine or pyridazine connecting group is the product of the rearrangement of the tetrazabicyclo[2.2.2]octane connecting group, formed upon reaction of triazine or tetrazine with alkyne (but not alkene), respectively, with loss of N₂. Connecting groups Z depicted in Figure 4 are preferred connecting groups to be used in the present invention.
Figure 5 shows cyclic alkynes suitable for metal-free click chemistry, and preferred embodiments for reactive moiety Q. The list is not comprehensive, for example alkynes can be further activated by fluorination, by substitution of the aromatic rings or by introduction of heteroatoms in the aromatic ring.
Figure 6 depicts a specific example of site-specific conjugation of a payload based on glycan remodeling of a full-length IgG followed by azide-cyclooctyne click chemistry. The IgG is first enzymatically remodeled by endoglycosidase-mediated trimming of all different glycoforms, followed by glycosyltransferase-mediated transfer of azido-sugar onto the core GlcNAc liberated by endoglycosidase. In the next step, the azido-remodeled IgG is subjected to an immune cell-engaging polypeptide, which has been modified with a single cyclooctyne for metal-free click chemistry (SPAAC), leading to a bispecific antibody of 2:2 molecular format. It is also depicted that the cyclooctyne-polypeptide construct will have a specific spacer between cyclooctyne and polypeptide, which enables tailoring of IgG-polypeptide distance or impart other properties onto the resulting bispecific antibody.
Figure 7 depicts a specific example of site-specific conjugation of a payload based on glycan remodeling of a full-length IgG followed by thiol alkylation chemistry. The IgG is first enzymatically remodeled by endoglycosidase-mediated trimming of all different glycoforms, followed by glycosyltransferase-mediated transfer of a thiol-modified (and disulfide-protected) sugar derivative onto the core GIcNAc liberated by endoglycosidase. In the next step, the remodeled IgG is subjected to reduction (to convert the disulfide into thiol), potentially followed by oxidation, then reaction with a payload modified with a suitable thiol-reactive reagent.
Figure 8 describes the two-stage process as applied herein, whereby a glycan-remodeled antibody with functionality F¹ is reacted with a bifunctional linker Q₁-L-F₂ (wherein the click probes Q₁ and F₂ are mutually non-reactive), thereby undergoing a metal-free click chemistry reaction to form bond Z₁. The bifunctional linker may comprise 2 to 4 occurrences of F₂ (*i*.*e*. trivalent, tetravalent or pentavalent linker). In the second step, the antibody containing the reactive group F₂ is reacted with click probe Q₂, which is part of the linker-drug containing 1-3 occurrences of drug.
Figure 9 depicts three examples of a bifunctional linker Q₁-L-F₂ that fulfill the condition that Q₁ and F₂ are not mutually reactive, where Q₁ is either DBCO (as in **A**) or TMTHSI (as in **B** and **C**) and F₂ is a tetrazine analogue (as in **A-C**).
Figure 10 shows two examples of a bifunctional linker Q₁-L-F₂ that fulfill the condition that Q₁ and F₂ are not mutually reactive, where Q₁ is either TMTHSI (as in **D**) or DBCO (as in **E**), the tetrazine is a alkyl,methyltetrazine as in **D**) or a phenyltetrazine (as in **E**) and where the linker is either tetravalent (as in **D**) or pentavalent (as in **E**).
Figure 11 depicts an example of a bifunctional linker Q₁-L-F₂ (**F**), where Q₁ is DBCO and F₂ is an iminosydnone variant known to react with DBCO extremely slowly (k < 0.001 M⁻¹S⁻¹).
Figure 12 shows three examples of a bifunctional linker Q₁-L-F₂, whereby F₂ is a latent click-reactive group (*i*.*e*. not reactive as such but requiring chemical or enzymatic conversion to a reactive click probe), such as phenol or catechol in compound **G** (can be converted into *ortho*-quinone upon treatment with tyrosinase or NaIO₄, respectively), such as serine in compound **H** (can be converted into a nitrone upon treatment with NaIO₄, then N-methylhydroxylamine), such as tetrazole in compound I (can be converted into nitrile imine upon treatment with 200 nm light) or such as dihydrotetrazine in compound **M** (can be converted into tetrazine upon treatment with horseradish peroxidase or 660 nm light).
Figure 13 shows several examples of preferred linker-constructs Q₁-L-F₂.

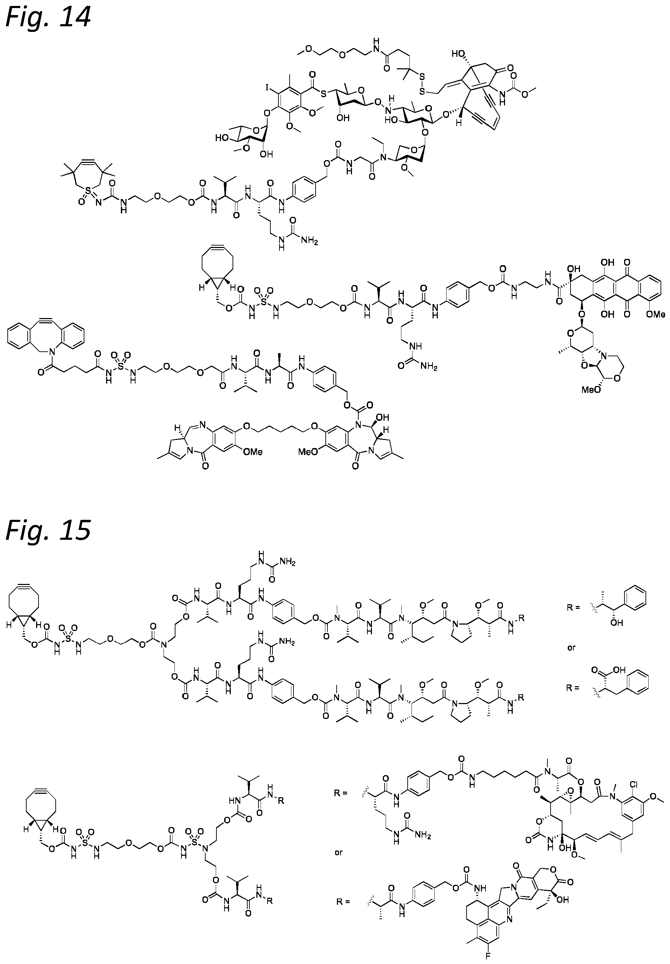

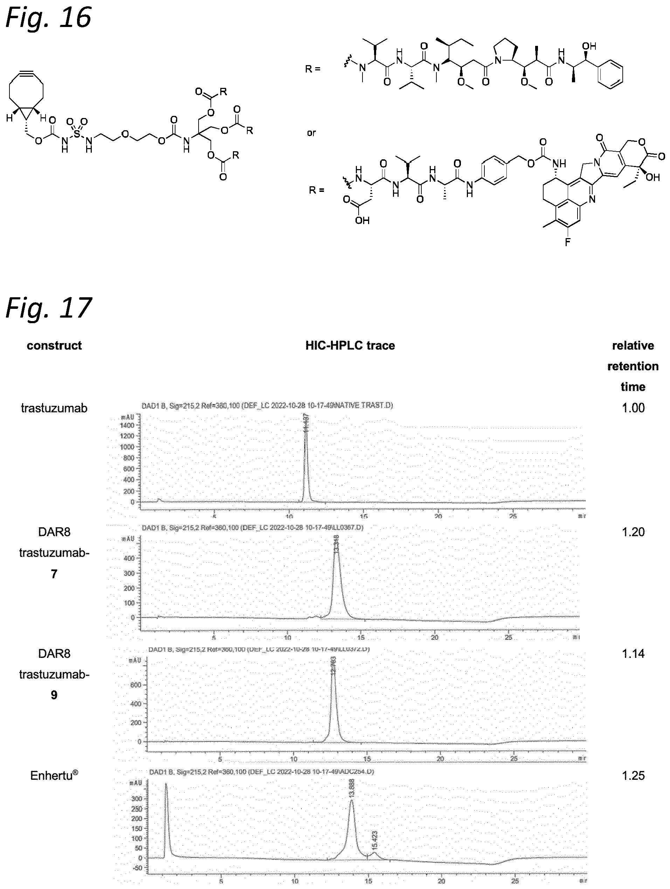

Figures 14, 15 and 16 show several examples of preferred linker-payload constructs Q²(L^{B})Dₓ, with x = 1 (Fig. 14), x = 2 (Fig. 15) and x = 3 (Fig. 16).
Figure 17 depicts the relative retention times (rrt) of various ADCs versus trastuzumab measured with HIC-HPLC, showing rrt = 1.14 for DAR4 and rrt = 1.20 for DAR8, while Enhertu^{®} as comparator shows rrt = 1.25.

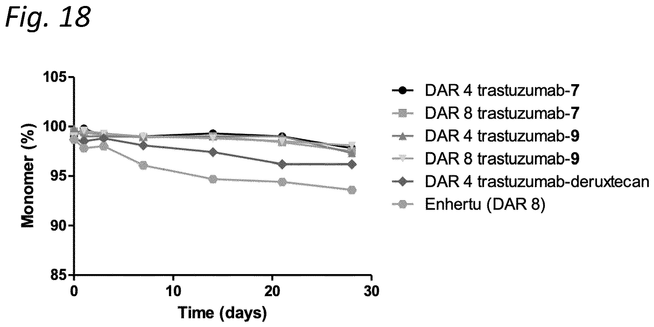

Figure 18 depicts monomer levels of various ADCs at physiological conditions, PBS at 37 °C.

### Examples

The invention is illustrated by the following examples.

### General reagents and analytics

General procedure for analytical RP-UPLC (DTT treated samples): Prior to RP-UPLC analysis, IgG (10 µL, 1 mg/mL in PBS pH 7.4) was added to 12.5 mM DTT, 100 mM TrisHCl pH 8.0 (40 µL) and incubated for 15 minutes at 37 °C. The reaction was quenched by adding 49% acetonitrile, 49% water, 2% formic acid (50 µL). RP-UPLC analysis was performed on a Waters Acquity UPLC-SQD. The sample (5 µL) was injected with 0.4 mL/min onto Bioresolve RP mAb 2.1*150 mm 2.7 µm (Waters) with a column temperature of 70 °C. A linear gradient was applied in 9 minutes from 30 to 54% acetonitrile in 0.1% TFA and water. Absorbance of eluted peaks was measured at 215 nm followed by automated integration (MassLynx, Waters) to determine reaction conversion.

General procedure for mass spectral analysis of (modified) monoclonal antibodies: Prior to mass spectral analysis, IgG was treated with IdeS, which allows analysis of the Fc/2 fragment. For analysis of both light and heavy chain, a solution of 20 µg (modified) IgG was incubated for 5 minutes at 37 °C with 100 mM DTT in a total volume of 4 µL. If present, azide-functionalities are reduced to amines under these conditions. For analysis of the Fc/2 fragment, a solution of 20 µg (modified) IgG was incubated for 1 hour at 37 °C with IdeS/Fabricator^{™} (1.25 U/µL) in phosphate-buffered saline (PBS) pH 6.6 in a total volume of 10 µL. Samples were diluted to 80 µL followed by analysis electrospray ionization time-of-flight (ESI-TOF) on a JEOL AccuTOF. Deconvoluted spectra were obtained using Magtran software.

*General procedure for analytical SEC analysis:* HPLC-SEC analysis was performed on an Agilent 1100 series (Hewlett Packard) using an Xbridge BEH200A (3.5 µM, 7.8x300 mm, PN 186007640 Waters) column. The sample was diluted to 1 mg/mL in PBS and measured with 0.86 mL/min isocratic method (100 mM sodium phosphate (NaHPO₄/Na₂PO₄), 200 mM NaCl, pH 6.8, containing 10% isopropanol) for 17 minutes.

General procedure for enzymatic remodeling of IgG to mAb-(6-N₃-GalNAc)₂: IgG (15 mg/mL) was incubated with 1% w/w EndoSH (as described in PCT/EP2017/052792, see Examples 1 - 3, and SEQ. ID No: 1, which is incorporated by reference herein), 3% w/w His-TnGalNAcT (as described in PCT/EP2016/059194, see Examples 3 and 4, and SEQ. ID No: 49, which is incorporated by reference herein), 0.01% AP (Roche) and UDP 6-N₃-GalNAc (compound **2d** in Figure 3, 10 eq compared to IgG) in 6 mM MnCl₂ and TBS for 16 hours at 30 °C. Next, the functionalized IgG was purified using a HiTrap MabSelect sure 5 mL column. After loading of the reaction mixture the column was washed with TBS+0.2% triton and TBS. The IgG was eluted with 0.1 M AcOH pH 2.7 and neutralized with 2.5 M Tris-HCl pH 8.8. After three times dialysis to 20 mM histidine, 150 mM NaCl pH 7.5, the IgG was concentrated to 15-20 mg/mL using a Vivaspin Turbo 15 ultrafiltration unit (Sartorius).

### Examples 1-3: Synthesis of click probe 3 and linker conjugate 7 and 9

### Example 1: Preparation of compound 3

To a solution of compound **1** (10.0 mg, 1 Eq, 12.2 µmol) in anhydrous dry DMF (850 µL) was added compound **2** (10.3 mg, 3 Eq, 36.6 µmol), followed by triethylamine (6.18 mg, 8.51 µL, 5 Eq, 61.1 µmol). The reaction mixture was stirred at room temperature for 2 hours. The reaction mixture was purified by prep-HPLC; (30% --> 100%, Acetonitrile/ Water + 10 mM NH4HCO3, runtime 21 minutes, Column Xbridge prep C18 5 um OBD, 30x100 mm) to afford compound **3** (5 mg, 4.7 µmol, 38%) as a pink solid. LCMS (ESI+) calculated for C₅₇H₆₇N₁₂O₁₀⁺ (M+H)⁺ 1079.5, found 1079.9.

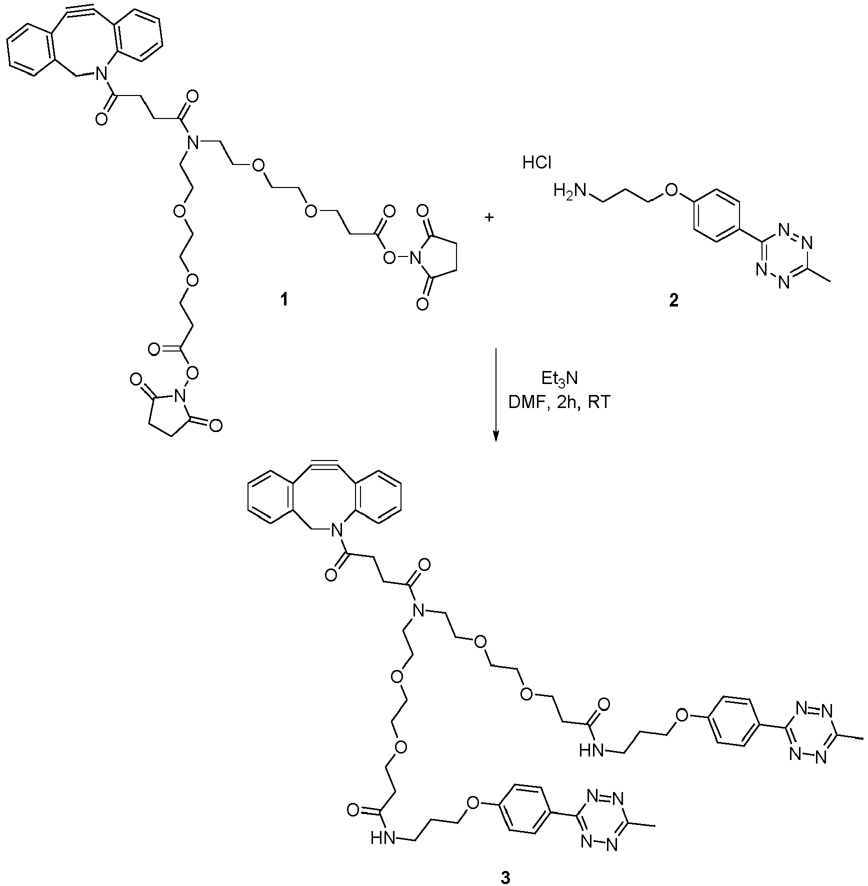

### Example 2: Preparation of compound 5

Compound **4** (163 mg, 240 µmol) was added to a mixture of exatecan mesylate (125 mg, 235 µmol) and DIPEA (61 mg, 82 µL, 0.47 mmol) in dry DMF (0.9 mL). After 20 h, the reaction mixture was diluted to 9 mL DCM and purified by gradient column chromatography (0 → 40% MeOH/DCM) to afford **5** (155 mg, 159 µmol, 68%). LCMS (ESI+) calculated for C₅₅H₅₄FN₆O₁₀⁺ (M+H)⁺ 977.39, found 977.72. In addition to **5,** free base of exatecan (82.4 mg, 189 µmol, 20%) was recovered. LCMS (ESI+) calculated for C₂₄H₂₃FN₃O₄⁺ (M+H)⁺ 436.46, found 436.54.

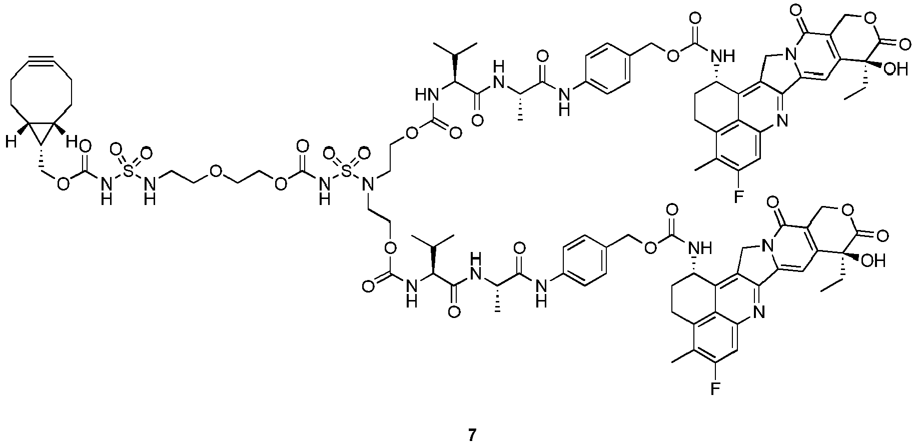

### Example 3. Preparation of compound 7

The synthesis of BCN-HS-(va-PABC-Ex)₂ (**7**) is also described in PCT/EP2021/075401 (example 4), incorporated herein. To a solution of compound **5** (155 mg, 159 µmol) in DMF (1.6 mL) were added EtsN (73 mg, 101 µL, 0.72 mmol) and a solution of compound **6** (65 mg, 72 µmol) in DMF (1.4 mL). The reaction mixture was stirred for 18 h, diluted with DCM (20 mL) and purified by gradient column chromatography (0 → 40% MeOH/DCM) to afford **7** as a pale-yellow solid (94 mg, 44 µmol, 28%). LCMS (ESI+) calculated for C₁₀₂H₁₁₈F₂N₁₆O₂₉S₂ ²⁺ (M/2+H)⁺ 1066.88, found 1067.12.

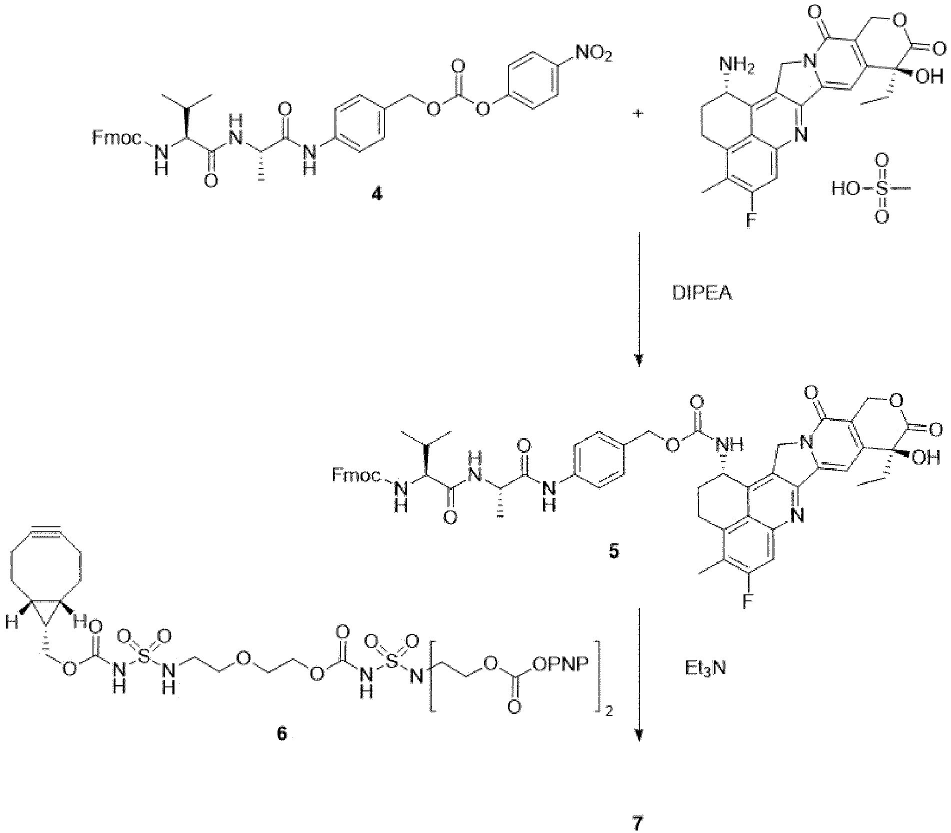

### Example 4. Preparation of compound 9

A solution of BCN-HS-PEG₂-b-(Glu(OFm)-OH)₂ (**8**, 12.1 mg, 10 µmol, 1.0 eq) dissolved in anhydrous DMF (180 µL) was added to a solution of NHz-Val-Ala-PABC-exatecan (**5b**, Fmoc-deprotected **5,** 19 mg, 25 µmol, 2.5 eq) in anhydrous DCM (180 µL), DIPEA (11 µL, 63 µmol, 6.2 eq) and HATU (8.9 mg, 23 µmol, 2.3 eq). After stirring for 2 h at room temperature, the reaction mixture was further diluted with DCM (800 µL) and purified by flash column chromatography over silicagel (0% → 20% MeOH in DCM) to give the product as a clear oil (difficult to determine yield due to presence of DMF). LCMS (ESI+) calculated for C₁₄₀H₁₅₀FzN₁₇O₃₃S⁺ (M/2+H⁺) 1334.01, found 1334.79.

This compound was dissolved in DMF (300 µL) and triethylamine (21 µL, 150 µmol, 15 eq) was added. After 17 h at room temperature, the reaction mixture was diluted with DCM (700 µL) and purified by flash column chromatography over silicagel (0% ---> 45% MeOH in DCM) to give compound 9 in 44% yield as a yellow solid (10.2 mg, 4.4 µL). LCMS (ESI+) calculated for C₁₁₂H₁₃₀F₂N₁₇O₃₃S⁺ (M/2+H⁺) 1156.2, found 1156.74.

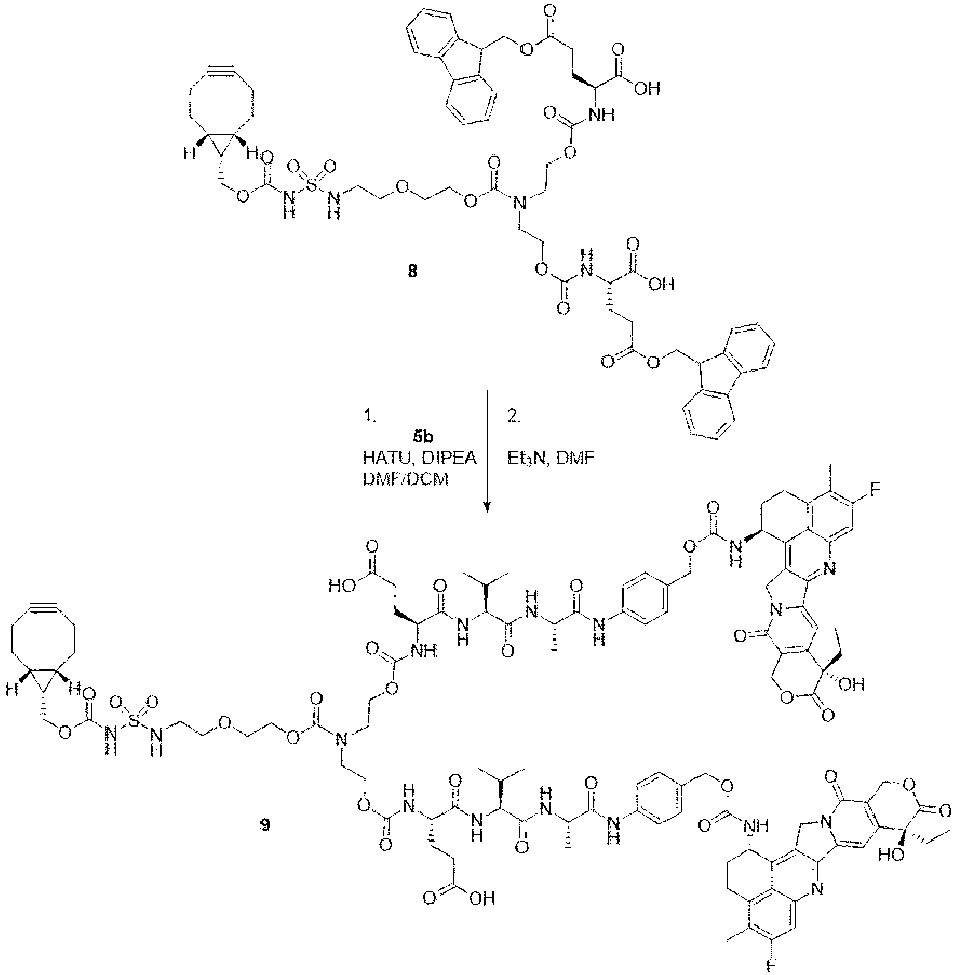

### Example 5. Preparation of compound 11

Diethanolamine (894.1 mg, 816.5 µL, 1.1 Eq, 8.504 mmol) and sodium carbonate (1.188 g, 1.45 Eq, 11.21 mmol) were added to 1,4-Dioxane (20 mL) and Water (20 mL). The mixture was stirred at room temperature to form a suspension. (9H-fluoren-9-yl)methyl carbonochloridate (2000 mg, 1 Eq, 7.731 mmol) was dissolved in Toluene (5 mL) and added dropwise to the above reaction solution (the internal temperature is controlled not to exceed 25 ° C during the dropwise addition) and stirring was continued for 2 hours. The pH was adjusted to 3 with 4 M HCl(aq) and the aqueous phase was extracted twice with ethyl acetate (50 mL). The organic phase was washed saturated brine (2x 50 mL), dried over anhydrous sodium sulfate and concentrated in vacuo to afford **10** (3.13 g, 7.6 mmol, 99 %, 80% Purity) as an opaque oil. LCMS (ESI+) calculated for C₁₉H₂₂NO₄⁺ (M+H⁺) 328.2, found 928.3.

Compound **10** (1260 mg, 1 Eq, 3.849 mmol) was dissolved in dry DCM (50 mL). Then, bis(perfluorophenyl) carbonate (3.185 g, 2.1 Eq, 8.082 mmol) was added followed by DIPEA (1.492 g, 2.01 mL, 3 Eq, 11.55 mmol) and stirring was continued for 3 hours. The residue was then purified by by flash column chromatography over silicagel (0-10% Acetone in DCM) to afford 11 (2.5 g, 3.3 mmol, 87 %, 100% Purity) as a clear oil. LCMS (ESI+) calculated for C₃₃H₂₀F₁₀NO₈⁺ (M+H⁺) 748.1, found 748.4.

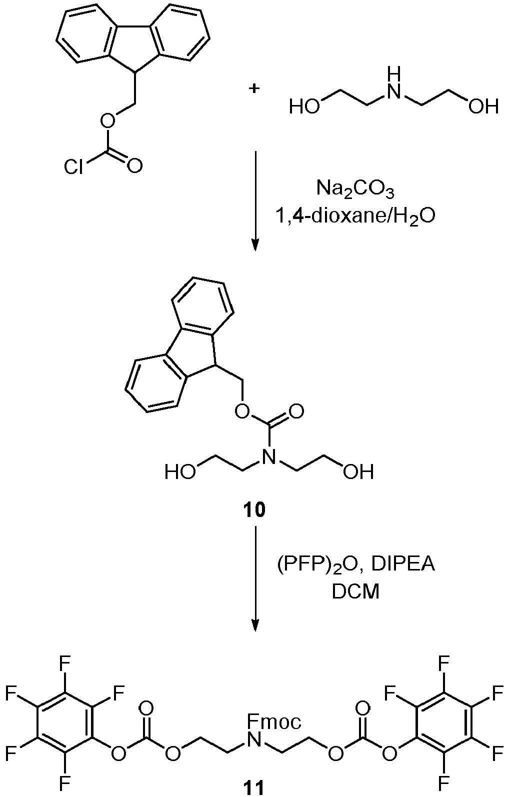

### Example 6. Preparation of compound 13

A solution of 2,2'-(ethane-1,2-diylbis(oxy))bis(ethan-1-amine) (3.40 g, 3.35 mL, 10 Eq, 22.9 mmol) in DCM (40 mL) was cooled to 0 °C. To this di-tert-butyl dicarbonate (500 mg, 1 Eq, 2.29 mmol) was added and the mixture was stirred at room temperature for 18 hours. The organic phase was washed with water (3 × 40 mL), until all the unreacted 2,2'-(ethane-1,2-diylbis(oxy))bis(ethan-1-amine) was extracted. The organic phase was dried (Na2SO4) and concentrated in vacuo to afford **12** (361 mg, 1.3 mmol, 57 %, 90% Purity) as a yellow oil. LCMS (ESI+) calculated for C₁₁H₂₅N₂O₄⁺ (M+H⁺) 249.2, found 249.4.

To a solution of amine **12** (150.0 mg, 1 Eq, 200.7 µmol) in dry DCM (5.00 mL) was added EP0139A (109.6 mg, 2.2 Eq, 441.5 µmol), followed by DIPEA (129.7 mg, 175 µL, 5 Eq, 1.003 mmol). The reaction mixture was stirred at room temperature for 1h. The reaction mixture was concentrated in vacuo. The resulting crude was dissolved in DCM (1 mL) and was loaded onto the column for purification flash column chromatography over silicagel (0-20% MeOH in DCM) afford the Boc-protected intermediate (218 mg, 0.20 mmol, 99 %, 80% Purity) as a colorless oil. LCMS (ESI+) calculated for C₄₃H₆₆N₅O₁₄⁺ (M+H⁺) 876.5, found 876.7.

To a solution of the intermediate (176 mg, 1 Eq, 201 µmol) in dry DCM (5.00 mL) was added HCl in dioxane (147 mg, 1.00 mL, 4 molar, 20 Eq, 4.02 mmol).The reaction mixture was stirred at room temperature for 18h.The reaction mixture was concentrated in vacuo to afford the HCl salt **13** (150 mg, 200 µmol, 99 %) as an off-white foam. LCMS (ESI+) calculated for C₃₃H₅₀N₅O₁₀⁺ (M+H⁺) 676.4, found 676.6.

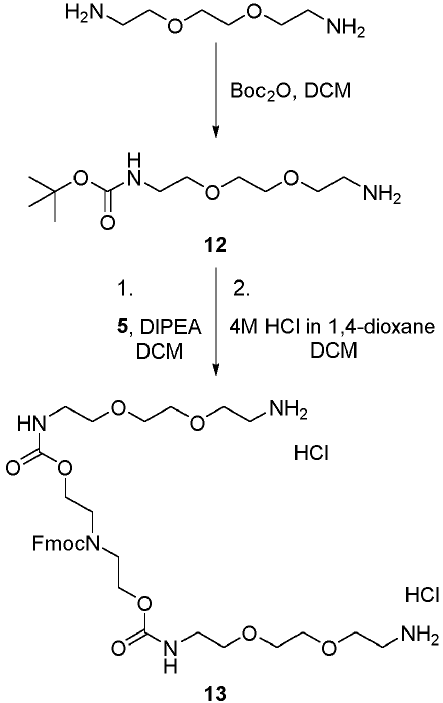

### Example 7. Preparation of compound 15

To a solution of sulfo-D-alanine hydrate (554 mg, 1 Eq, 2.96 mmol) and triethylamine (599 mg, 825 µL, 2 Eq, 5.92 mmol) in DMF (15 mL) was added di-tert-butyl dicarbonate (775 mg, 1.2 Eq, 3.55 mmol).The reaction mixture was stirred at room temperature for 3 h. The solvent was evaporated in vacuo. The residue was redissolved in CH2Cl2 (10 mL) and treated with Et2O (30 mL) under vigorous stirring. The organic solvents were decanted and discarded. The oily residue was washed with Et2O (20 mL) and dried in vacuo to give **14** (1134 mg, 2.9 mmol, 100 %, 70% Purity) as a colorless oil. LCMS (ESI-) calculated for C₈H₁₄NO₇S⁻ (M-H⁻) 268.1, found 268.3.

To a solution of N-Boc-cysteic acid **14** (54 mg, 3 Eq, 0.20 mmol) in DMF (1 mL) was added compound **13** (50 mg, 1 Eq, 67 µmol), HATU (76 mg, 3 Eq, 0.20 mmol) and DIPEA (52 mg, 70 µL, 6 Eq, 0.40 mmol) and stirring was continued for 2 hours. The mixture was concentrated in vacuo and the crude dissolved in DCM (10 ml). The mixture was purified by flash column chromatography over silicagel (0% --> 70% MeOH in DCM) to afford Boc-protected intermediate (50 mg, 36 µmol, 53 %, 84% Purity) as a colorless oil. LCMS (ESI+) calculated for C₄₉H₇₅N₇O₂₂S₂⁺ (M+H⁺) 1178.4, found 1178.7.

To a solution of the Boc-protected intermediate (50 mg, 1 Eq, 42 µmol) in dry DCM (2.00 mL) and MeOH (1.00 mL) was added hydrogen chloride in 1,4-Dioxane (46 mg, 0.32 mL, 4 molar, 30 Eq, 1.3 mmol). The reaction mixture was stirred at room temperature for 26h after which additional hydrogen chloride in Dioxane (46 mg, 0.32 mL, 4 molar, 30 Eq, 1.3 mmol) was added to the reaction mixture. Stirring was continued for an additional hour upon which the reaction mixture was concentrated in vacuo to afford **15** (45 mg, 43 µmol, 100 %) as an off-white solid. LCMS (ESI+) calculated for C₃₉H₆₀N₇O₁₈S₂⁺ (M+H⁺) 978.3, found 978.6.

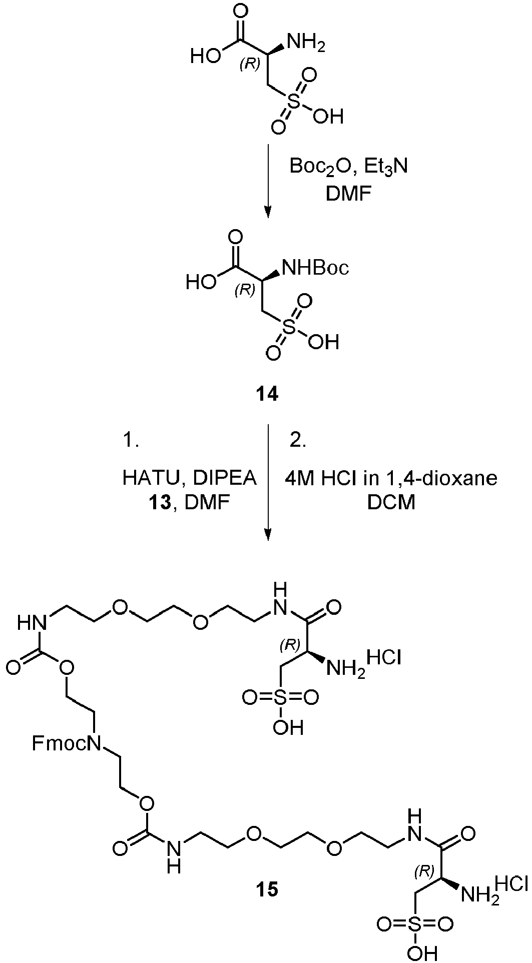

### Example 8. Preparation of compound 17

To a solution of 4-(6-methyl-1 ,2,4,5-tetrazin-3-yl)butanoic acid (4.3 mg, 2.5 Eq, 24 µmol) in DMF (1 mL) was added HATU (11 mg, 3 Eq, 29 µmol) and DIPEA (7.4 mg, 9.9 µL, 6 Eq, 57 µmol). After 10 minutes HCl salt **15** (10 mg, 1 Eq, 9.5 µmol) was added and stirring was continued for 1 hour. The reaction mixture was concentrated in vacuo and the crude dissolved in MeCN (1 ml). The solution was purified by Reverse phase chromatography (10% --> 100%, Acetonitrile in Water) to afford **16** (7 mg, 5 µmol, 50 %, 95% Purity) as a pink solid. LCMS (ESI+) calculated for C₅₃H₇₆N₁₅O₂₀S₂⁺ (M+H⁺) 1306.5, found 1306.7.

Triethylamine (11 mg, 15 µL, 20 Eq, 0.11 mmol) was added to a solution of compound **16** (7.0 mg, 1 Eq, 5.4 µmol) in DMF (0.200 mL) and H2O (10 µL). The reaction mixture was stirred for 18 h until complete Fmoc deprotection was observed. TMTHSI-OSu (2.7 mg, 1.5 Eq, 8.0 µmol) was added to the reaction mixture and stirring was continued for 4 days.The reaction mixture was purified by prep-HPLC; (30% --> 100%, Acetonitrile/ Water + 1% AcOH, runtime 21 minutes, Column Xbridge prep C18 5 um OBD, 30x100 mm) to afford **17** (2.5 mg, 1.9 µmol, 36 %) as a pink solid. LCMS (ESI-) calculated for C₄₉H₇₈N₁₆O₂₀S₃⁻² (M/2-H⁻) 653.2, found 653.6.

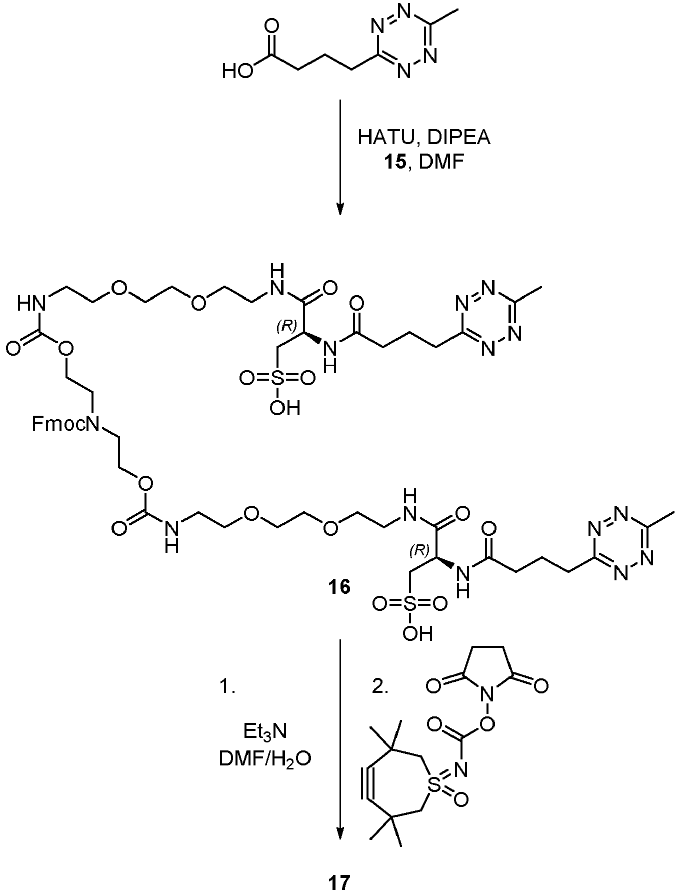

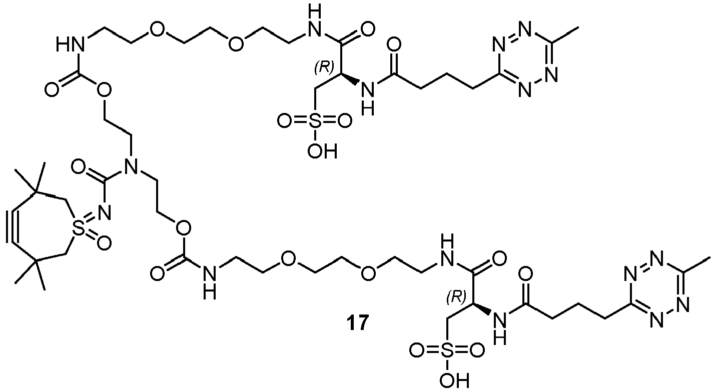

### Example 9. Preparation of compound 19

To a solution of compound **11** (50.0 mg, 1 Eq, 66.9 µmol) in dry DMF (1.00 mL) was added 3-(2-(2-aminoethoxy)ethoxy)propanoic acid (26.1 mg, 2.2 Eq, 147 µmol), followed by DIPEA (43.2 mg, 334 µL, 5 Eq, 334 µmol). After stirring for 18 hours, the reaction mixture was concentrated in vacuo. The resulting crude was dissolved in DCM (3 mL) and was loaded onto the column for purification by flash column chromatography over silicagel (0-10% MeOH in DCM with 1% AcOH) to afford **18** (55.3 mg, 75.4 µmol, 113 %) as a colorless oil. LCMS (ESI+) calculated for C₃₅H₄₈N₃O₁₄⁺ (M+H⁺) 734.3, found 734.6.

DCC (32.3 mg, 2.3 Eq, 157 µmol) and 1-hydroxypyrrolidine-2,5-dione (18.0 mg, 2.3 Eq, 157 µmol) were added to a solution of compound **18** (50.0 mg, 1 Eq, 68.1 µmol) dissolved in DCM (2 mL). And DMF (2 mL). The reaction mixture was stirred for 18 h at room temperature. sulfo-L-alanine (34.6 mg, 3 Eq, 204 µmol) and DIPEA (105.6 mg, 142.4 µL, 12 Eq, 818 µmol) were added to the reaction mixture and stirring was continued for 24 hours. The mixture was filtered, and the filtrate was concentrated under reduced pressure. The resulting crude was dissolved in water and acetonitrile and was purified by Reverse phase chromatography (0-100% CH3CN 1% AcOH in H2O 1% AcOH) to afford **19** (73.1 mg, 70.6 µmol, 104 %) as a colorless oil. LCMS (ESI-) calculated for C₄₁H₅₆N₅O₂₂S₂⁻ (M-H⁻) 1034.3, found 1034.7.

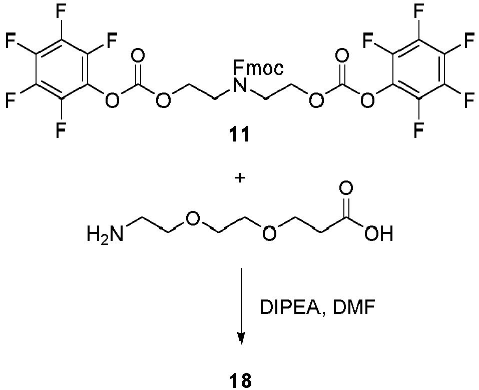

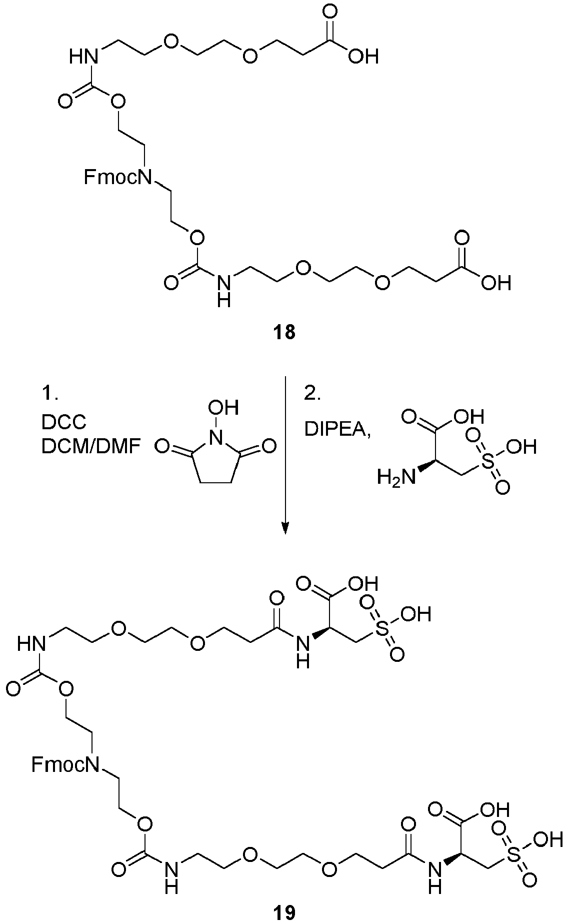

### Example 10. Preparation of compound 21

DIPEA (7.49 mg, 10.1 µL, 6 Eq, 57.9 µmol) and HATU (9.18 mg, 2.5 Eq, 24.1 µmol) were added to compound **19** (10.0 mg, 1 Eq, 9.65 µmol) in DMF (0.5 mL). Subsequently (4-(6-methyl-1,2,4,5-tetrazin-3-yl)phenyl)methanamine hydrochloride (5.05 mg, 2.2 Eq, 21.2 µmol) was added. The mixture was stirred for 1 hour. The mixture was concentrated in vacuo and the crude dissolved in DCM (10 ml). The residue was purified by flash column chromatography over silicagel (0% --> 30% MeOH in DCM) to afford **20** (2.4 mg, 1.7 µmol, 18 %) as a pink solid. LCMS (ESI-) calculated for C₆₁H₇₄N₁₅O₂₀S₂⁻ (M-H⁻) 1400.5, found 1400.7.

Triethylamine (1.7 mg, 2.4 µL, 10 Eq, 17 µmol) was added to compound **20** (2.4 mg, 1.0 Eq, 1.7 µmol) in DMF (0.1 mL). The reaction mixture was stirred for 42h until Fmoc deprotection was observed. TMTH-SI-OSu (0.70 mg, 1.2 Eq, 2.1 µmol) was added to the reaction mixture stirring was continued for 92 hours. LCMS analysis indicated formation of **21.** LCMS (ESI-) calculated for C₅₇H₇₉N₁₆O₂₀S₃⁻ (M-H⁻) 1403.5, found 1403.6.

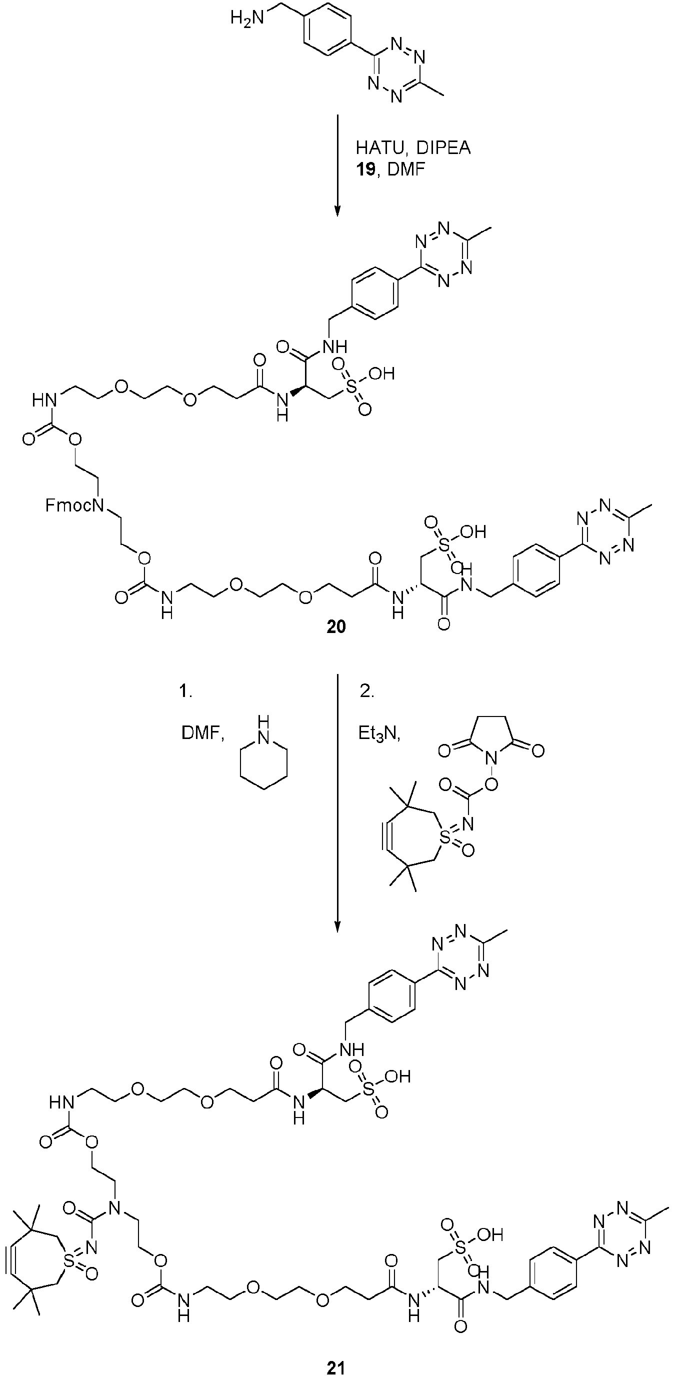

### Examples 11-12: Preparation of click probe-functionalized antibodies

### Example 11: Preparation of trastuzumab-(6-N₃-GalNAc)₂

According to the general procedure for enzymatic remodeling, trastuzumab (obtained from the pharmacy) was converted to trastuzumab-(6-N₃-GalNAc)₂. Mass spectral analysis of a sample after IdeS treatment showed one major Fc/2 product (observed mass 24366 Da, approximately 85% of total Fc/2 and observed mass for minor -fucose product 24220 Da, approximately 10% of the total Fc/2), corresponding to the expected product.

### Example 12: Preparation of trastuzumab-(tetrazine)₄

To a solution of trastuzumab-(6-N₃-GalNAc)₂ (1.2 mL, 25 mg, 20.61 mg/mL in TBS pH 7.5) was added 167 µL bistetrazine compound **3** (10 mM stock solution in DMF) in propylene glycol (1.08 mL, 50% of final volume). The reaction was incubated overnight at rt. To remove the excess of bistetrazine compound **3,** a HiTrap 26-10 desalting column (Cytiva) was used. The column was rinsed with 0.2M NaOH and equilibrated with TBS pH 7.5 before the injection and elution of trastuzumab-(tetrazine)₄. Subsequently the product was concentrated to 27.9 mg/mL using a Vivaspin Turbo 4 10 kDa MWCO ultrafiltration unit (Sartorius). Mass spectral analysis of the sample after IdeS treatment showed one major Fc/2 product (calcd mass 25445, observed mass 25446 Da, approximately 95% of total Fc/2), corresponding to the pink coloured trastuzumab-**3.**

### Examples 13 - 16: Conjugation of linker-payloads to (modified) monoclonal antibodies

### Example 13: Conjugation of trastuzumab(6-N₃-GalNAc)₂ with BCN-HS-PEG2-HS-(va-PAB-Ex)₂ 7 to obtain conjugate DAR 4 trastuzumab-7

To a solution of trastuzumab(6-N₃-GalNAc)₂ (2846 µL, 60.0 mg, 21.08 mg/ml in TBS pH 7.5) was added TBS (754 µL), sodium deoxycholate (600 µL, 110 mM) and BCN-HS-PEG2-HS-(va-PAB-Ex)₂ 7 (160 µL, 10 mM solution in DMF) in propylene glycol (1640 µL). The reaction was incubated overnight at rt. Next the conjugate was purified on a HiLoad 16/600 Superdex200 PG column (Cytiva) on an AKTA Pure (Cytiva). To remove the excess of free payload, 13 mg of active charcoal (Carbon RHC, Filtrox AG) was added and rotated for overnight. The charcoal was removed by centrifugation and subsequently filtered over a PES syringe filter (pore 0.20 µm, Corning). Subsequently the solution was dialysed three times to 20 mM histidine, 6% sucrose buffer pH 6.0, and concentrated using a Vivaspin Turbo 4 10 kDa MWCO ultrafiltration unit (Sartorius). 0.04% Tween-20 was added before filter sterilization. Mass spectral analysis of the sample after IdeS treatment showed one major Fc/2 product (observed mass 26493 Da, approximately 90% of total Fc/2 and observed mass for minor-fucose product 26347 Da, approximately 10% of the total Fc/2), corresponding to the conjugate **DAR 4** trastuzumab**-7**. RP-UPLC analysis of the sample under reducing conditions showed an average DAR of 3.77.

### Example 14: Conjugation of trastuzumab(tetrazine)₄ with BCN-HS-PEG2-HS-(va-PAB-Ex)₂ 7 to obtain DAR 8 conjugate DAR 8 trastuzumab-7

To a solution of trastuzumab-(tetrazine)₄ (269 µL, 7.5 mg, 27.88 mg/ml in TBS pH 7.5) was added sodium deoxycholate (75 µL, 110 mM) and BCN-HS-PEG2-HS-(va-PAB-Ex)₂ **7** (30 µL, 10 mM solution in DMF) and propylene glycol (270 µL). The reaction was incubated at room temperature for five minutes, an immediate colour change from pink to yellow/colourless was observed. The precipitate of the reaction was removed by centrifugation, the pellet was rinsed with TBS (200 µL), centrifuged and combined. The mixture was diluted in 1 mL 2 M (NH₄)₂SO₄ in 50 mM sodium phosphate buffer pH 7.0 and subsequently filtered over a PES syringe filter (pore 0.20 µm, Corning). The product was then purified over a HIC, 4.6 mL Hi Screen butyl HP, column using an Akta Purifier 10. A gradient from 1 M (NH₄)₂SO₄in 50 mM sodium phosphate buffer pH 7.0 to 10% MeCN in 50 mM phosphate pH 7.0 was used with a flow rate of 0.7 mL/min. First, the gradient was increased from 0-75% B in 70 minutes and subsequently from 75-100% B in another 60 minutes. The product was collected at 85% B. Subsequently the solution was buffer exchanged using a HiTrap 26-10 desalting column (Cytiva), rinsed with 0.2M NaOH and equilibrated with PBS. Lastly the product was concentrated to 3.28 mg/mL using a Vivaspin Turbo 4 10 kDa MWCO ultrafiltration unit (Sartorius). Mass spectral analysis of the sample after IdeS treatment showed one major Fc/2 product (calcd mass 29654, observed mass 29655 Da, approximately 95% of total Fc/2), corresponding to the **DAR 8** trastuzumab**-7**. RP-UPLC analysis of the sample showed an average DAR of 7.43.

### Example 15: Conjugation of trastuzumab(6-N₃-GalNAc)₂ with BCN-HS-PEG2 -(eva-PAB-Ex)₂ 7 to obtain conjugate DAR 4 trastuzumab-9

To a solution of trastuzumab(6-N₃-GalNAc)₂ (2372 µL, 50.0 mg, 21.08 mg/ml in TBS pH 7.5) was added TBS (628 µL), sodium deoxycholate (500 µL, 110 mM) and BCN-HS-PEG2-(eva-PAB-Ex)₂ **9** (133 µL, 10 mM solution in DMF) in propylene glycol (1367 µL). The reaction was incubated overnight at rt. Next the conjugate was purified on a HiLoad 16/600 Superdex200 PG column (Cytiva) on an AKTA Pure (Cytiva). To remove the excess of free payload, 11 mg of active charcoal (Carbon RHC, FiltroxAG) was added and rotated for overnight. The charcoal was removed by centrifugation and subsequently filtered over a PES syringe filter (pore 0.20 µm, Corning). Subsequently the solution was dialysed three times to 20 mM histidine, 6% sucrose buffer pH 6.0, and concentrated using a Vivaspin Turbo 4 10 kDa MWCO ultrafiltration unit (Sartorius). 0.04% Tween-20 was added before filter sterilization. Mass spectral analysis of the sample after IdeS treatment showed one major Fc/2 product (observed mass 26672 Da, approximately 90% of total Fc/2), corresponding to the conjugate **DAR 4** trastuzumab-**9**. RP-UPLC analysis of the sample under reducing conditions showed an average DAR of 3.94.

### Example 16: Conjugation of trastuzumab(tetrazine)₄ with BCN-HS-PEG2-(eva-PAB-Ex)₂ 9 to obtain DAR 8 conjugate DAR 8 trastuzumab-9

To a solution oftrastuzumab-(tetrazine)₄ (269 µL, 7.5 mg, 27.88 mg/ml in TBS pH 7.5) was added sodium deoxycholate (75 µL, 110 mM) and BCN-HS-PEG2-(eva-PAB-Ex)₂ **9** (15 µL, 20 mM solution in DMF) and propylene glycol (285 µL). The reaction was incubated at room temperature for five minutes, an immediate colour change from pink to yellow/colourless was observed. The precipitate of the reaction was removed by centrifugation, the pellet was rinsed with TBS (200 µL), centrifuged and combined. The mixture was diluted in 1 mL 2 M (NH₄)₂SO₄ in 50 mM sodium phosphate buffer pH 7.0 and subsequently filtered over a PES syringe filter (pore 0.20 µm, Corning). The product was then purified over a HIC, 4.6 mL Hi Screen butyl HP, column using an Akta Purifier 10. A gradient from 1 M (NH₄)₂SO₄ in 50 mM sodium phosphate buffer pH 7.0 to 10% MeCN in 50 mM phosphate pH 7.0 was used with a flow rate of 0.7 mL/min. First the gradient was increased from 0-75% B in 70 minutes. The product was collected at 65% B. Subsequently the solution was buffer exchanged using a HiTrap 26-10 desalting column (Cytiva), rinsed with 0.2M NaOH and equilibrated with PBS. Lastly the product was concentrated to 4.77 mg/mL using a Vivaspin Turbo 4 10 kDa MWCO ultrafiltration unit (Sartorius). Mass spectral analysis of the sample after IdeS treatment showed one major Fc/2 product (calcd mass 30012, observed mass 30014 Da, approximately 95% of total Fc/2), corresponding to the **DAR 8** trastuzumab**-9**. RP-UPLC analysis of the sample showed an average DAR of 7.57.

### Examples 17-18: Stability testing of DAR 8 constructs and DAR 4 constructs

### Example 17: Aggregation study of DAR 8 and DAR 4 constructs at physiological conditions and comparison to Enhertu^{®}

All samples were buffer exchanged using a HiTrap 26-10 desalting column (Cytiva), rinsed with 0.2M NaOH and equilibrated with PBS. Samples were diluted to 1 mg/mL and stored at 37 °C for 28 days. At designated time points (t=0, 1, 3, 7, 14, 21 and 28 days) a sample was taken and analyzed using SE-HPLC and RP-UPLC, as illustrated in Figure 18.

### Example 18: Serum stability studies in human and mouse serum

Stability of ADCs in mice and human serum was tested. Prior to the assay, the serum (obtained from Sigma-Aldrich) was depleted from all IgG using CaptivA^{®} Protein A agarose (1 mL agarose/mL serum). ADCs were added to the depleted human/mouse serum to a final concentration of 0.1 mg/mL followed by incubation at 37°C. At each time point, 0.5 mL was snap frozen and stored at -80°C until further analysis. To isolate the ADCs after incubation, 20 µl CaptivA^{®} Protein A agarose resin was added to the samples and incubated for 1 hour at room temperature. The resin was washed three times with PBS (1 mL) and subsequently 0.1 M Glycine-HCl pH 2.7 (0.4 mL) was added to elute the ADCs. After elution, the samples were immediately neutralized with 1.0 M Tris pH 8.0 (0.1 mL). The samples were spin filtrated against PBS for three times using Amicon Ultra spin-filter 0.5 mL MWCO 10 kDa (Merck Millipore) and the volume was reduced to 40 µL, yielding a final ADC concentration of approximately 1 mg/mL. Samples were analyzed on RP-HPLC (DTT reduced) to determine the DAR, tables below.

**Table 1. Human serum DAR (% from DAR at t=0):**

| | **DAR t=0** | **DAR t=3** | **DAR t=7** |
|---|---|---|---|
| trastuzumab**-7** | 8.0 | 7.8 (98%) | 7.6 (95%) |
| trastuzumab-**9** | 8.0 | 7.9 (98%) | 7.8 (97%) |
| Enhertu^{®} | 7.9 | 6.3 (80%) | 5.1 (65%) |

**Table 2. Mouse serum DAR (% from DAR at t=0):**

| | **DAR t=0** | **DAR t=3** | **DAR t=7** |
|---|---|---|---|
| trastuzumab-**7** | 8.0 | 7.9 (99%) | 7.8 (98%) |
| trastuzumab-**9** | 8.0 | 7.2 (90%) | 7.4 (92%) |
| Enhertu^{®} | 7.7 | 6.3 (82%) | 4.2 (54%) |

### Example 19: HIC-HPLC measurements for relative retention time determination

Samples were diluted to 1 mg/mL in PBS. HIC analysis is performed on an Agilent 1200 series using a TSKgel^{®} Butyl-NPR HPLC Column (3.5 cm × 4.6 mm, 2.5 µm). 10 µL sample is injected at a flow rate of 0.5 mL/min using a gradient starting from 100% buffer A (2 M ammonium sulfate in 50 mM potassium phosphate pH 6.0) to 100% buffer B (50 mM potassium phosphate pH 6.0 + 20% isopropanol) in 20 minutes. The native mAb (trastuzumab) is measured and its retention time is set as 1. The other measured samples are relative retention times compared to the native mAb, as summarized in Table 3 and illustrated in Figure 17.

**Table 3. relative retention times to trastuzumab measured with HIC-HPLC:**

| construct | relative retention time |
|---|---|
| trastuzumab | 1.00 |
| DAR8 trastuzumab**-7** | 1.20 |
| DAR8 trastuzumab-**9** | 1.14 |
| Enhertu^{®} | 1.25 |

## Claims

1. A process for preparing an antibody-payload-conjugate having a DAR of 6 or higher, comprising:
(a) providing a modified antibody having the structure Ab(F¹)_{z}, wherein Ab is an antibody, z is 2 or 4, and F¹ is a click probe;
(b) reacting the modified antibody with z equivalents of Q¹L^{A}(F²)_{y}, wherein Q¹ is a click probe that is reactive towards F¹, L^{A} is a heterobifunctional (y + 1)-valent linker, y is 2, 3 or 4, and F² is a click probe that is not reactive towards Q¹, to obtain an antibody-linker construct of structure Ab(Z¹L^{A}(F²)_{y})₂, wherein Z¹ is a connecting group obtained by reaction of F¹ and Q¹;
(c) reacting the antibody-linker construct with z × y equivalents of Q²(L^{B})Dₓ, wherein Q² is a click probe that is reactive towards F², L^{B} is a (x + 1)-valent linker, x is 1, 2, 3 or 4, provided that x + y is at least 4, and D is a payload molecule, to obtain the conjugate of structure Ab(Z¹L^{A}(Z²(L^{B})Dₓ)_{y})_{z}, wherein Z² is a connecting group obtained by reaction of F² and Q².

2. The process according to claim 1, wherein y is 2 and L^{A} is a heterobifunctional trivalent linker of structure
-(L¹¹)-BM(L¹²-)(L¹³-)
wherein:
- L¹¹ is connected to Q¹ or Z¹, and L¹² and L¹³ are connected to F² or Z², wherein L¹¹, L¹² and L¹³ each individually consist of one or more building blocks selected from CH₂, CH=CH, C≡C, C(O), NR¹³, O, S, S(O), S(O)z, PR¹³ and P(O)R¹³, preferably the building blocks are selected from CH₂, CH=CH, C(O), NR¹³, O, S, S(O) and S(O)z, and
- BM is a branching moiety, preferably selected from a carbon atom, a nitrogen atom, a phosphorus atom, a (hetero)aromatic ring, a (hetero)cycle or a polycyclic moiety, more preferably BM is a nitrogen atom.

3. The process according to claim 1 or 2, wherein x is 2 and L^{B} is a heterobifunctional trivalent linker of structure
-(L¹)-[(L²)ₒ-(L³)ₚ-(L⁴)_{q}]2
wherein:
- L¹ is connected to Q² or Z², and both occurrences of L⁴ are connected to D;
- L¹, L², L³ and L⁴ are each individually linkers that together link Q² or Z² to D;
- o, p and q are each individually 0 or 1, preferably wherein o = p = 1;
and preferably wherein:
(a) linker L¹ is represented by:
-(W)ₖ-(A)_{d}-(B)ₑ-(A)_{f}(C(O))_{g}-BM[-(A)_{d'}-(B)_{e'}-(A)_{f'}-(C(O))_{g'}-]₂,
wherein:
- d and d' are individually 0 or 1;
- e and e' are individually an integer in the range 1 - 10;
- f and f' are individually 0, or 1;
- g and g' are individually an integer in the range 0 - 10;
- k = 0 or 1 with the proviso that if k = 1 then d = 0;
- A is a carbamoyl or acyl sulfamide group according to structure (**23**) 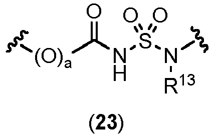 wherein a = 0 or 1, and R¹³ is selected from the group consisting of hydrogen, C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups, the C₁ - C₂₄ alkyl groups, C₃ - C₂₄ cycloalkyl groups, C₂ - C₂₄ (hetero)aryl groups, C₃ - C₂₄ alkyl(hetero)aryl groups and C₃ - C₂₄ (hetero)arylalkyl groups optionally substituted and optionally interrupted by one or more heteroatoms selected from O, S and NR¹⁴ wherein R¹⁴ is independently selected from the group consisting of hydrogen and C₁ - C₄ alkyl groups, or R¹³ is D connected to N via a spacer moiety, preferably wherein the spacer moiety is -(B)_{g}-(C(O))_{g}-(L²)ₒ-(L³)ₚ-(L⁴)_{q}-;
- W is -OC(O)-, -C(O)O-, -C(O)NH-, -NHC(O)-, -OC(O)NH-, -NHC(O)O-, -C(O)(CH₂)ₘC(O)-, -C(O)(CH₂)ₘC(O)NH- or -(4-Ph)CH₂NHC(O)(CH₂)ₘC(O)NH-, wherein m is an integer in the range 0 - 10;
- B is a -CH₂-CH₂-O- or a -O-CH₂-CH₂- moiety, or (B)ₑ is a -(CH₂-CH₂-O)ₑ₁-CH₂-CH₂- moiety, wherein e1 is an integer in the range 1-10;
- BM is a branching moiety, to which two instances of -(A)_{d}-(B)ₑ-(A)_{f}-(C(O))_{g}- are connected and both (C(O))_{g'} moieties are connected to -(L²)ₒ-(L³)ₚ-(L⁴)_{q}-D, wherein L², L³, L⁴, o, p, q and D are each selected individually;
and/or
(b) linker L² is a peptide spacer, preferably comprising 1 - 5 amino acids, more preferably a dipeptide, tripeptide or tetrapeptide spacer;
and/or
(c) linker L³ is a self-immolative spacer, preferably:
- when o = 1, L³ is a para-aminobenzyloxycarbonyl (PABC) derivative according to structure (**26**): 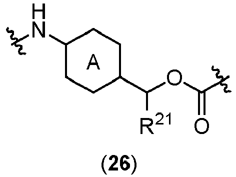
- when o = 1, L³ is a glucuronide derivative according to structure (**27**): 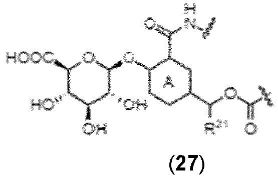 wherein R²¹ is H, R²⁶ or C(O) R²⁶, wherein R²⁶ is C₁ - C₂₄ (hetero)alkyl groups, C₃ - C₁₀ (hetero)cycloalkyl groups, C₂ - C₁₀ (hetero)aryl groups, C₃ - C₁₀ alkyl(hetero)aryl groups and C₃ - C₁₀ (hetero)arylalkyl groups, which are optionally substituted and optionally interrupted by one or more heteroatoms selected from O, S and NR²⁸ wherein R²⁸ is independently selected from the group consisting of hydrogen and C₁ - C₄ alkyl groups, preferably wherein R²¹ is H or C(O)R²⁶, wherein R²⁶ = 4-N-methyl-piperazine or morpholine, most preferably wherein R²¹ is H;
and/or
(d) linker L⁴ is a spacer selected from:
- an aminoalkanoic acid spacer according to the structure - NR²²-(Cₓ-alkylene)-C(O)-, wherein x is an integer in the range 1 - 20 and R²² is H or C₁ - C₄ alkyl; or
- an ethyleneglycol spacer according to the structure -NR²²-(CH₂-CH₂-O)ₑ₆-(CH₂)ₑ₇-C(O)-, wherein e6 is an integer in the range 1 - 10, e7 is an integer in the range 1 - 3 and R²² is H or C₁ - C₄ alkyl; or
- a diamine spacer according to the structure -NR²²-(Cₓ-alkylene)-NR²²-(C(O))ₕ-, wherein h is 0 or 1, x is an integer in the range 1-10 and R²² is H or C₁ - C₄ alkyl.

4. The process according to any one of claims 1 - 3, wherein the reaction of step (b) and the reaction of step (c) are both cycloadditions, preferably wherein the reaction of step (b) is a 1,3-dipolar cycloaddition and the reaction of step (c) is an inverse electron-demand Diels-Alder cycloaddition.

5. The process according to any one of claims 1 - 4, wherein:
- Q¹ and Q² are both individually click probes comprising a (hetero)cycloalkyne moiety or a (hetero)cyclo-E-alkene moiety, preferably wherein the click probes are selected from the group consisting of (Q2) - (Q20) according to the following structures: 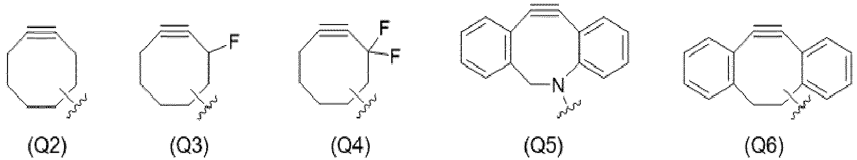 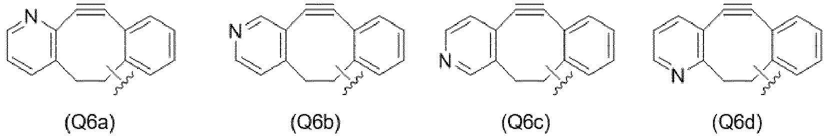 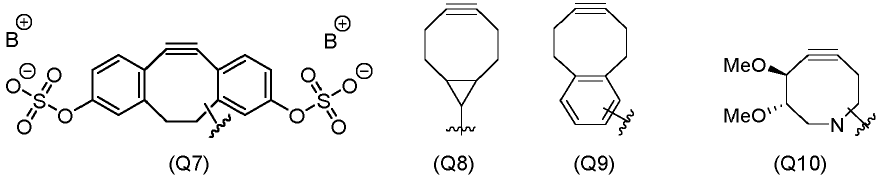 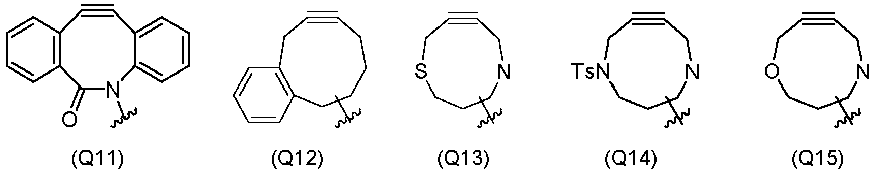 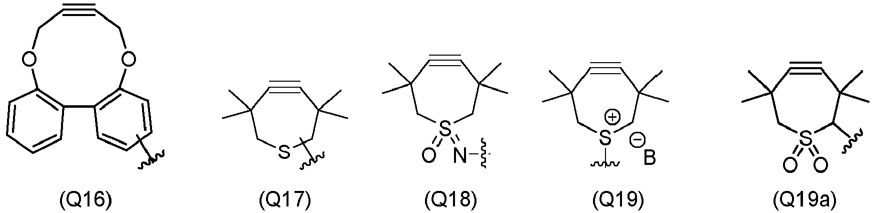 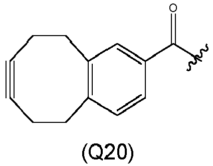 wherein:
- the wavy bond represents the connection to L^{A} or L^{B}, and is connected to any available carbon or nitrogen atom;
- the nitrogen atom of (Q10), (Q13), (Q14) and (Q15) may bear the connection to L^{A} or L^{B}, or contains a hydrogen atom or is substituted;
- B⁽⁻⁾ is an anion;
- B⁽⁺⁾ is a cation;
and/or
- F¹ and F² are both individually click probes selected from the group consisting of azide, tetrazine, triazine, nitrone, nitrile oxide, nitrile imine, diazo compound, dioxothiophene, sydnone, iminosydnone and catechol, preferably wherein the click probes are selected from the group consisting of (F1) - (F10) according to the following structures: 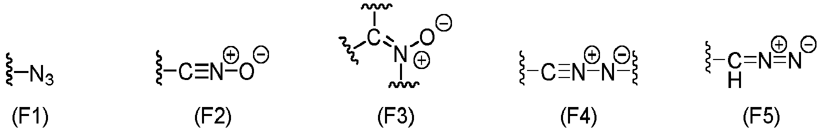 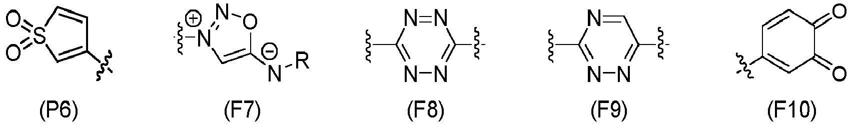 wherein:
- the wavy bond represents the connection to Ab or L^{A}, where for (F3), (F4), (F8) and (F9), the connection can be via any one of the wavy bonds, and the other wavy bond may then be connected to a group selected from hydrogen, C₁ - C₂₄ alkyl groups, C₂ - C₂₄ acyl groups, C₃ - C₂₄ cycloalkyl groups, C₂ - C₂₄ (hetero)aryl groups, C₃ - C₂₄ alkyl(hetero)aryl groups, C₃ - C₂₄ (hetero)arylalkyl groups and C₁ - C₂₄ sulfonyl groups, which may optionally be substituted and optionally be interrupted by one or more heteroatoms selected from O, S and NR³², wherein R³² is independently selected from the group consisting of hydrogen and C₁ - C₄ alkyl groups.

6. The process according to any one of claims 1 - 5, wherein:
- F¹ is an azide and Q¹ is a benzoannulated or tetramethylated (hetero)cycloalkyne, preferably selected from (Q5), (Q6), (Q6a), (Q6b), (Q6c), (Q6d), (Q7), (Q18) and (Q19a) as defined in claim 5; and
- F² is a tetrazine and Q² is a bicyclononyne, preferably wherein Q² is according to (Q9) as defined in claim 5 and F² is according to (F8a): 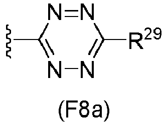 wherein:
- R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C¹⁻⁶ alkyl and C(O)-NR³³-aryl, wherein R³³ is H or C₁₋₄ alkyl, preferably wherein R²⁹ is selected from hydrogen, methyl, phenyl, pyridyl, pyridinyl and pyrimidinyl.

7. The process according to any one of claims 1 - 6, wherein the payload D is a pharmaceutically active compound, preferably a cytotoxin.

8. The process according to any one of claims 1 - 7, wherein the antibody provided in step (a) is according to structure:
Ab[(L⁶)S(F¹)]_{z},
wherein:
- z is 2 or 4;
- L⁶ is -GlcNAc(Fuc)_{w}-(G)ⱼ-S-(L⁷)_{w'}-, wherein G is a monosaccharide, j is an integer in the range of 0 - 10, S is a sugar or a sugar derivative, GIcNAc is N-acetylglucosamine and Fuc is fucose, w is 0 or 1, w' is 0 or 1 and L⁷ is -N(H)C(O)CH₂-, -N(H)C(O)CF₂- or -CH₂-.

9. A conjugate obtainable by the process according to any one of claims 1 - 8.

10. A conjugate having structure Ab(Z¹L^{A}(Z²(L^{B})Dₓ)_{y})₂, wherein:
- Ab is an antibody;
- Z¹ and Z² are connecting groups obtainable by reaction between two click probes;
- L^{A} is a heterobifunctional (y + 1)-valent linker;
- L^{B} is a (x + 1)-valent linker
- x is 1, 2, 3 or 4;
- y is 2, 3 or 4, provided that x + y is at least 4;
- D is a payload.

11. The conjugate according to claim 10, wherein Z¹ and Z² are individually selected from the structures (Z2) - (Z38a): 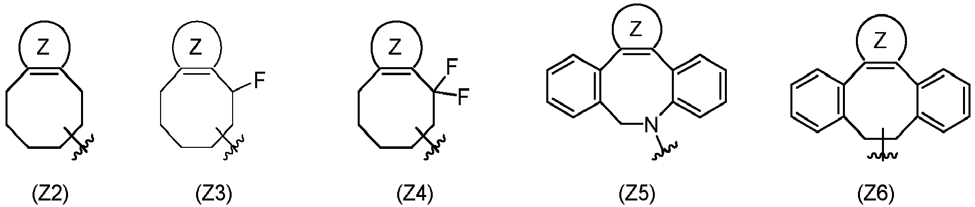 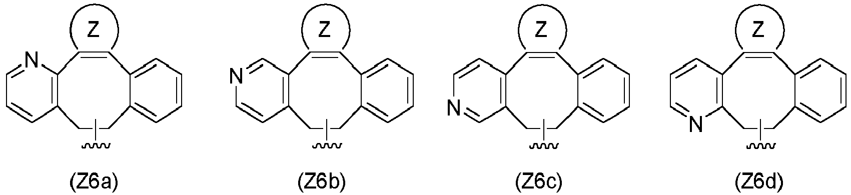 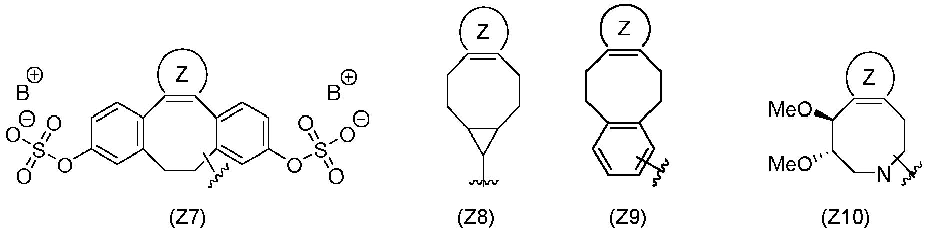 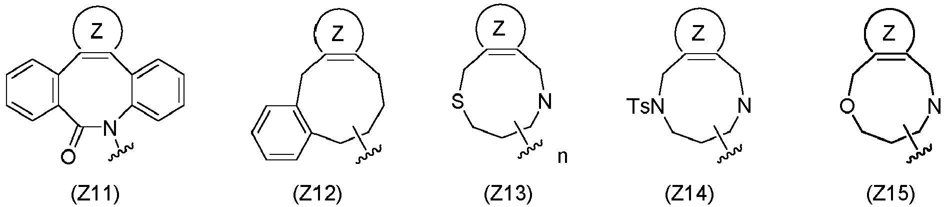 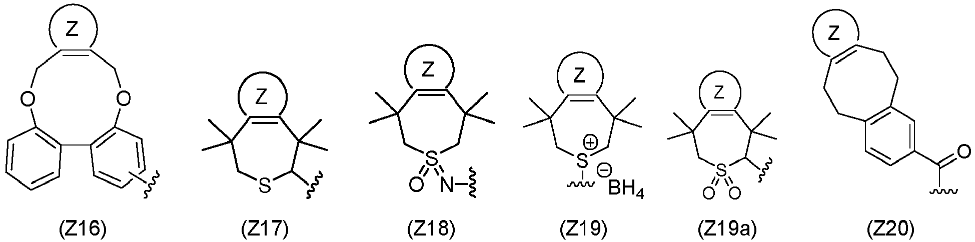 wherein:
- the wavy bond represents the connection to L^{A} or L^{B}, and is connected to any available carbon or nitrogen atom;
- ring Z is connected to Ab or L^{A} and is preferably selected from a triazole, a cyclohexene, a cyclohexadiene, a [2.2.2]-bicyclooctadiene, a [2.2.2]-bicyclooctene, an oxazoline, an isoxazolidine, a pyrazoline or a piperazine;
- the nitrogen atom of (Z10), (Z13), (Z14) and (Z15) may bear the connection to L^{A} or L^{B}, or contains a hydrogen atom or is substituted;
- B⁽⁻⁾ is an anion;
- B⁽⁺⁾ is a cation.

12. A pharmaceutical composition comprising the conjugate according to any one of claims 9-11 and a pharmaceutically acceptable carrier.

13. A linker-construct having structure Q¹L^{A}(F²)_{y}, wherein:
- Q¹ is a click probe;
- L^{A} is a heterobifunctional (y + 1)-valent linker;
- F² is a click probe that is not reactive towards Q¹;
- y is 2, 3 or 4.

14. The linker-construct according to claim 13, wherein:
- Q¹ is Q¹ is a benzoannulated or tetramethylated (hetero)cycloalkyne, preferably selected from (Q5), (Q6), (Q6a), (Q6b), (Q6c), (Q6d), (Q7), (Q18) and (Q19a) as defined in claim 5
- F² is a tetrazine, preferably according to (F8a): 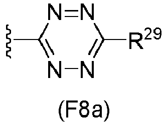 wherein:
- R²⁹ is selected from hydrogen, C₁₋₆ alkyl, aryl, C(O)-C₁₋₆ alkyl, C(O)-aryl, C(O)-O-C₁₋₆ alkyl, C(O)-O-aryl, C(O)-NR³³-C¹⁻⁶ alkyl and C(O)-NR³³-aryl, wherein R³³ is H or C₁₋₄ alkyl, preferably wherein R²⁹ is selected from hydrogen, methyl, phenyl, pyridyl, pyridinyl and pyrimidinyl; and
- y is 2, 3 or 4, preferably y is 2 or 3, most preferably y is 2.

15. A method for:
- targeting a tumour cell expressing a specific extracellular receptor, comprising contacting the conjugate according to any one of claims 9-11 with cells that may possibly express the extracellular receptor, wherein the antibody specifically targets the extracellular receptor; and/or
- the treatment of cancer, comprising administering to a subject in need thereof the conjugate according to any one of claims 9 - 11, wherein the cancer cells specifically express an extracellular receptor,
preferably wherein the extracellular receptor is selected from the group consisting of 5T4, ADAM-9, AMHRII, ASCT2, ASLG659, ASPHD1, av-integrin, Axl, B7-H3, B7-H4, BAFF-R, BCMA, BMPR1B, Brevican, c-KIT, c-Met, C4.4a, CA-IX, cadherin-6, CanAg, CD123, CD13, CD133, CD138/syndecan-1, CD166, CD19, CD20, CD203c, CD205, CD21, CD22, CD228, CD25, CD30, CD324, CD33, CD37, CD38, CD45, CD46, CD48a, CD56, CD70, CD71, CD72, CD74, CD79a, CD79b, CEACAM5, claudin-18.2, claudin-6, CLEC12A, CLL-1, Cripto, CRIPTO, CS1, CXCR5, DLK-1, DLL3, DPEP3, E16, EGFR, ENPP3, EpCAM, EphA2, EphB2R, ETBR, FAP, FcRH1, FcRH2, FcRH5, FGFR2, fibronectin, FLT3, folate receptor alpha, Gal-3BP, GD3, GDNF-Ra1, GEDA, GFRA1, Globo H, gpNMB, GPR172A, GPR19, GPR54, guanyl cyclase C, HER2, HER3, HLA-DOB, IGF-1R, IL13R, IL20Rα, Lewis Y, LGR5, LIV-1, LRRC15, LY64, Ly6E, Ly6G6D, LY6K, MDP, MFI2, MICA/B, MOSPD2, MPF, MSG783, MUC1, MUC16, NaPi2b, NCA, nectin-4, Notch3, P-cadherin, P2X5, PD-L1, PMEL17, PRLR, PSCA, PSCA hlg, PSMA, PTK7, RET, RNF43, RON, ROR1, ROR2, Sema 5b, SLITRK6, SSTR2, STEAP1, STEAP2, TAG72, TENB2, TF, TIM-1, TM4SF, TMEFF, TMEM118, TMEM46, transferrin, TROP-2, TrpM4, TWEAKR, receptor tyrosine kinases (RTK), tenascin, or antibody is specific to an extracellular protein resulting from a viral infection and/or for a tumour-associated carbohydrate antigen (TACA).
